# EUROPEAN PATENT APPLICATION

(11) **EP 4 324 828 A1**
(43) Date of publication of application: **21.02.2024**
(21) Application number: 22787651.3
(22) Date of filing: 15.04.2022
(51) Int. Cl.: C07D 401/14, C07D 401/06, C07D 413/14, C07D 209/02, A61K 31/454, A61P 27/00

(54) **BICYCLIC SUBSTITUTED AROMATIC CARBOXYLIC ACID COMPOUNDS**

(30) Priority: 16.04.2021 CN 202110412189
(71) Applicant: CHIA TAI TIANQING PHARMACEUTICAL GROUP CO., LTD., Lianyungang, Jiangsu 222062 (CN); Medshine Discovery Inc., Nanjing, Jiangsu 210032 (CN)
(72) Inventor: DING, Charles Z, Shanghai 200131 (CN); YAN, Xiaobing, Shanghai 200131 (CN); SUN, Fei, Shanghai 200131 (CN); JIANG, Wen, Shanghai 200131 (CN); LI, Jian, Shanghai 200131 (CN); CHEN, Shuhui, Shanghai 200131 (CN)
(74) Representative: Grünecker Patent- und Rechtsanwälte PartG mbB
(86) International application number: PCT/CN2022/087226
(87) International publication number: WO 2022/218429

(57) **Abstract**

The present application relates to a bicyclic substituted aromatic carboxylic acid compound, and in particular to a compound as represented by formula (I) and a pharmaceutically acceptable salt thereof. In formula (I), R₂ and R₃ and atoms connected thereto together form a 3-6 membered heterocyclic group, or R₂ and R₄ and atoms connected thereto together form a 3-6 membered heterocyclic group. The compound has obvious inhibitory activity on activation of a human serum alternative pathway and significant binding activity to human complement Factor B protein, and also has relatively good in-vivo pharmacodynamic and pharmacokinetic properties.

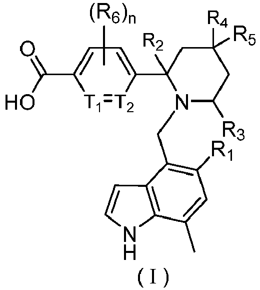

## Description

### CROSS-REFERENCE TO RELATED APPLICATIONS

The present application claims priority and benefit to the Chinese Patent Application No. 202110412189.5 filed with National Intellectual Property Administration, PRC on Apr. 16, 2021, the disclosure of which is incorporated herein by reference in its entirety.

### TECHNICAL FIELD

The present invention relates to a series of bicyclic substituted aromatic carboxylic acid compounds, and in particular to a compound of formula (I) and a pharmaceutically acceptable salt thereof.

### BACKGROUND

The complement system is an important component of innate immunity for the human body to resist infections by exogenous pathogens, bacteria, parasites, and the like, and is also an important component of the connection of innate immunity and adaptive immunity. A complement consists of plasma proteins, including soluble proteins, membrane-bound proteins, and complement receptors, which are mainly produced by the liver or membrane proteins expressed on the surface of a cell and function in plasma, tissues, or cells. The complement system is activated primarily via three pathways: the classical pathway (CP), the lectin pathway (LP), and the alternative pathway (AP).

In the normal physiological state of healthy individuals, the AP pathway remains activated at a low level at all times to monitor the invasion of exogenous pathogens at any time. Complement proteins are distributed on the surface of apoptotic cells. The activation of the complement is strictly regulated only to eliminate apoptotic cells without further activation of other innate or adaptive immune responses. In the case of an infection by exogenous pathogens, the complement system is globally activated, producing inflammatory responses, opsonization or phagocytosis, etc., destroying the pathogen and ultimately activating the adaptive immune response. Low efficiency and excessive stimulation of the complement may be harmful to humans and associated with increased susceptibility to infections or non-communicable diseases, such as autoimmune diseases, chronic inflammation, thrombotic microangiopathy, transplant rejection, tumors, and the like.

The complement factor B (Factor B) acts on the AP pathway, and inhibiting the activity of Factor B can prevent the activation of the AP pathway without interfering with the CP and LP pathways, which can avoid the increased risk of infection due to the inhibition of the complement system. At present, no small-molecule Factor B inhibitor is on the market. The Factor B inhibitor LNP023 of Novartis is under phase III clinical trials and is used for treating diseases such as PNH, IgAN, C3G, and the like. Therefore, there is a need to develop a novel small-molecule inhibitors of the complement system Factor B, increase clinical trials and verification, and use them in treating various diseases caused by complement abnormality to provide a new treatment for unmet clinical needs.

### SUMMARY

The present invention provides a compound of formula (I) or a pharmaceutically acceptable salt thereof, 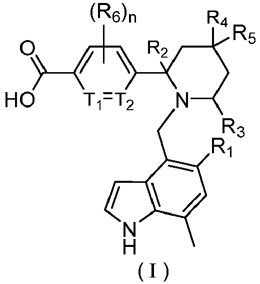 wherein
T₁ and T₂ are each independently selected from the group consisting of C, CH, and N;
R₁ is selected from the group consisting of C₁₋₃ alkoxy and C₁₋₃ alkyl, wherein the C₁₋₃ alkoxy and the C₁₋₃ alkyl are each independently optionally substituted with 1, 2, or 3 Rₐ;
R₅ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
when R₂ and R₃, together with the atom linked thereto, form 3- to 6-membered heterocyclyl, wherein the 3- to 6-membered heterocyclyl is optionally substituted with 1, 2, or 3 R_{b}, R₄ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
alternatively, when R₂ and R₄, together with the atom linked thereto, form 3- to 6-membered heterocyclyl, wherein the 3- to 6-membered heterocyclyl is optionally substituted with 1, 2, or 3 R_{b}, R₃ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
R₆ is selected from the group consisting of halogen, CN, C₁₋₃ alkyl, and C₁₋₃ alkoxy, wherein the C₁₋₃ alkyl and the C₁₋₃ alkoxy are each independently optionally substituted with 1, 2, or 3 R_{c};
each Rₐ is independently selected from the group consisting of F, Cl, Br, and I;
each R_{c} is independently selected from the group consisting of F, Cl, Br, and I;
each R_{b} is independently selected from the group consisting of halogen, C₁₋₃ alkyl, C₁₋₃ alkoxy, -C(=O)-C₁₋₃ alkyl, and -S(=O)ₘ-C₁₋₃ alkyl, wherein the C₁₋₃ alkyl, the C₁₋₃ alkoxy, the -C(=O)-C₁₋₃ alkyl, and the -S(=O)ₘ-C₁₋₃ alkyl are each independently optionally substituted with 1, 2, or 3 R;
each R is independently selected from the group consisting of F, Cl, Br, I, and OH;
n is selected from the group consisting of 0, 1, and 2;
m is selected from the group consisting of 0, 1, and 2.

In another aspect, the present invention further provides the pharmaceutically acceptable salt of the compound of formula (I), 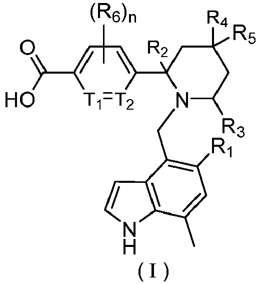 wherein
T₁ and T₂ are each independently selected from the group consisting of C, CH, and N;
R₁ is selected from the group consisting of C₁₋₃ alkoxy and C₁₋₃ alkyl, wherein the C₁₋₃ alkoxy and the C₁₋₃ alkyl are each independently optionally substituted with 1, 2, or 3 Rₐ;
R₅ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
when R₂ and R₃, together with the atom linked thereto, form 3- to 6-membered heterocyclyl, wherein the 3- to 6-membered heterocyclyl is optionally substituted with 1, 2, or 3 R_{b}, R₄ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
alternatively, when R₂ and R₄, together with the atom linked thereto, form 3- to 6-membered heterocyclyl, wherein the 3- to 6-membered heterocyclyl is optionally substituted with 1, 2, or 3 R_{b}, R₃ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
R₆ is selected from the group consisting of halogen, CN, C₁₋₃ alkyl, and C₁₋₃ alkoxy, wherein the C₁₋₃ alkyl and the C₁₋₃ alkoxy are each independently optionally substituted with 1, 2, or 3 R_{c};
each Rₐ is independently selected from the group consisting of F, Cl, Br, and I;
each R_{c} is independently selected from the group consisting of F, Cl, Br, and I;
each R_{b} is independently selected from the group consisting of halogen, C₁₋₃ alkyl, C₁₋₃ alkoxy, -C(=O)-C₁₋₃ alkyl, and -S(=O)ₘ-C₁₋₃ alkyl, wherein the C₁₋₃ alkyl, the C₁₋₃ alkoxy, the -C(=O)-C₁₋₃ alkyl, and the -S(=O)ₘ-C₁₋₃ alkyl are each independently optionally substituted with 1, 2, or 3 R;
each R is independently selected from the group consisting of F, Cl, Br, I, and OH;
n is selected from the group consisting of 0, 1, and 2;
m is selected from the group consisting of 0, 1, and 2.

In some embodiments of the present invention, the compound of formula (I) is not 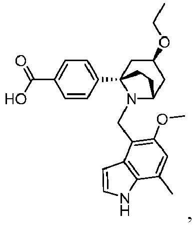 and the other variables are as defined herein.

In some embodiments of the present invention, the compound of formula (I) is not 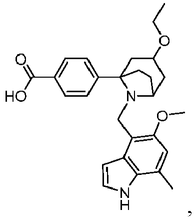 and the other variables are as defined herein.

In some embodiments of the present invention, T₁ is selected from the group consisting of CH or N, and the other variables are as defined herein.

In some embodiments of the present invention, T₂ is selected from the group consisting of CH or N, and the other variables are as defined herein.

In some embodiments of the present invention, T₁ is selected from the group consisting of CH, T₂ is selected from the group consisting of CH, and the other variables are as defined herein.

In some embodiments of the present invention, T₁ is selected from the group consisting of CH, T₂ is selected from the group consisting of N, and the other variables are as defined herein.

In some embodiments of the present invention, T₁ is selected from the group consisting of N, T₂ is selected from the group consisting of CH, and the other variables are as defined herein.

In some embodiments of the present invention, R₁ is selected from the group consisting of CH₃, CH₂CH₃, CH₂CH₂CH₃, CH(CH₃)₂, OCH₃, OCH₂CH₃, OCH₂CH₂CH₃, and OCH(CH₃)₂, wherein the CH₃, the CH₂CH₃, the CH₂CH₂CH₃, the CH(CH₃)₂, the OCH₃, the OCH₂CH₃, the OCH₂CH₂CH₃, and the OCH(CH₃)₂ are each independently optionally substituted with 1, 2, or 3 Rₐ, and the other variables are as defined herein.

In some embodiments of the present invention, R₁ is selected from the group consisting of CH₃ and OCH₃, wherein the CH₃ and the OCH₃ are each independently optionally substituted with 1, 2, or 3 Rₐ, and the other variables are as defined herein.

In some embodiments of the present invention, R₁ is selected from the group consisting of CH₃, CF₃, OCF₃, and OCH₃, and the other variables are as defined herein.

In some embodiments of the present invention, R₁ is selected from the group consisting of CH₃ and OCH₃, and the other variables are as defined herein.

In some embodiments of the present invention, R₁ is selected from the group consisting of OCH₃, and the other variables are as defined herein.

In some embodiments of the present invention, R₅ is selected from the group consisting of H, F, CH₃, CH₂CH₃, CH₂CH₂CH₃, CH(CH₃)₂, OCH₃, OCH₂CH₃, OCH₂CH₂CH₃, and OCH(CH₃)₂, wherein the CH₃, the CH₂CH₃, the CH₂CH₂CH₃, the CH(CH₃)₂, the OCH₃, the OCH₂CH₃, the OCH₂CH₂CH₃, and the OCH(CH₃)₂ are each independently optionally substituted with 1, 2, or 3 halogens, and the other variables are as defined herein.

In some embodiments of the present invention, R₅ is selected from the group consisting of H, CH₃, OCH₃, and OCH₂CH₃, wherein the CH₃, the OCH₃, and the OCH₂CH₃ are each independently optionally substituted with 1, 2, or 3 F, and the other variables are as defined herein.

In some embodiments of the present invention, R₅ is selected from the group consisting of H, CH₃, CH₂F, CHF₂, CF₃, OCH₃, OCH₂F, OCHF₂, OCF₃, and OCH₂CH₃, and the other variables are as defined herein.

In some embodiments of the present invention, R₅ is selected from the group consisting of H, CH₃, OCH₃, CHF₂, and OCH₂CH₃, and the other variables are as defined herein.

In some embodiments of the present invention, R₅ is selected from the group consisting of H and OCH₂CH₃, and the other variables are as defined herein.

In some embodiments of the present invention, R₅ is selected from the group consisting of OCH₂CH₃, and the other variables are as defined herein.

In some embodiments of the present invention, R₅ is selected from the group consisting of OCH₃, and the other variables are as defined herein.

In some embodiments of the present invention, R₅ is selected from the group consisting of H, and the other variables are as defined herein.

In some embodiments of the present invention, R₂ and R₃, together with the atom linked thereto, form aza-cyclobutyl, aza-cyclopentyl, and aza-cyclohexyl, wherein the aza-cyclobutyl, the aza-cyclopentyl, and the aza-cyclohexyl are each independently optionally substituted with 1, 2, or 3 R_{b}, and the other variables are as defined herein. In some embodiments of the present invention, R₄ is selected from the group consisting of H, CH₃, OCH₃, and F, and the other variables are as defined herein.

In some embodiments of the present invention, R₂ and R₃, together with the atom linked thereto, form aza-cyclopentyl, wherein the aza-cyclopentyl is optionally substituted with 1, 2, or 3 R_{b}, and the other variables are as defined herein. In some embodiments of the present invention, R₄ is selected from the group consisting of H, and the other variables are as defined herein.

In some embodiments of the present invention, R₂ and R₄, together with the atom linked thereto, form oxa-cyclobutyl, oxa-cyclopentyl, oxa-cyclohexyl, aza-cyclobutyl, aza-cyclopentyl, and aza-cyclohexyl, wherein the oxa-cyclobutyl, the oxa-cyclopentyl, the oxa-cyclohexyl, the aza-cyclobutyl, the aza-cyclopentyl, and the aza-cyclohexyl are each independently optionally substituted with 1, 2, or 3 R_{b}, and the other variables are as defined herein. In some embodiments of the present invention, R₃ is selected from the group consisting of H, CH₃, OCH₃, and F, and the other variables are as defined herein.

In some embodiments of the present invention, R₂ and R₄, together with the atom linked thereto, form oxa-cyclohexyl and aza-cyclohexyl, wherein the oxa-cyclohexyl and the aza-cyclohexyl are each independently optionally substituted with 1, 2, or 3 R_{b}, and the other variables are as defined herein. In some embodiments of the present invention, R₃ is selected from the group consisting of H, and the other variables are as defined herein.

In some embodiments of the present invention, R₂ and R₄, together with the atom linked thereto, form aza-cyclohexyl, wherein the aza-cyclohexyl is each independently optionally substituted with 1, 2, or 3 R_{b}, and the other variables are as defined herein. In some embodiments of the present invention, R₃ is selected from the group consisting of H, and the other variables are as defined herein.

In some embodiments of the present invention, "R₂ and R₃, together with the atom linked thereto, form" means that R₂ and R₃, together with the carbon atom linked thereto and the N atom between the two carbon atoms, form. In some embodiments of the present invention, "R₂ and R₄, together with the atom linked thereto, form" means that R₂ and R₄, together with the carbon atom linked thereto and the methylene between the two carbon atoms, form.

In some embodiments of the present invention, each R_{b} is independently selected from the group consisting of F, CH₃, CH₂CH₃, OCH₃, OCH₂CH₃, -C(=O)CH₃, -C(=O)CH₂CH₃, SCH₃, SCH₂CH₃, S(=O)CH₃, and S(=O)₂CH₃, wherein the CH₃, the CH₂CH₃, the OCH₃, the OCH₂CH₃, the -C(=O)CH₃, the -C(=O)-CH₂CH₃, the SCH₃, the SCH₂CH₃, the S(=O)CH₃, and the S(=O)₂CH₃ are each independently optionally substituted with 1, 2, or 3 R, and the other variables are as defined herein.

In some embodiments of the present invention, each R_{b} is independently selected from the group consisting of CH₂CH₃, -C(=O)CH₃, and S(=O)₂CH₃, wherein the CH₂CH₃, the -C(=O)CH₃, and the S(=O)₂CH₃ are each independently optionally substituted with 1, 2, or 3 R, and the other variables are as defined herein.

In some embodiments of the present invention, each R_{b} is independently selected from the group consisting of CH₂CH₃, -C(=O)CH₃, and S(=O)₂CH₃, wherein the CH₂CH₃, the -C(=O)CH₃, and the S(=O)₂CH₃ are each independently optionally substituted with 1, 2, or 3 halogens, and the other variables are as defined herein.

In some embodiments of the present invention, each R_{b} is independently selected from the group consisting of CH₂CH₃, -C(=O)CH₃, and S(=O)₂CH₃, wherein the CH₂CH₃, the -C(=O)CH₃, and the S(=O)₂CH₃ are each independently optionally substituted with 1, 2, or 3 F, and the other variables are as defined herein.

In some embodiments of the present invention, each R_{b} is independently selected from the group consisting of F, Cl, CH₃, CF₃, CH₂CH₃, CH₂CH₂F, CH₂CHF₂, CH₂CF₃, -C(=O)-CH₃, S(=O)CH₃, and -S(=O)₂-CH₃, and the other variables are as defined herein.

In some embodiments of the present invention, each R_{b} is independently selected from the group consisting of CH₂CH₃, CH₂CH₂F, CH₂CHF₂, CH₂CF₃, -C(=O)CH₃, and S(=O)₂CH₃, and the other variables are as defined herein.

In some embodiments of the present invention, each R_{b} is independently selected from the group consisting of CH₂CH₃, CH₂CH₂F, CH₂CHF₂, and CH₂CF₃, and the other variables are as defined herein.

In some embodiments of the present invention, each R_{b} is independently selected from the group consisting of CH₂CH₃ and CH₂CF₃, and the other variables are as defined herein.

In some embodiments of the present invention, each Rₐ is independently selected from the group consisting of F and Cl, and the other variables are as defined herein.

In some embodiments of the present invention, each R_{c} is independently selected from the group consisting of F and Cl, and the other variables are as defined herein.

In some embodiments of the present invention, n is selected from the group consisting of 0 and 1, and the other variables are as defined herein.

In some embodiments of the present invention, n is selected from the group consisting of 0, and the other variables are as defined herein.

In some embodiments of the present invention, each R is independently selected from the group consisting of F, and the other variables are as defined herein.

In some embodiments of the present invention, the 3- to 6-membered heterocyclyl is selected from the group consisting of 3- to 6-membered saturated heterocyclyl containing N or O.

In some embodiments of the present invention, the 3- to 6-membered heterocyclyl is selected from the group consisting of 4- to 6-membered saturated heterocyclyl containing one N or one O.

In some embodiments of the present invention, the 3- to 6-membered heterocyclyl is selected from the group consisting of 4- to 6-membered saturated heterocyclyl containing one N.

In some embodiments of the present invention, the 3- to 6-membered heterocyclyl is selected from the group consisting of 4- to 6-membered saturated heterocyclyl containing one O.

In some embodiments of the present invention, the structural unit 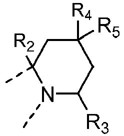 is selected from the group consisting of 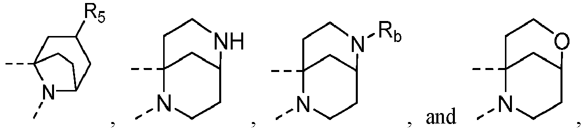 and the other variables are as defined herein.

In some embodiments of the present invention, the structural unit 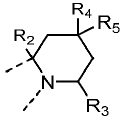 is selected from the group consisting of 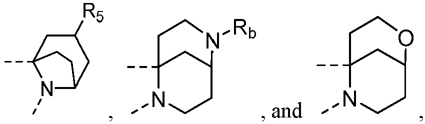 and the other variables are as defined herein.

In some embodiments of the present invention, the structural unit 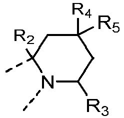 is selected from the group consisting of 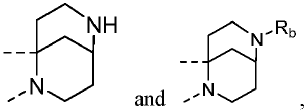 and the other variables are as defined herein.

In some embodiments of the present invention, the structural unit 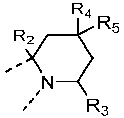 is selected from the group consisting of 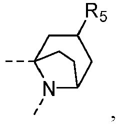 and the other variables are as defined herein.

In some embodiments of the present invention, the structural unit 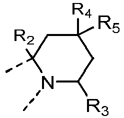 is selected from the group consisting of 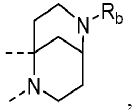 and the other variables are as defined herein.

In some embodiments of the present invention, the structural unit 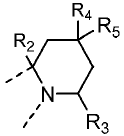 is selected from the group consisting of 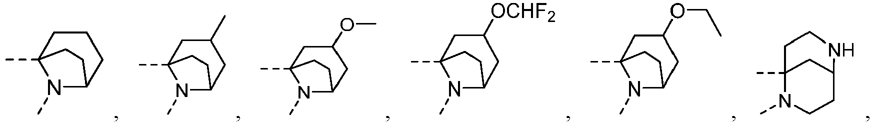 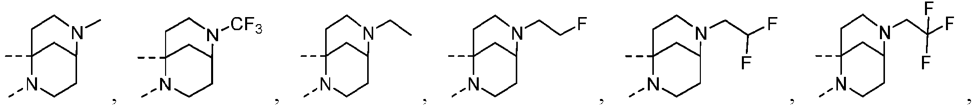 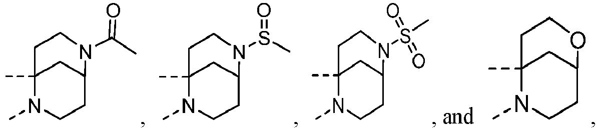 and the other variables are as defined herein.

In some embodiments of the present invention, the structural unit 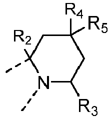 is selected from the group consisting of 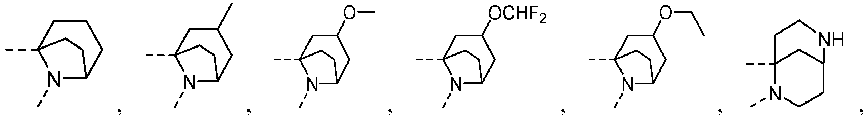 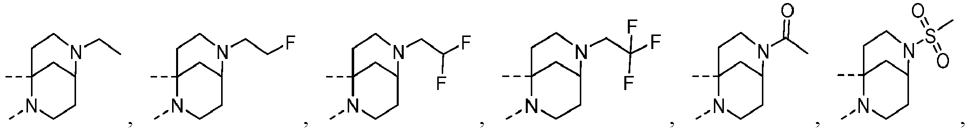 and 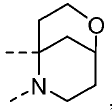 , and the other variables are as defined herein.

In some embodiments of the present invention, the structural unit 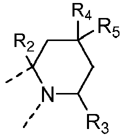 is selected from the group consisting of 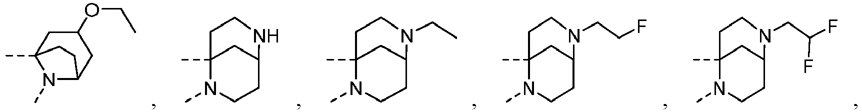 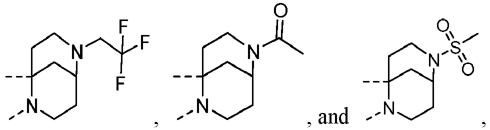 and the other variables are as defined herein.

In some embodiments of the present invention, the structural unit 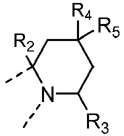 is selected from the group consisting of 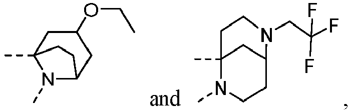 and the other variables are as defined herein.

In some embodiments of the present invention, the structural unit 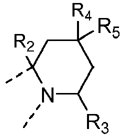 is selected from the group consisting of 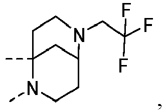 and the other variables are as defined herein.

In some embodiments of the present invention, R₆ is selected from the group consisting of F, Cl, Br, I, CN, CH₃, CH₂CH₃, OCH₃, and OCH₂CH₃, wherein the CH₃, the CH₂CH₃, the OCH₃, and the OCH₂CH₃ are each independently optionally substituted with 1, 2, or 3 R_{c}, and the other variables are as defined herein.

In some embodiments of the present invention, R₆ is selected from the group consisting of F and CN, and the other variables are as defined herein.

In some embodiments of the present invention, the structural unit 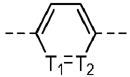 is selected from the group consisting of phenyl, pyridinyl, and pyridazinyl, and the other variables are as defined herein.

In some embodiments of the present invention, the structural unit 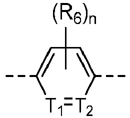 is selected from the group consisting of 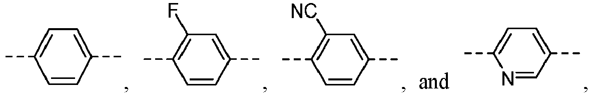 and the other variables are as defined herein.

In some embodiments of the present invention, the structural unit 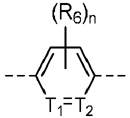 is selected from the group consisting of 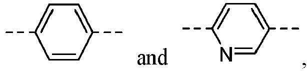 and the other variables are as defined herein.

In some embodiments of the present invention, the structural unit 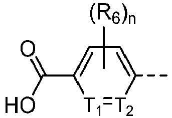 is selected from the group consisting of 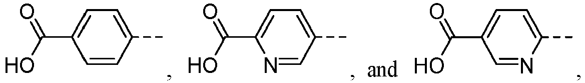 and the other variables are as defined herein.

In some embodiments of the present invention, the compound of formula (I) or the pharmaceutically acceptable salt of the compound of formula (I) is selected from the group consisting of 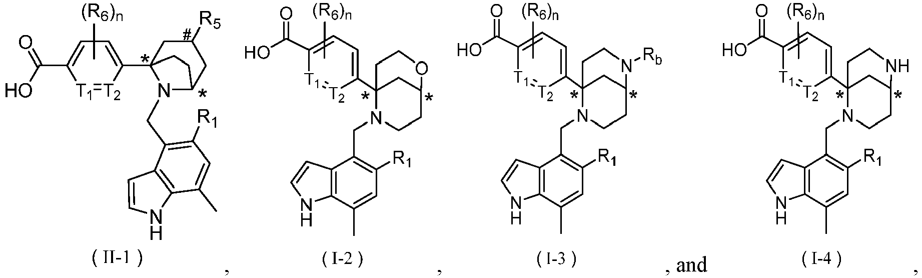 wherein
R₁, R₅, R₆, R_{b}, T₁, T₂, and n are as defined above;
when R₅ is not H, the carbon atom with "#" is a chiral carbon atom present in a form of a single (*R*) or (*S*) enantiomer or in a form enriched with one enantiomer;
the carbon atom with "*" is a chiral carbon atom present in a form of a single (*R*) or (S) enantiomer or in a form enriched with one enantiomer.

In some embodiments of the present invention, the compound of formula (II-1) is not 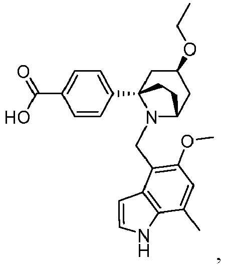 and the other variables are as defined herein.

In some embodiments of the present invention, the compound of formula (I) or the pharmaceutically acceptable salt of the compound of formula (I) is selected from the group consisting of 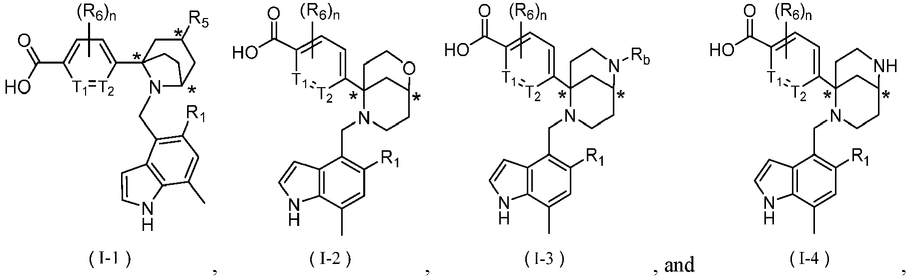 wherein
R₅ is selected from the group consisting of halogen, C₁₋₃ alkyl, and C₁₋₃ alkoxy, wherein the C₁₋₃ alkyl and the C₁₋₃ alkoxy are optionally substituted with 1, 2, or 3 R_{b};
R₁, R₆, R_{b}, T₁, T₂, and n are as defined above;
the carbon atom with "*" is a chiral carbon atom present in a form of a single (*R*) or (*S*) enantiomer or in a form enriched with one enantiomer.

In some embodiments of the present invention, the compound of formula (I-1) is not 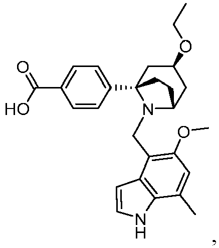 and the other variables are as defined herein.

In some embodiments of the present invention, the compound of formula (I) or the pharmaceutically acceptable salt of the compound of formula (I) is selected from the group consisting of 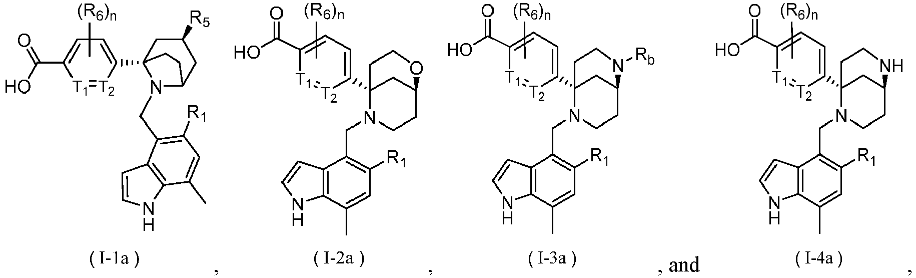 wherein
R₅ is selected from the group consisting of halogen, C₁₋₃ alkyl, and C₁₋₃ alkoxy, wherein the C₁₋₃ alkyl and the C₁₋₃ alkoxy are optionally substituted with 1, 2, or 3 R_{b}; R₁, R₆, R_{b}, T₁, T₂, and n are as defined herein.

Still some other embodiments of the present invention are derived from any combination of the variables.

In another aspect, the present invention provides the compound of formula (I) or the pharmaceutically acceptable salt thereof, 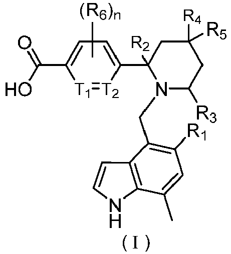 wherein
T₁ and T₂ are each independently selected from the group consisting of C, CH, and N;
R₁ is selected from the group consisting of C₁₋₃ alkoxy and C₁₋₃ alkyl, wherein the C₁₋₃ alkoxy and the C₁₋₃ alkyl are each independently optionally substituted with 1, 2, or 3 Rₐ;
R₃, R₄, and R₅ are each independently selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 R_{b}; R₂ and R₃, together with the atom linked thereto, form 3- to 6-membered heterocyclyl, wherein the 3- to 6-membered heterocyclyl is optionally substituted with 1, 2, or 3 R_{b};
alternatively, R₂ and R₄, together with the atom linked thereto, form 3- to 6-membered heterocyclyl, wherein the 3- to 6-membered heterocyclyl is optionally substituted with 1, 2, or 3 R_{b};
R_{b} is selected from the group consisting of H, halogen, CN, C₁₋₃ alkyl, and C₁₋₃ alkoxy, wherein the C₁₋₃ alkyl and the C₁₋₃ alkoxy are each independently optionally substituted with 1, 2, or 3 R_{c};
Rₐ and R_{c} are each independently selected from the group consisting of H, F, Cl, Br, and I;
R_{b} is selected from the group consisting of H, halogen, C₁₋₃ alkyl, C₁₋₃ alkoxy, -C(=O)-C₁₋₃ alkyl, and -S(=O)ₘ-C₁₋₃ alkyl, wherein the C₁₋₃ alkyl, the C₁₋₃ alkoxy, the -C(=O)-C₁₋₃ alkyl, and the -S(=O)ₘ-C₁₋₃ alkyl are each independently optionally substituted with 1, 2, or 3 R;
R is selected from the group consisting of H, F, Cl, Br, I, and OH;
n is selected from the group consisting of 0, 1, and 2;
m is selected from the group consisting of 0, 1, and 2.

In another aspect, the present invention provides the pharmaceutically acceptable salt of the compound of formula (I), 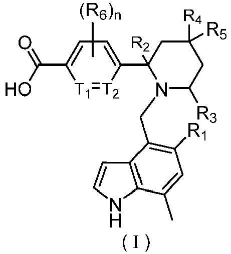 wherein
T₁ and T₂ are each independently selected from the group consisting of C, CH, and N;
R₁ is selected from the group consisting of C₁₋₃ alkoxy and C₁₋₃ alkyl, wherein the C₁₋₃ alkoxy and the C₁₋₃ alkyl are each independently optionally substituted with 1, 2, or 3 Rₐ;
R₃, R₄, and R₅ are each independently selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 R_{b}; R₂ and R₃, together with the atom linked thereto, form 3- to 6-membered heterocyclyl, wherein the 3- to 6-membered heterocyclyl is optionally substituted with 1, 2, or 3 R_{b};
alternatively, R₂ and R₄, together with the atom linked thereto, form 3- to 6-membered heterocyclyl, wherein the 3- to 6-membered heterocyclyl is optionally substituted with 1, 2, or 3 R_{b};
R_{b} is selected from the group consisting of H, halogen, CN, C₁₋₃ alkyl, and C₁₋₃ alkoxy, wherein the C₁₋₃ alkyl and the C₁₋₃ alkoxy are each independently optionally substituted with 1, 2, or 3 R_{c};
Rₐ and R_{c} are each independently selected from the group consisting of H, F, Cl, Br, and I;
R_{b} is selected from the group consisting of H, halogen, C₁₋₃ alkyl, C₁₋₃ alkoxy, -C(=O)-C₁₋₃ alkyl, and -S(=O)ₘ-C₁₋₃ alkyl, wherein the C₁₋₃ alkyl, the C₁₋₃ alkoxy, the -C(=O)-C₁₋₃ alkyl, and the -S(=O)ₘ-C₁₋₃ alkyl are each independently optionally substituted with 1, 2, or 3 R;
R is selected from the group consisting of H, F, Cl, Br, I, and OH;
n is selected from the group consisting of 0, 1, and 2;
m is selected from the group consisting of 0, 1, and 2.

In some embodiments of the present invention, the compound of formula (I) is not 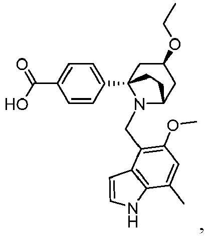 and the other variables are as defined herein.

In some embodiments of the present invention, R₁, R₂, and R₃ together with the atom linked thereto, R₂ and R₄ together with the atom linked thereto, Rₐ, R_{b}, R_{c}, R₆, R, the structural unit 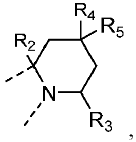 and the structural unit 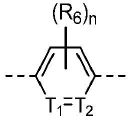 described above are as defined above.

In some embodiments of the present invention, R₃, R₄, and R₅ described above are each independently selected from the group consisting of H, F, CH₃, CH₂CH₃, CH₂CH₂CH₃, CH(CH₃)₂, OCH₃, OCH₂CH₃, OCH₂CH₂CH₃, and OCH(CH₃)₂, wherein the CH₃, the CH₂CH₃, the CH₂CH₂CH₃, the CH(CH₃)₂, the OCH₃, the OCH₂CH₃, the OCH₂CH₂CH₃, and the OCH(CH₃)₂ are each independently optionally substituted with 1, 2, or 3 R_{b}, and the other variables are as defined herein.

In some embodiments of the present invention, R₃, R₄, and R₅ described above are each independently selected from the group consisting of H, CH₃ CH₂F, CHF₂, CF₃, OCH₃, OCH₂F, OCHF₂, OCF₃, and OCH₂CH₃, and the other variables are as defined herein.

In some embodiments of the present invention, R₃, R₄, and R₅ described above are each independently selected from the group consisting of H and OCH₂CH₃, and the other variables are as defined herein.

The present invention further provides compounds as shown below or pharmaceutically acceptable salts thereof, 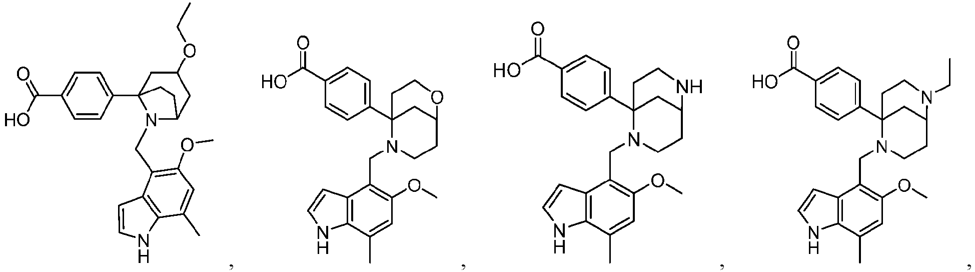 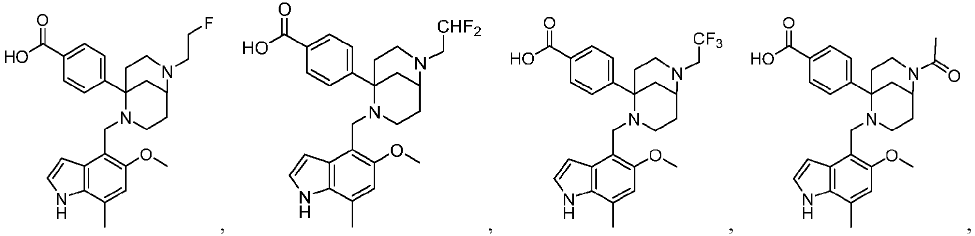 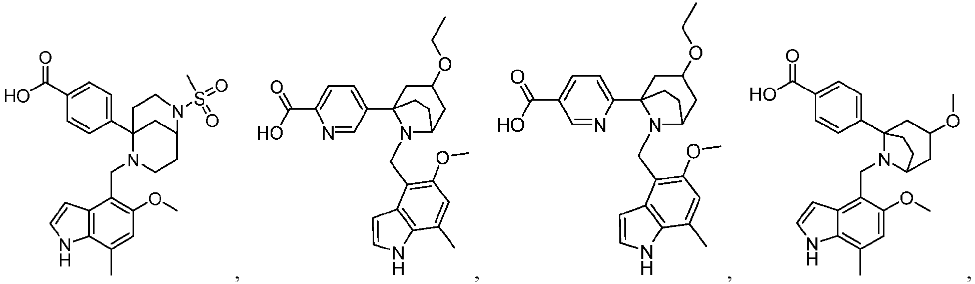 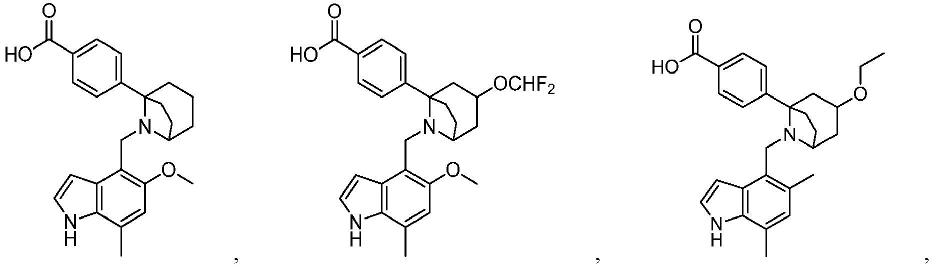 and

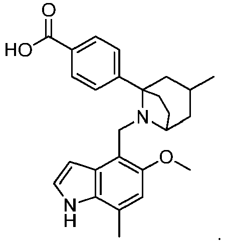

The present invention further provides pharmaceutically acceptable salts of compounds as shown below

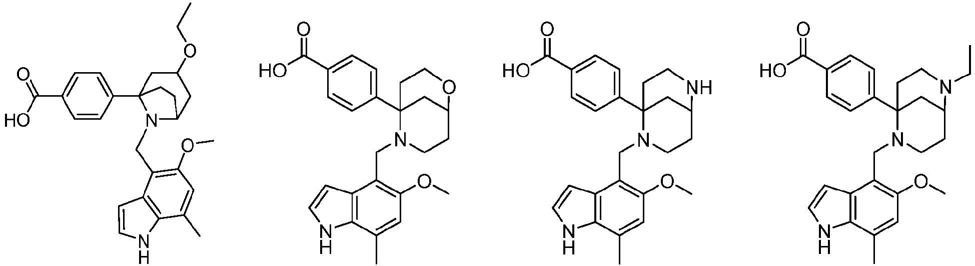

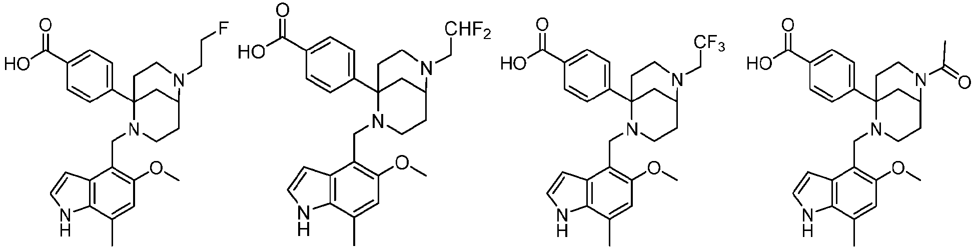

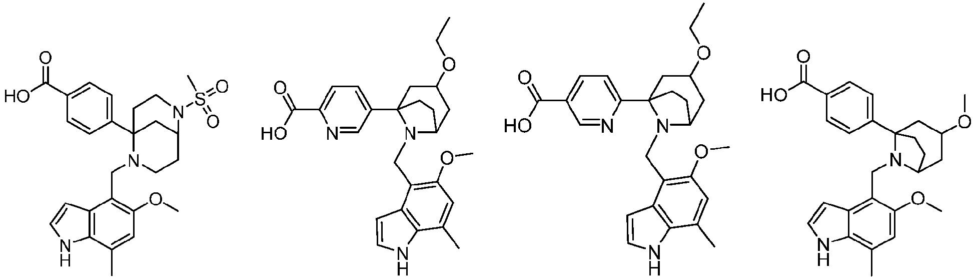

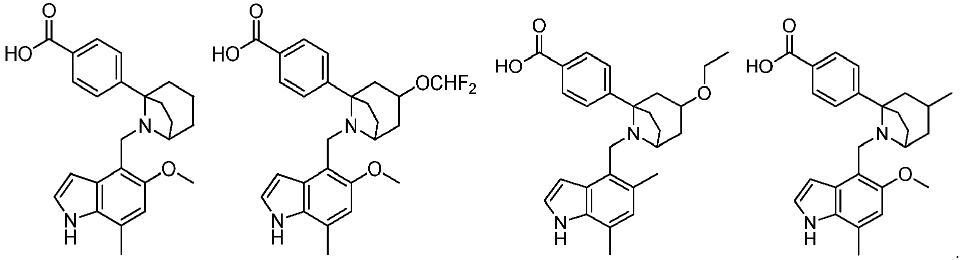

The present invention further provides compounds as shown below or pharmaceutically acceptable salts thereof:

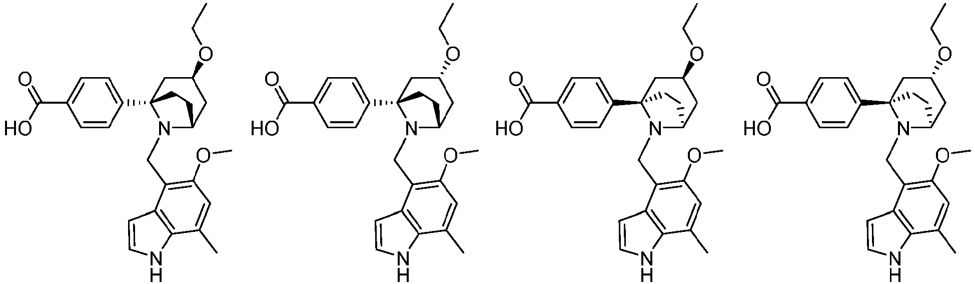

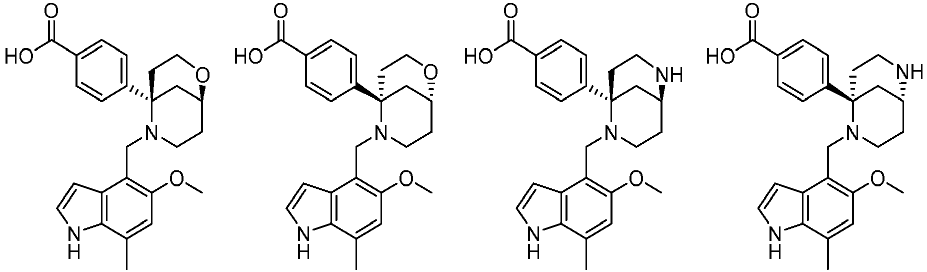

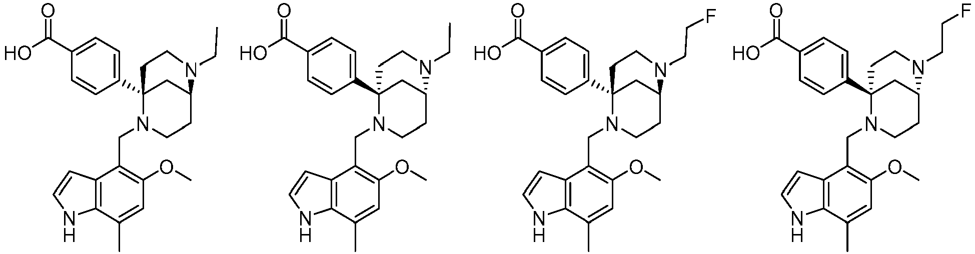

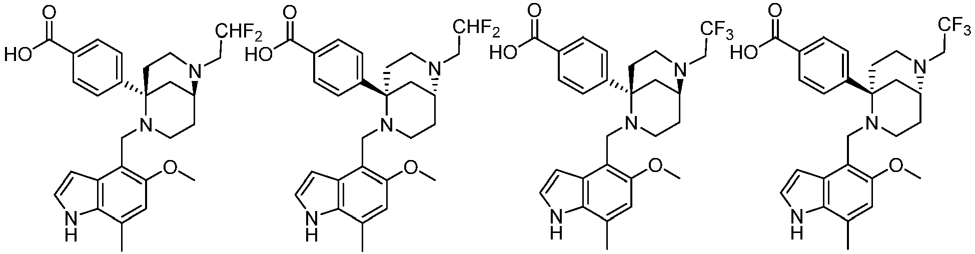

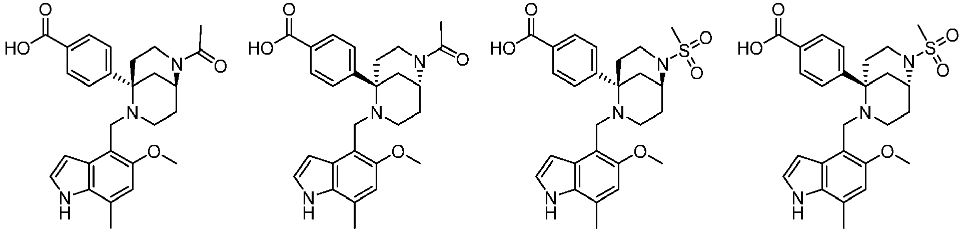

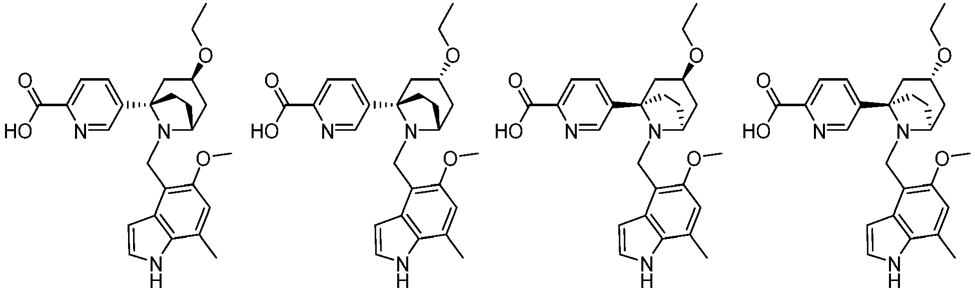

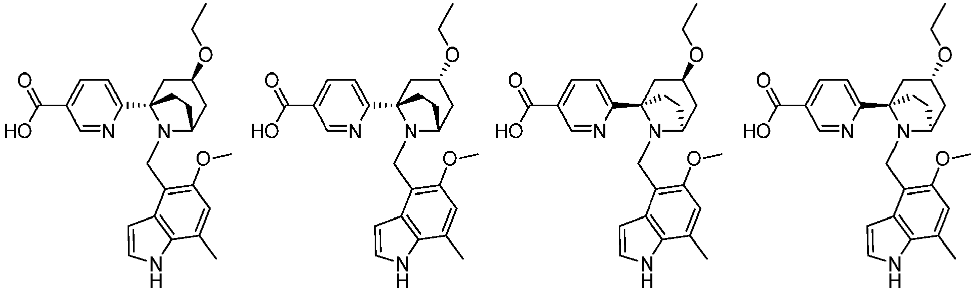

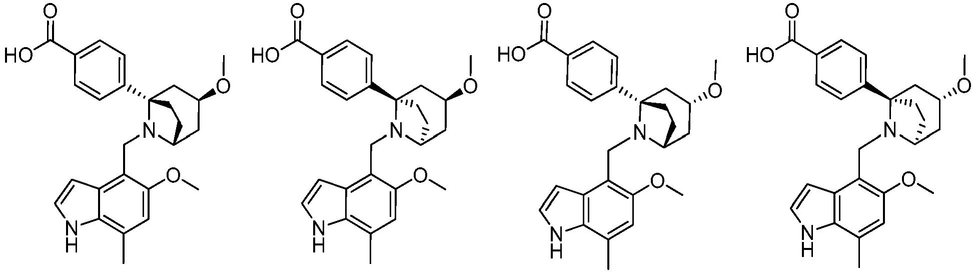

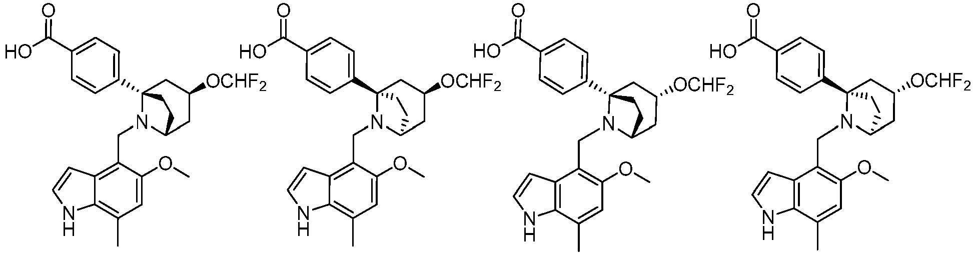

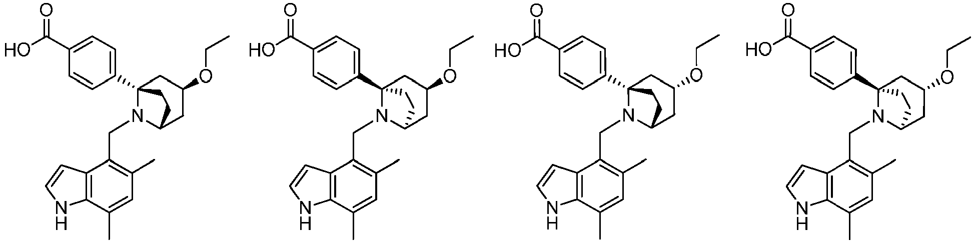

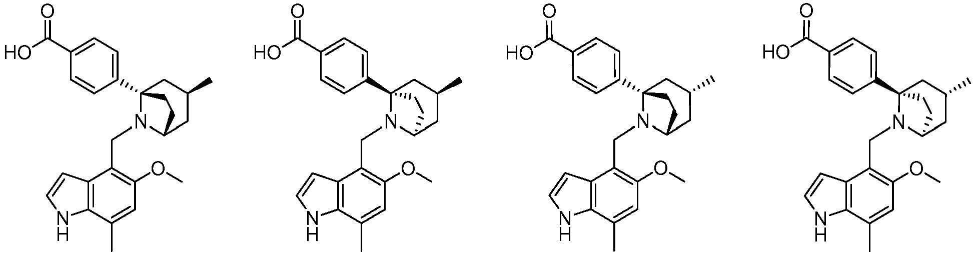

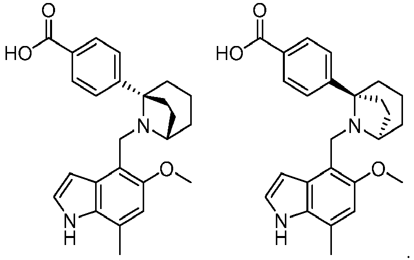

The present invention further provides pharmaceutically acceptable salts of compounds as shown below:

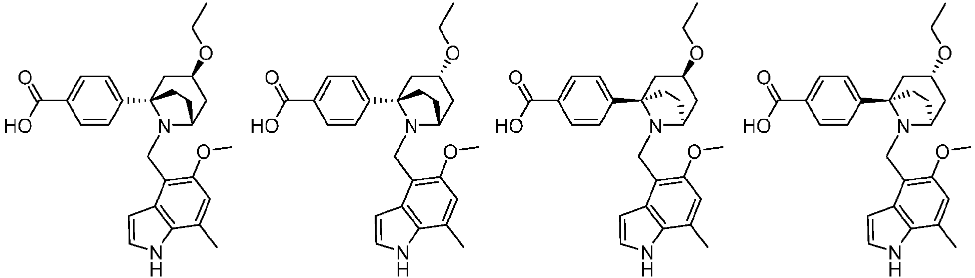

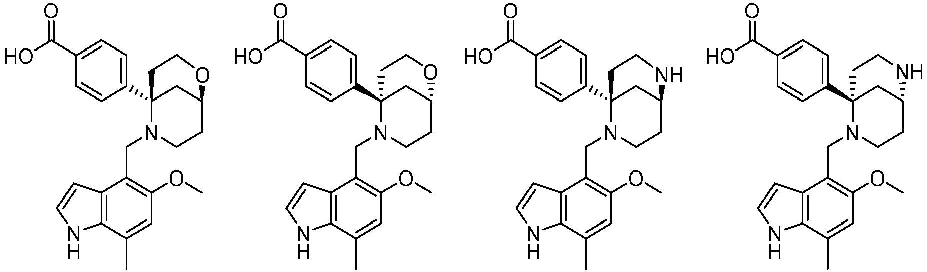

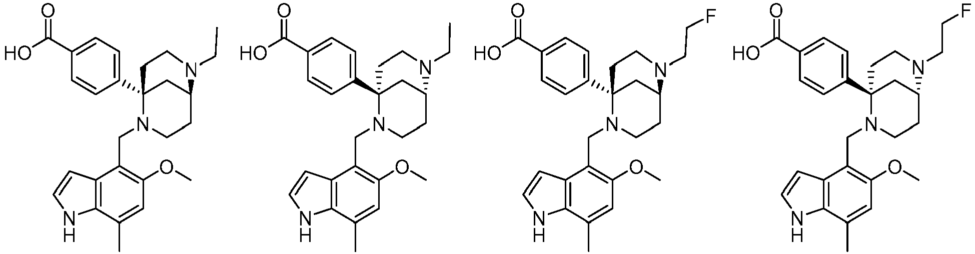

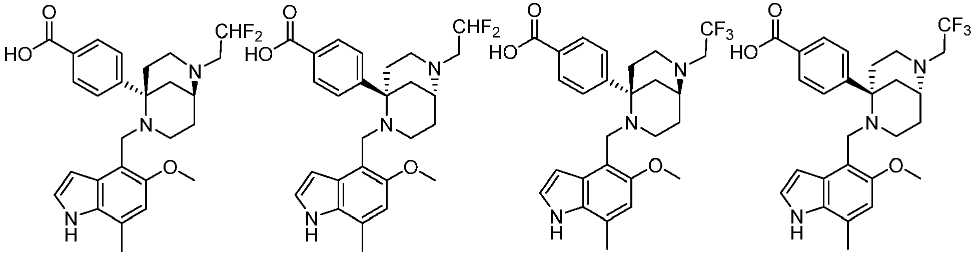

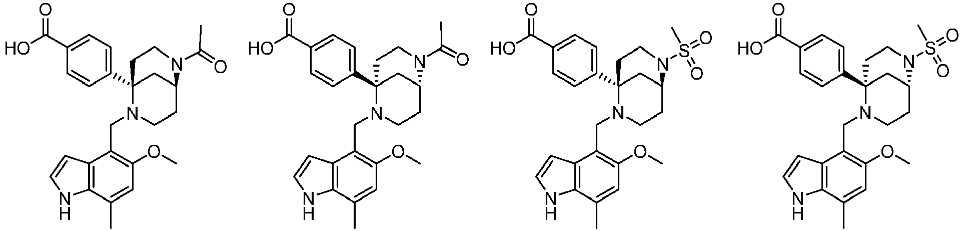

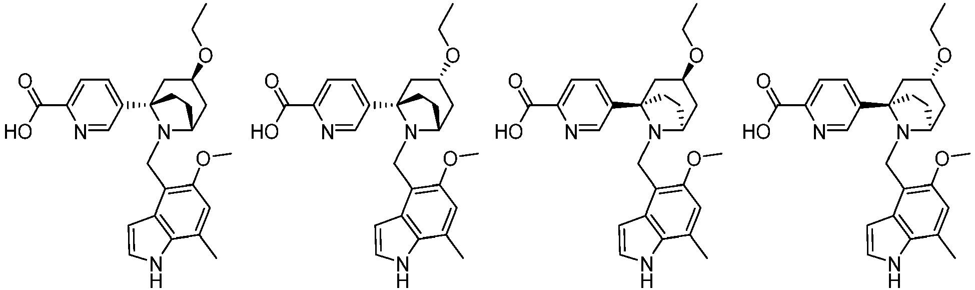

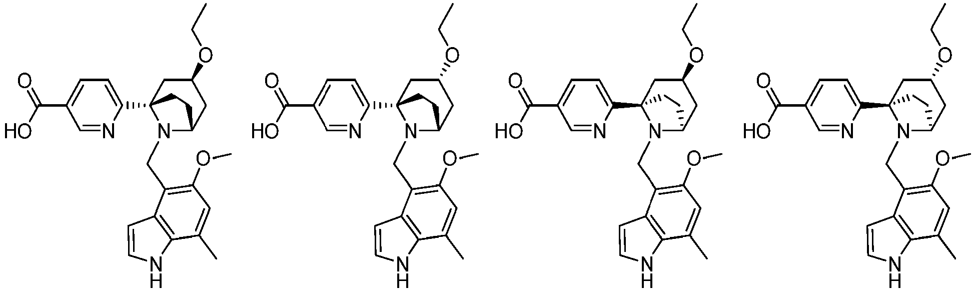

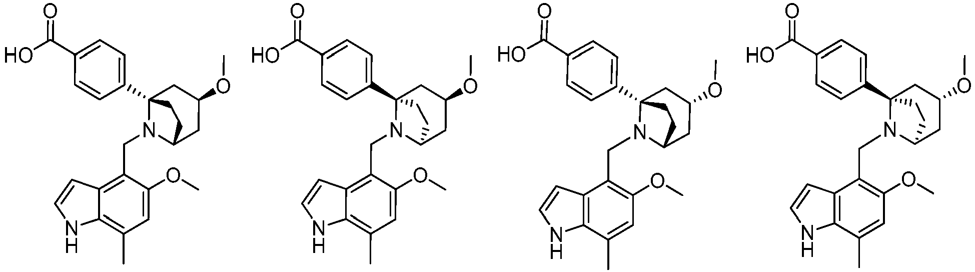

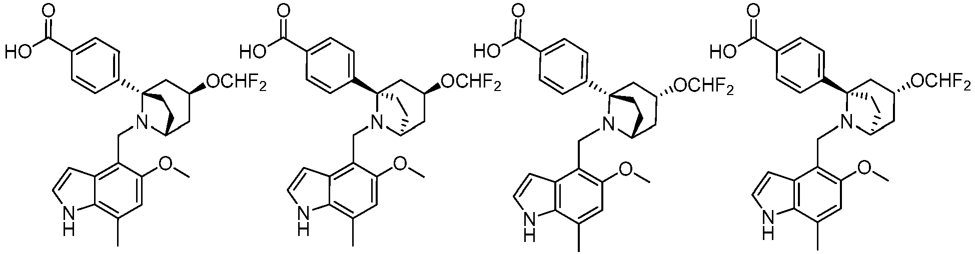

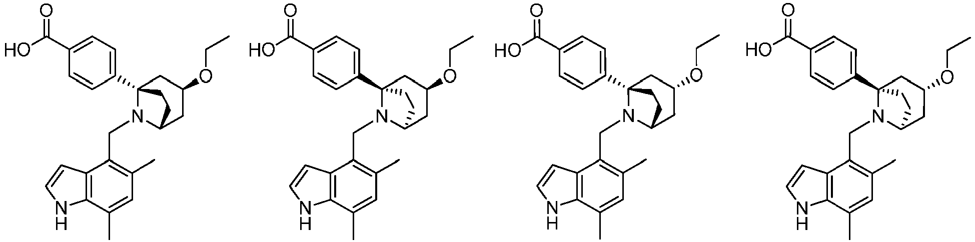

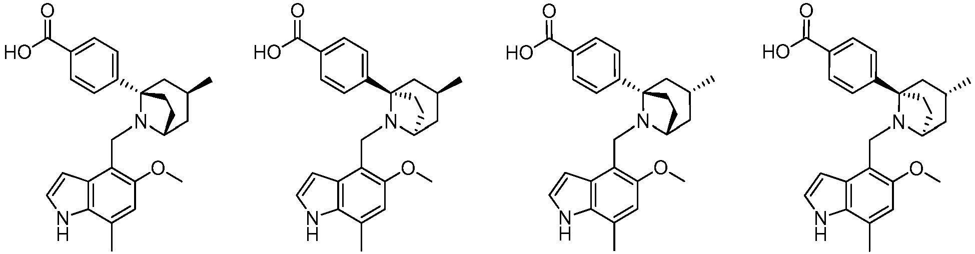

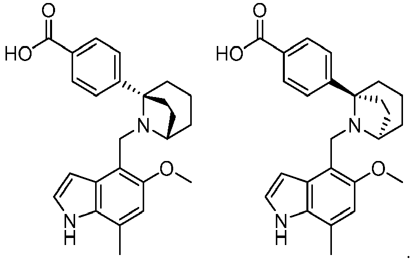

The present invention further provides a pharmaceutical composition comprising a therapeutically effective amount of the compound or the pharmaceutically acceptable salt thereof disclosed herein. Further, the pharmaceutical composition also comprises a pharmaceutically acceptable carrier.

The present invention further provides use of the compound or the pharmaceutically acceptable salt thereof disclosed herein in preparing a medicament for use in treating a disease related to complement factor B.

The present invention further provides a method for treating a disease related to complement factor B, comprising administering to a mammal, preferably a human, in need of such treatment a therapeutically effective amount of the compound or the pharmaceutically acceptable salt thereof disclosed herein.

The present invention further provides use of the compound or the pharmaceutically acceptable salt thereof disclosed herein in treating a disease related to complement factor B.

The present invention further provides the compound or the pharmaceutically acceptable salt thereof disclosed herein for treating a disease related to complement factor B.

In some embodiments of the present invention, the disease related to complement factor B is selected from the group consisting of inflammatory disorders and autoimmune diseases.

In some embodiments of the present invention, the pharmaceutically acceptable salt is selected from the group consisting of pharmaceutically acceptable acid addition salts. In some embodiments of the present invention, the pharmaceutically acceptable salt is selected from the group consisting of pharmaceutically acceptable inorganic acid salts, organic acid salts, and amino acid salts.

In some embodiments of the present invention, examples of the pharmaceutically acceptable salt include inorganic acid salts and organic acid salts. The inorganic acid includes, for example, hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid, phosphorous acid, and the like. The organic acid includes, for example, formic acid, acetic acid, propionic acid, trifluoroacetic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, methanesulfonic acid, and the like. Also included are salts of amino acids (e.g., arginine) and salts of organic acids such as glucuronic acid.

In some embodiments of the present invention, the pharmaceutically acceptable salt is selected from the group consisting of hydrochloride, hydrobromide, nitrate, carbonate, bicarbonate, phosphate, monohydrogen phosphate, dihydrogen phosphate, sulfate, bisulfate, hydroiodide, phosphite, formate salt, acetate, propionate, trifluoroacetate salt, isobutyrate, maleate, malonate, benzoate, succinate, suberate, fumarate, lactate, mandelate, phthalate, benzenesulfonate, p-toluenesulfonate, citrate, tartrate, methanesulfonate, arginine salt, and glucuronate.

In some embodiments of the present invention, the pharmaceutically acceptable salt is selected from the group consisting of hydrochloride, formate salt, and trifluoroacetate salt.

In some embodiments of the present invention, the pharmaceutically acceptable salt is selected from the group consisting of formate salt.

The present invention further provides a compound of formula (P) and a pharmaceutically acceptable salt thereof, 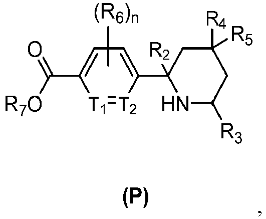 wherein
T₁ and T₂ are each independently selected from the group consisting of C, CH, and N;
R₅ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
when R₂ and R₃, together with the atom linked thereto, form 3- to 6-membered heterocyclyl, wherein the 3- to 6-membered heterocyclyl is optionally substituted with 1, 2, or 3 R_{b}, R₄ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
alternatively, when R₂ and R₄, together with the atom linked thereto, form 3- to 6-membered heterocyclyl, wherein the 3- to 6-membered heterocyclyl is optionally substituted with 1, 2, or 3 R_{b}, R₃ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
R₆ is selected from the group consisting of halogen, CN, C₁₋₃ alkyl, and C₁₋₃ alkoxy, wherein the C₁₋₃ alkyl and the C₁₋₃ alkoxy are each independently optionally substituted with 1, 2, or 3 R_{c}:
R₇ is selected from the group consisting of H and C₁₋₄ alkyl;
each R_{c} is independently selected from the group consisting of F, Cl, Br, and I;
each R_{b} is independently selected from the group consisting of halogen, C₁₋₃ alkyl, C₁₋₃ alkoxy, -C(=O)-C₁₋₃ alkyl, -S(=O)ₘ-C₁₋₃ alkyl, and N atom-protecting groups, wherein the C₁₋₃ alkyl, the C₁₋₃ alkoxy, the -C(=O)-C₁₋₃ alkyl, and the -S(=O)ₘ-C₁₋₃ alkyl are each independently optionally substituted with 1, 2, or 3 R;
each R is independently selected from the group consisting of F, Cl, Br, I, and OH;
n is selected from the group consisting of 0, 1, and 2;
m is selected from the group consisting of 0, 1, and 2.

In some embodiments of the present invention, R₂, R₃, R₄, R₅, R₆, R_{b}, R_{c}, R, T₁, T₂, n, and m in the compound of formula (P) are as defined above for the compound of formula (I).

In some embodiments of the present invention, R₇ in the compound of formula (P) is selected from the group consisting of H and methyl.

In some embodiments of the present invention, each R_{b} in the compound of formula (P) is independently selected from the group consisting of C₁₋₃ alkyl and N atom-protecting groups selected from the group consisting of benzyl.

In some embodiments of the present invention, each R_{b} in the compound of formula (P) is independently selected from the group consisting of ethyl and benzyl.

In some embodiments of the present invention, the compound of formula (P) and the pharmaceutically acceptable salt thereof are selected from the group consisting of:

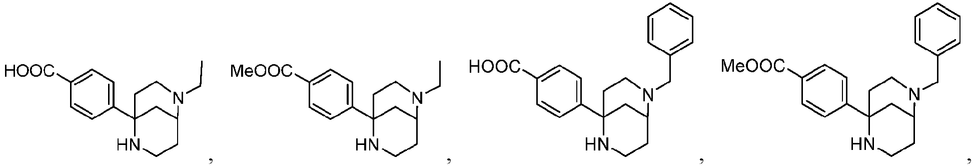

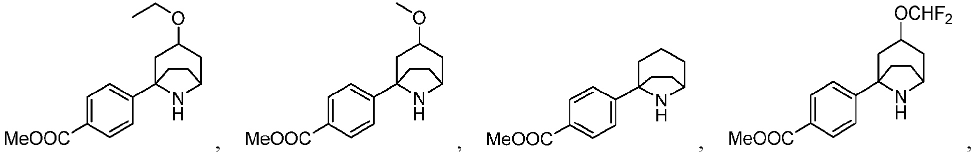

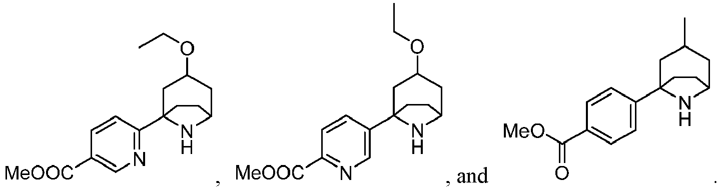

The present invention further provides use of the compound of formula (P) in preparing the compound of formula (I) described above.

The present invention further provides a method for preparing the compound of formula (I) described above, comprising: reacting the compound of formula (P) with a compound of formula (Q) to give a compound of formula (W); subjecting the compound of formula (W) to deprotection reaction to give the compound of formula (I), 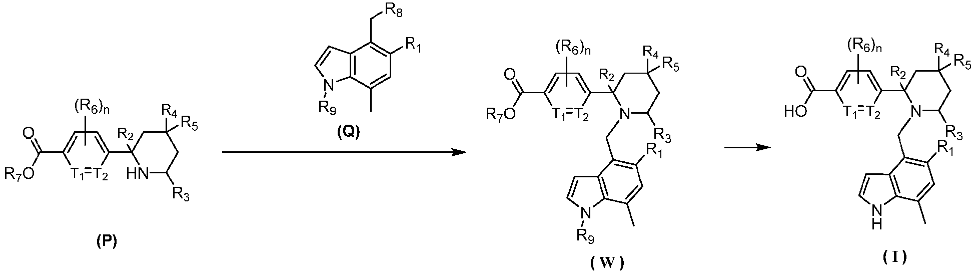
wherein R₉ is selected from the group consisting of N atom-protecting groups;
wherein R₂, R₃, R₄, R₅, R₆, R₇, R_{b}, R_{c}, R, T₁, T₂, n, and m in the compound of formula (P) are as described above for the compound of formula (P);
wherein R₁ and Rₐ in the compound of formula (Q) are as described above for the compound of formula (I); R₈ is selected from the group consisting of F, Cl, Br, I, and OH. In some embodiments of the present invention, R₈ is selected from the group consisting of Cl and OH,
wherein R₁, R₂, R₃, R₄, R₅, R₆, Rₐ, R_{b}, R_{c}, R, T₁, T₂, n, and m in the compound of formula (W) are as described above for the compound of formula (I); R₇ is selected from the group consisting of H and C₁₋₄ alkyl;
wherein R₁, R₂, R₃, R₄, R₅, R₆, Rₐ, R_{b}, R_{c}, R, T₁, T₂, n, and m in the compound of formula (I) are as described above for the compound of formula (I).

The present invention further provides a method for preparing the compound of formula (I-3) described above, comprising: reacting the compound of formula (P-1) with a compound of formula (Q) to give a compound of formula (V); subjecting the compound of formula (V) to N-substitution reaction and deprotection reaction respectively to give the compound of formula (I-3), 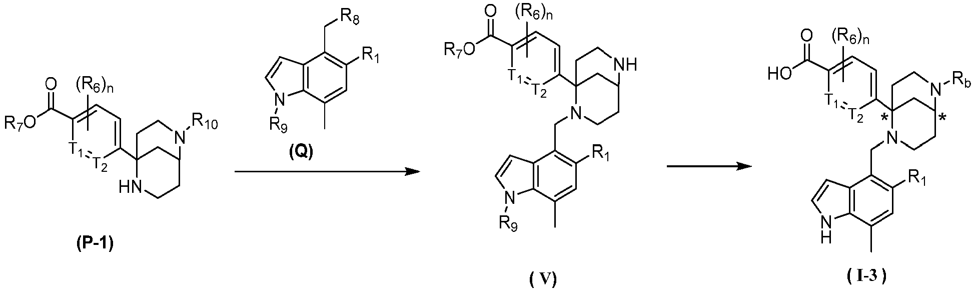
wherein R₉ and R₁₀ are each independently selected from the group consisting of N atom-protecting groups;
wherein R₆, R₇, T₁, T₂, and n in the compound of formula (P-1) are as described above for the compound of formula (P);
wherein R₁ and Rₐ in the compound of formula (Q) are as described above for the compound of formula (I); R₈ is selected from the group consisting of F, Cl, Br, I, and OH. In some embodiments of the present invention, R₈ is selected from the group consisting of Cl and OH,
wherein R₁, R₆, Rₐ, T₁, T₂, and n in the compound of formula (V) are as described above for the compound of formula (I); R₇ is selected from the group consisting of H and C₁₋₄ alkyl;
wherein R₁, R₆, Rₐ, R_{b}, T₁, T₂, n, and m in the compound of formula (I-3) are as described above for the compound of formula (I);
wherein the carbon atom with "*" is a chiral carbon atom present in a form of a single (R) or (5) enantiomer or in a form enriched with one enantiomer.

In some embodiments of the present invention, the N atom-protecting group is selected from the group consisting of benzyloxycarbonyl (Cbz), 2,2,2-trichloroethoxycarbonyl (Troc), 2-(trimethylsilyl)ethoxycarbonyl (Teoc), 2-(4-trifluoromethylbenzenesulfonyl)ethoxycarbonyl (Tsc), tert-butyloxycarbonyl (Boc), 1-adamantyloxycarbonyl (Adoc), 2-adamantylcarbonyl (2-Adoc), 2,4-dimethylpent-3-yloxycarbonyl (Doc), cyclohexyloxycarbonyl (Hoc), 1,1-dimethyl-2,2,2-trichloroethoxycarbonyl (TcBoc), vinyl, 2-chloroethyl, 2-benzenesulfonylethyl, p-nitrobenzenesulfonyl, p-methylbenzenesulfonyl, benzenesulfonyl, methanesulfonyl, allyl, benzyl, 2-nitrobenzyl, 4-nitrobenzyl, diphenyl-4-pyridinylmethyl, *N*,*N*'-dimethylhydrazino, methoxymethyl, tert-butoxymethyl (Bum), benzyloxymethyl (Bom), 2-tetrahydropyranyl (THP), tri(C₁₋₄ alkyl)silyl, 1,1-diethoxymethyl, 2-(trimethylsilyl)ethoxymethyl (SEM), or N-pivaloyloxymethyl (POM).

In some embodiments of the present invention, R₇ is selected from the group consisting of H and methyl. In some embodiments of the present invention, R₉ is selected from the group consisting of Boc. In some embodiments of the present invention, R₁₀ is selected from the group consisting of benzyl.

The present invention further provides compounds as shown below or salts thereof:

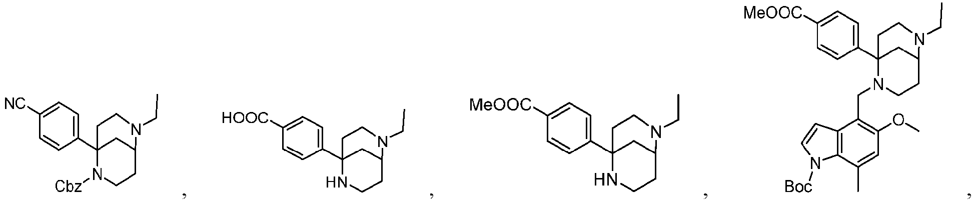

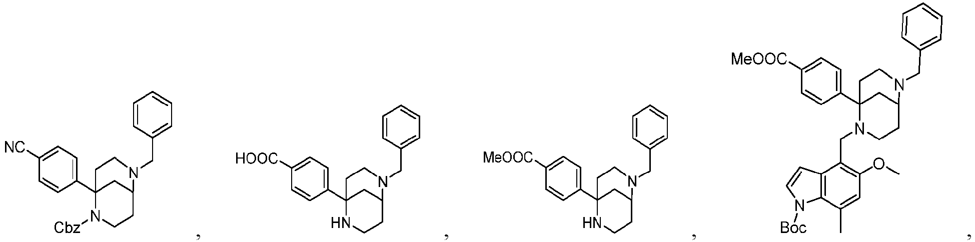

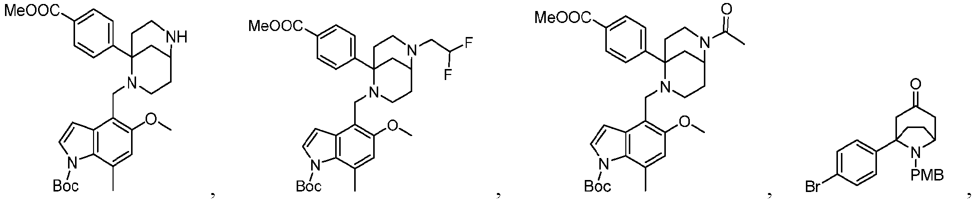

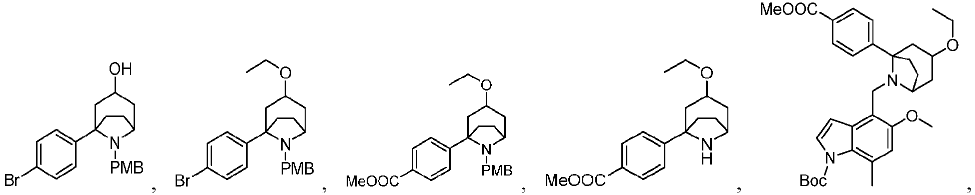

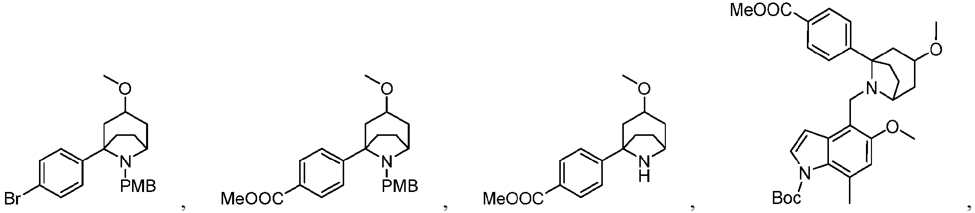

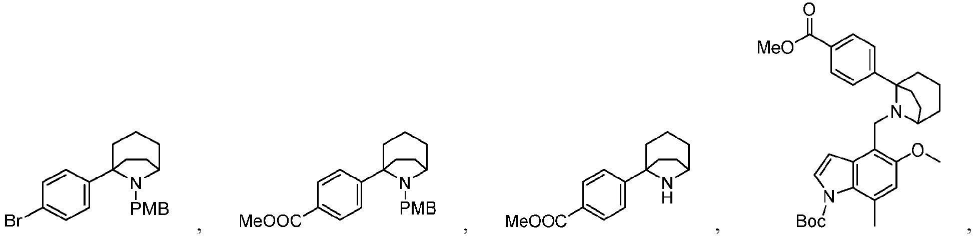

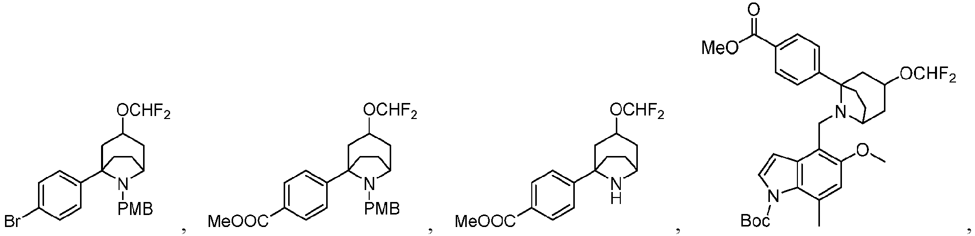

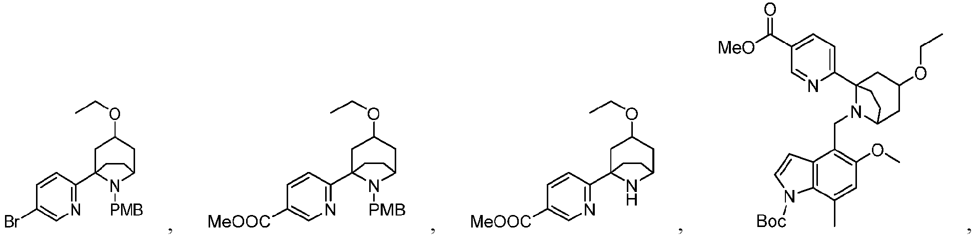

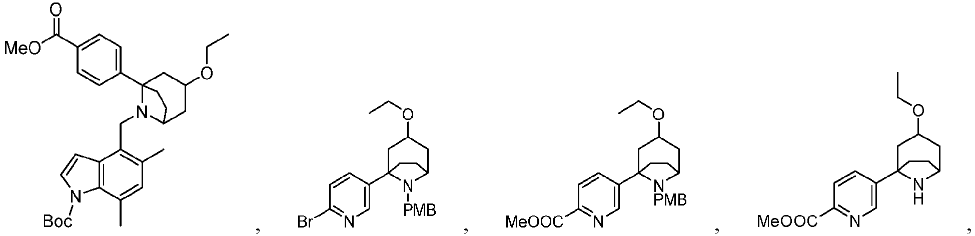

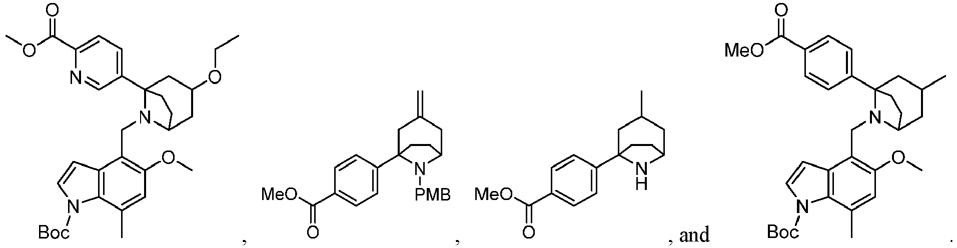

### Technical Effects

The compound disclosed herein, including the example compounds, has significant inhibitory activity against the activation of human serum alternative pathway, has significant binding activity to human complement Factor B protein, and also has a good *in-vivo* drug effect and pharmacokinetic properties. The compound disclosed herein can be developed into a novel small-molecule inhibitor of complement system Factor B.

### Definitions

Unless otherwise stated, the following terms and phrases used herein are intended to have the following meanings. A particular term or phrase, unless otherwise specifically defined, should not be considered as indefinite or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound disclosed herein, which is prepared from the compound having particular substituents disclosed herein and a relatively nontoxic acid or base. When the compound disclosed herein contains a relatively acidic functional group, a base addition salt can be given by contacting such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound disclosed herein contains a relatively basic functional group, an acid addition salt can be obtained by contacting such a compound with a sufficient amount of an acid in a pure solution or a suitable inert solvent. Examples of the pharmaceutically acceptable acid addition salt include inorganic acid salts and organic acid salts. The inorganic acid includes, for example, hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid, phosphorous acid, and the like. The organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, methanesulfonic acid, and the like. Also included are salts of amino acids (e.g., arginine) and salts of organic acids such as glucuronic acid. Certain specific compounds disclosed herein contain both basic and acidic functional groups that allow the compounds to be converted into either base or acid addition salts.

The pharmaceutically acceptable salts disclosed herein can be synthesized from a parent compound having an acidic or basic group by conventional chemical methods. In general, such salts are prepared by subjecting the free acid or base form of the compound to a reaction with a stoichiometric amount of the appropriate base or acid in water or an organic solvent or a mixture thereof.

The compound disclosed herein may demonstrate a specific geometric isomerism or stereoisomerism. All such compounds are contemplated herein, including *cis* and *trans* isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereomer enriched mixture, all of which are encompassed within the scope of the present invention. The substituents such as alkyl may have an additional asymmetric carbon atom. All these isomers and mixtures thereof are encompassed within the scope of the present invention.

Unless otherwise stated, the term "enantiomer" or "optical isomer" refers to stereoisomers that are mirror images of each other.

Unless otherwise stated, the term *"cis-trans* isomer" or "geometric isomer" results from the inability of a single bond of a ring carbon atom or a double bond to rotate freely.

Unless otherwise stated, the term "diastereoisomer" refers to stereoisomers in which molecules each have two or more chiral centers and are not mirror images of each other.

Unless otherwise stated, "(+)" stands for dextrorotation, "(-)" stands for levorotation, and "(±)" stands for racemization.

Unless otherwise stated, the absolute configuration of a stereogenic center is represented by a wedged solid bond ( ) and a wedged dashed bond ( 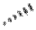 ), and the relative configuration of a stereogenic center is represented by a straight solid bond ( 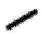 ) and a straight dashed bond ( 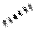 ). A wavy line ( 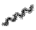 ) represents a wedged solid bond ( 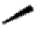 ) or a wedged dashed bond ( 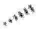 ), or a wavy line ( 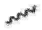 ) represents a straight solid bond ( 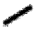 ) and a straight dashed bond ( 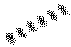 ).

Unless otherwise stated, when a double bond structure such as a carbon-carbon double bond, a carbon-nitrogen double bond, and a nitrogen-nitrogen double bond is present in the compound, and each atom on the double bond is linked to two different substituents (in the double bond including a nitrogen atom, a lone pair of electrons on the nitrogen atom is regarded as a substituent to which the nitrogen atom is linked), if the atom on the double bond of the compound and its substituents are linked using a wavy line ( 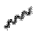 ), it means that the compound exists in the form of a (*Z*)-type isomer, an (E)-type isomer, or a mixture of the two isomers. For example, the following formula (A) represents that the compound exists in the form of a single isomer of formula (A-1) or formula (A-2) or in the form of a mixture of both isomers of formula (A-1) and formula (A-2); the following formula (B) represents that the compound exists in the form of a single isomer of formula (B-1) or formula (B-2) or in the form of a mixture of both isomers of formula (B-1) and formula (B-2); and the following formula (C) represents that the compound exists in the form of a single isomer of formula (C-1) or formula (C-2) or in the form of a mixture of both isomers of formula (C-1) and formula (C-2).

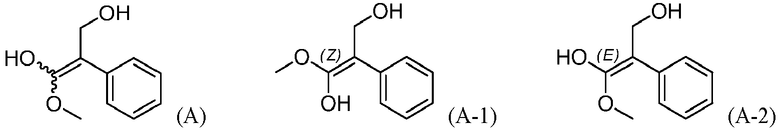

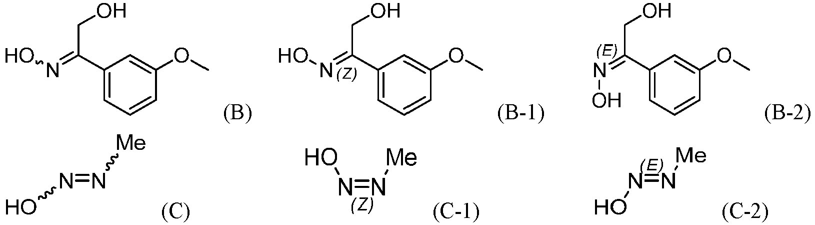

Unless otherwise stated, the term "tautomer" or "tautomeric form" means that different functional isomers are in dynamic equilibrium at room temperature and can be rapidly converted into each other. If tautomers are possible (e.g., in solution), the chemical equilibrium of the tautomers can be achieved. For example, a proton tautomer, also known as a prototropic tautomer, includes the interconversion by proton transfer, such as keto-enol isomerization and imine-enamine isomerization. A valence isomer includes the interconversion by recombination of some bonding electrons. A specific example of the keto-enol tautomerization is the interconversion between tautomers pentane-2,4-dione and 4-hydroxypent-3-en-2-one.

Unless otherwise stated, the term "enriched with one isomer", "isomer enriched", "enriched with one enantiomer", or "enantiomer enriched" means that the content of one of the isomers or enantiomers is less than 100% and more than or equal to 60%, or more than or equal to 70%, or more than or equal to 80%, or more than or equal to 90%, or more than or equal to 95%, or more than or equal to 96%, or more than or equal to 97%, or more than or equal to 98%, or more than or equal to 99%, or more than or equal to 99.5%, or more than or equal to 99.6%, or more than or equal to 99.7%, or more than or equal to 99.8%, or more than or equal to 99.9%.

Unless otherwise stated, the term "isomeric excess" or "enantiomeric excess" refers to the difference between the relative percentages of two isomers or enantiomers. For example, if the content of one of the isomers or enantiomers is 90% and the content of the other isomer or enantiomer is 10%, the isomeric or enantiomeric excess (ee value) is 80%.

The compound disclosed herein may contain an unnatural proportion of atomic isotope at one or more of the atoms that constitute the compound. For example, the compound may be labeled with a radioisotope, such as tritium (³H), iodine-125 (¹²⁵ I), or C-14 (¹⁴C). For another example, hydrogen can be substituted by deuterium to form a deuterated drug, and the bond formed by deuterium and carbon is stronger than that formed by common hydrogen and carbon. Compared with an un-deuterated drug, the deuterated drug has the advantages of reduced toxic side effects, increased stability, enhanced efficacy, prolonged biological half-life and the like. All isotopic variations of the compound disclosed herein, whether radioactive or not, are encompassed within the scope of the present invention.

The term "optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted by substituents, wherein the substituents may include deuterium and hydrogen variants, as long as the valence of the specific atom is normal and the substituted compound is stable. When the substituent is oxygen (i.e., =O), it means that two hydrogen atoms are substituted. Substitution by oxygen does not occur on aromatic groups. The term "optionally substituted" means that an atom can be or cannot be substituted by a substituent. Unless otherwise specified, the type and number of the substituent may be arbitrary as long as being chemically achievable.

When any variable (e.g., R) occurs once or more in the constitution or structure of a compound, the definition of the variable in each case is independent. Thus, for example, if a group is substituted with 0-2 R, the group can be optionally substituted with up to two R, and the definition of R in each case is independent. Furthermore, a combination of the substituent and/or the variant thereof is permissible only if the combination can result in a stable compound.

When the number of a connecting group is 0, for example, -(CRR)₀-, it means that the connecting group is a single bond.

When one of the variables is selected from the group consisting of a single bond, it means that the two groups which it connects are connected directly. For example, in A-L-Z, when L represents a single bond, it means that the structure is actually A-Z.

When a substituent is absent, it means that the substituent does not exist. For example, when X is absent in A-X, the structure of A-X is actually A.

When it is not specified by which atom the listed substituent is linked to the group to be substituted, the substituent can be linked via any atom of the group. For example, pyridinyl as a substituent can be linked to the group to be substituted through any carbon atom on the pyridine ring.

When the direction for connection of the listed connecting group is not specified, the direction for connection is arbitrary. For example, when the connecting group L contained in 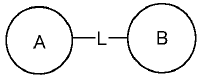 is -M-W-, -M-W- can either connect ring A and ring B in a direction same as left-to-right reading order to form 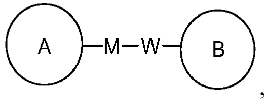 or connect ring A and ring B in a direction opposite to the left-to-right reading order to form 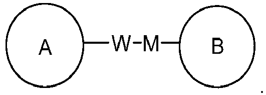 A combination of the connecting group, the substituent and/or the variant thereof is permissible only if the combination can result in a stable compound.

Unless otherwise specified, when a group has one or more connectable sites, any one or more sites of the group may be connected to other groups by chemical bonds. When there is no designated connecting mode for a chemical bond and H atoms are present at a connectable site, the number of the H atoms at the connectable site is correspondingly reduced based on the number of the connected chemical bonds, and a number of groups corresponding to the valence number are thus formed. The chemical bond that connects the site and another group may be represented by a straight solid bond ( 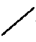 ), a straight dashed bond ( 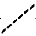 ), or a wavy line ( 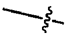 ). For example, the straight solid bond in -OCH₃ refers to being connected to another group via the oxygen atom in the group; the straight dashed bond in 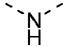 refers to being connected to another group via two ends of the nitrogen atom in the group; the wavy line in 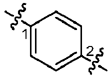 refers to being connected to another group via the carbon atoms at positions 1 and 2 in the phenyl group; 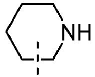 means that any connectable site on the piperidinyl can be connected to another group via one chemical bond in at least 4 connecting modes: 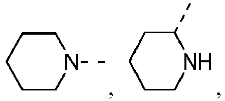 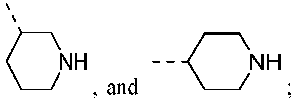 even if -N- is connected with an H atom, 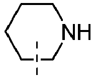 includes the connection mode of 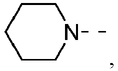 but when one chemical bond is connected to a site, the number of H at that site is correspondingly reduced by one and a monovalent piperidinyl is thus formed.

When a variable (e.g., R) is substituted on one of the rings of a polycyclic system, unless otherwise specified, the variable (e.g., R) is designated as substituted on that ring but not on the other rings of the polycyclic system. For example, 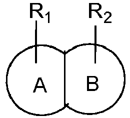 means that ring A is fused with ring B, the substituent R₁ is a substituent for ring A, the substituent R₂ is a substituent for ring B, and 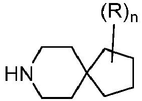 means that the five-membered ring in the spiro ring system is substituted with n R.

Unless otherwise specified, the term "halo" or "halogen", by itself or as part of another substituent, refers to a fluorine, chlorine, bromine, or iodine atom.

Unless otherwise specified, the term "C₁₋₃ alkyl" refers to a linear or branched saturated hydrocarbon group consisting of 1 to 3 carbon atoms. The C₁₋₃ alkyl includes, but is not limited to, C₁₋₂ and C₂₋₃ alkyl, and the like, and may be monovalent (e.g., methyl), divalent (e.g., methylene) or polyvalent (e.g., methenyl). Examples of C₁₋₃ alkyl include, but are not limited to, methyl (Me), ethyl (Et), propyl (including n-propyl and isopropyl), and the like.

Unless otherwise specified, the term "C₁₋₄ alkyl" refers to a linear or branched saturated hydrocarbon group consisting of 1 to 4 carbon atoms. The C₁₋₄ alkyl includes, but is not limited to, C₁₋₂ alkyl, C₁₋₃ alkyl, C₂₋₃ alkyl, and the like, and may be monovalent (e.g., methyl), divalent (e.g., methylene) or polyvalent (e.g., methenyl). Examples of C₁₋₄ alkyl include, but are not limited to, methyl (Me), ethyl (Et), propyl (including n-propyl and isopropyl), butyl (including n-butyl, isobutyl, s-butyl, t-butyl), and the like.

Unless otherwise specified, the term "C₁₋₃ alkoxy" refers to those alkyl groups that each contain 1 to 3 carbon atoms and are linked to the rest part of the molecule via an oxygen atom. The C₁₋₃ alkoxy includes, but is not limited to, C₁₋₂, C₂₋₃, C₃ and C₂ alkoxy, and the like. Examples of C₁₋₃ alkoxy include, but are not limited to, methoxy, ethoxy, propoxy (including *n*-propoxy and isopropoxy), and the like.

Unless otherwise specified, the term "3- to 6-membered heterocyclyl", by itself or in combination with other terms, refers to a saturated or partially unsaturated monocyclic group consisting of 3 to 6 ring atoms, of which 1, 2, 3, or 4 are heteroatoms independently selected from the group consisting of O, S, and N, and the rest are carbon atoms, wherein the nitrogen atom is optionally quaternized, and the nitrogen and sulfur heteroatoms can be optionally oxidized (i.e., NO and S(O)ₚ, wherein p is 1 or 2). Furthermore, with respect to the "3- to 6-membered heterocyclyl", a heteroatom may occupy the position where the heterocyclyl is connected to the rest of the molecule. The 3- to 6-membered heterocyclyl includes 4- to 6-membered, 5- to 6-membered, 4-membered, 5-membered, 6-membered heterocyclyl, and the like. Examples of 3- to 6-membered heterocyclyl include, but are not limited to, aza-cyclobutyl, oxa-cyclobutyl, thietanyl, pyrrolidinyl, pyrazolidinyl, imidazolidinyl, tetrahydrothienyl (including tetrahydrothien-2-yl, tetrahydrothien-3-yl, and the like), tetrahydrofuranyl (including tetrahydrofuran-2-yl, and the like), tetrahydropyranyl, piperidinyl (including 1-piperidinyl, 2-piperidinyl, 3-piperidinyl, and the like), piperazinyl (including 1-piperazinyl, 2-piperazinyl, and the like), morpholinyl (including 3-morpholinyl, 4-morpholinyl, and the like), dioxanyl, dithianyl, isoxazolidinyl, isothiazolidinyl, 1,2-oxazinyl, 1,2-thiazinyl, hexahydropyridazinyl, 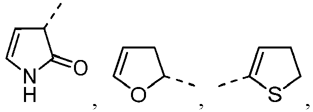 or the like.

Unless otherwise specified, Cₙ₋ₙ₊ₘ or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁ and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc. Similarly, n- to n+m-membered represents that the number of atoms on the ring is n to n+m. For example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. n- to n+m-membered also represents any range within n to n+m. For example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, and the like.

The term "protecting group" includes, but is not limited to, "amino protecting group", "hydroxyl protecting group" or "thiol protecting group". The term "amino protecting group", also known as an N atom-protecting group, refers to a protecting group suitable for preventing side reactions at the nitrogen atom of the amino. Representative amino protecting groups include, but are not limited to: acyl, such as alkanoyl (such as formyl, acetyl, trichloroacetyl or trifluoroacetyl); alkoxycarbonyl, such as *tert*-butoxycarbonyl (Boc) and allyloxycarbonyl (Alloc); arylmethoxycarbonyl, such as carbobenzoxy (Cbz) and 9-fluorenylmethoxycarbonyl (Fmoc); arylmethyl, such as benzyl (Bn), *p*-methoxybenzyl (PMB), 3,4-dimethoxybenzyl (DMB), trityl (Tr), 1,1-di-(4'-methoxyphenyl)methyl; silyl, such as trimethylsilyl (TMS) and *tert*-butyldimethylsilyl (TBS); and sulfonyl, such as *p*-toluenesulfonyl (Tos), *o*-nitrobenzenesulfonyl (Nos), and the like. The term "hydroxyl protecting group" refers to a protecting group suitable for preventing side reactions of the hydroxy group. Representative hydroxy protecting groups include, but are not limited to: alkyl, such as methyl, ethyl, and *tert-*butyl; acyl, such as alkanoyl (such as acetyl); arylmethyl, such as benzyl (Bn), p-methoxybenzyl (PMB), 9-fluorenylmethyl (Fm), and diphenylmethyl (DPM); and silyl, such as trimethylsilyl (TMS), tert-butyldimethylsilyl (TBS), and the like.

The compound disclosed herein can be prepared by a variety of synthetic methods well known to those skilled in the art, including the specific examples listed below, examples formed by combinations thereof with other chemical synthetic methods, and equivalents thereof known to those skilled in the art. Preferred examples include, but are not limited to, the examples of the present invention.

Abbreviations: Pd(dppf)Cl₂ represents 1,1'-bis(diphenylphosphino)ferrocenepalladium dichloride; PMB represents *p*-methoxybenzyl, which is a protecting group; Cbz represents benzyloxycarbonyl, which is a protecting group; Boc represents *tert*-butyloxycarbonyl, which is a protecting group; Pd/C represents palladium on carbon; psi represents pound force per square inch, which is a unit of pressure; eq represents equivalent; mol represents mole; mmol represents millimole; µmol represents micromole; g represents gram; mg represents milligram; mL represents milliliter; µL represents microliter; mm represents millimeter; µm represents micrometer; h represents hour; min represents minute.

The compound disclosed herein may be structurally confirmed by conventional methods well known to those skilled in the art; if the present invention relates to the absolute configuration of the compound, this absolute configuration may be confirmed by means of conventional techniques in the art. For example, in single crystal X-ray diffraction (SXRD), intensity data of diffraction of the single crystal grown are collected with a Bruker D8 venture diffractometer, with the light source being CuKα radiation and the scanning mode being ϕ/ω scanning; after related data are collected, a direct method (Shelxs97) is further employed to analyze the crystal structure, and thus the absolute configuration can be confirmed.

The solvents used in the present invention are commercially available.

Compounds are named according to conventional nomenclature rules in the art or using ChemDraw^{®} software, and supplier's catalog names are given for commercially available compounds.

### DETAILED DESCRIPTION

The present invention is described in detail below by way of examples. However, this is by no means disadvantageously limiting the scope of the present invention. Although the present invention has been described in detail herein and specific examples have also been disclosed, it will be apparent to those skilled in the art that various changes and modifications can be made to the specific examples without departing from the spirit and scope of the present invention.

### Reference Example 1: Synthesis of Intermediates N-1 and N-2

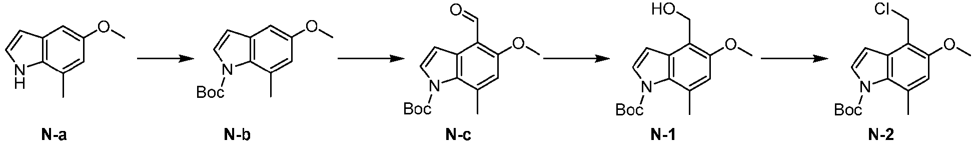

### Step 1

Di-*tert*-butyl dicarbonate (812.33 mg, 3.72 mmol, 855.09 µL, 1.2 eq) and *N*,*N*-diisopropylethylamine (37.89 mg, 310.17 µmol, 0.1 eq) were added to a solution of compound **N-a** (0.5 g, 3.10 mmol, 1 eq) in tetrahydrofuran (5 mL) at 15 °C. The reaction solution was stirred at 15 °C for 1 h. The reaction solution was added to water (15 mL) and stirred for another 5 min. The phases were separated, and the aqueous phase was extracted 3 times with ethyl acetate (20 mL). The combined organic layers were washed twice with saturated brine (20 mL), dried over anhydrous sodium sulfate, and filtered, and the filtrate was concentrated under reduced pressure. The residue was separated and purified by silica gel column chromatography (ethyl acetate:petroleum ether = 0: 1 to 1:10) to give compound **N-b.**

¹H NMR (400 MHz, CDCl₃) δ ppm 7.54-7.48 (m, 1H), 6.90-6.84 (m, 1H), 6.77-6.70 (m, 1H), 6.50-6.43 (m, 1H), 3.90-3.77 (m, 3H), 2.70-2.55 (m, 3H), 1.66-1.61 (m, 9H); LC-MS: *m*/*z* = 206.1 [M-56+H]⁺.

### Step 2

Oxalyl chloride (431.90 mg, 3.40 mmol, 297.86 µL, 1.3 eq) was slowly added dropwise to a solution of *N*-methyl-*N-*formylaniline (459.93 mg, 3.40 mmol, 418.12 µL, 1.3 eq) in dichloromethane (2.7 mL) at 15 °C under nitrogen atmosphere. After the addition, the reaction solution was stirred at 15 °C for 12 h and then added dropwise to a solution of compound **N-b** (0.684 g, 2.62 mmol, 1 eq) in dichloromethane (2.8 mL) at -14 °C. After the addition, the reaction solution was stirred at -15 °C for another 1.5 h. The reaction solution was poured into ice water (20 mL) and stirred for another 5 min. The aqueous phase was extracted 3 times with ethyl acetate (20 mL), and the combined organic phases were washed 3 times with saturated brine (20 mL), dried over anhydrous sodium sulfate, and filtered. The filtrate was concentrated under reduced pressure, and the residue was separated and purified by silica gel column chromatography (ethyl acetate:petroleum ether = 0:1 to 1:10) to give compound **N-c**. ¹H NMR (400 MHz, DMSO-*d*₆) δ ppm 10.61-10.41 (m, 1H), 7.91-7.71 (m, 1H), 7.38-7.21 (m, 1H), 7.07-6.97 (m, 1H), 3.97-3.93 (m, 3H), 2.63-2.59 (m, 3H), 1.60-1.58 (m, 9H); LC-MS: m/z =290.1 [M+H]⁺.

### Step 3

Sodium borohydride (1.71 g, 45.28 mmol, 2.5 eq) was slowly added to a solution of compound N-c (5.24 g, 18.11 mmol, 1 eq) in tetrahydrofuran (20 mL) and methanol (20 mL) at 0 °C under nitrogen atmosphere. After the addition, the reaction solution was stirred at 15 °C for 0.5 h, and then diluted hydrochloric acid (0.5 M, 20 mL) was added slowly. After the addition, the reaction solution was stirred for another 20 min. The mixture was extracted 3 times with ethyl acetate (60 mL), and the combined organic phases were washed twice with saturated brine (60 mL), dried over anhydrous sodium sulfate, and filtered. The filtrate was concentrated under reduced pressure, and the residue was separated and purified by silica gel column chromatography (ethyl acetate:petroleum ether = 1:10 to 1:3) to give compound **N-1**.

¹H NMR (400 MHz, CDCl₃) δ ppm 7.59 - 7.50 (m, 1H), 6.80 - 6.70 (m, 1H), 6.66 - 6.60 (m, 1H), 4.97 - 4.85 (m, 2H), 3.96 - 3.86 (m, 3H), 2.68 - 2.58 (m, 3H), 1.65 - 1.63 (m, 9H).

### Step 4

(Chloromethylene)dimethylammonium chloride (197.71 mg, 1.54 mmol, 1.5 eq) was added to a solution of compound N-1 (0.3 g, 1.03 mmol, 1 eq) in dichloromethane (6.87 mL) at 15 °C under nitrogen atmosphere. After the addition, the reaction solution was stirred at -15 °C for 1 h. The reaction solution was cooled to 0 °C, an aqueous sodium bicarbonate solution (5%, 30 mL) was added, and the mixture was extracted 3 times with dichloromethane (20 mL). The combined organic phases were washed twice with saturated brine (10 mL), dried over anhydrous sodium sulfate, and filtered. The filtrate was concentrated under reduced pressure to give compound **N-2**.

¹H NMR (400 MHz, DMSO-*d*₆) δ ppm 7.73 - 7.61 (m, 1H), 6.91 - 6.86 (m, 1H), 6.82 - 6.75 (m, 1H), 4.99 - 4.91 (m, 2H), 3.89 - 3.82 (m, 3H), 2.56 - 2.54 (m, 3H), 1.60 - 1.57 (m, 9H).

### Example 1

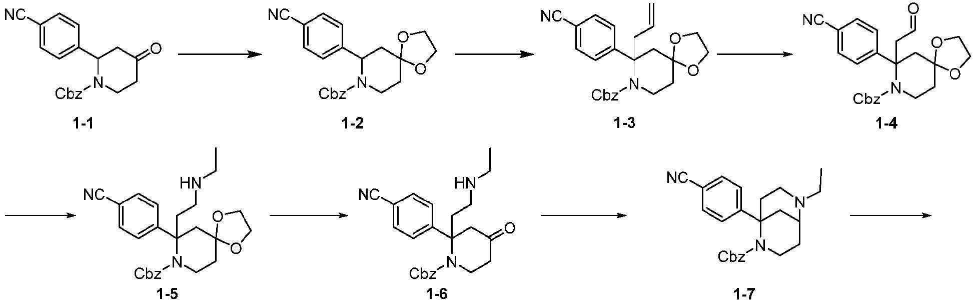

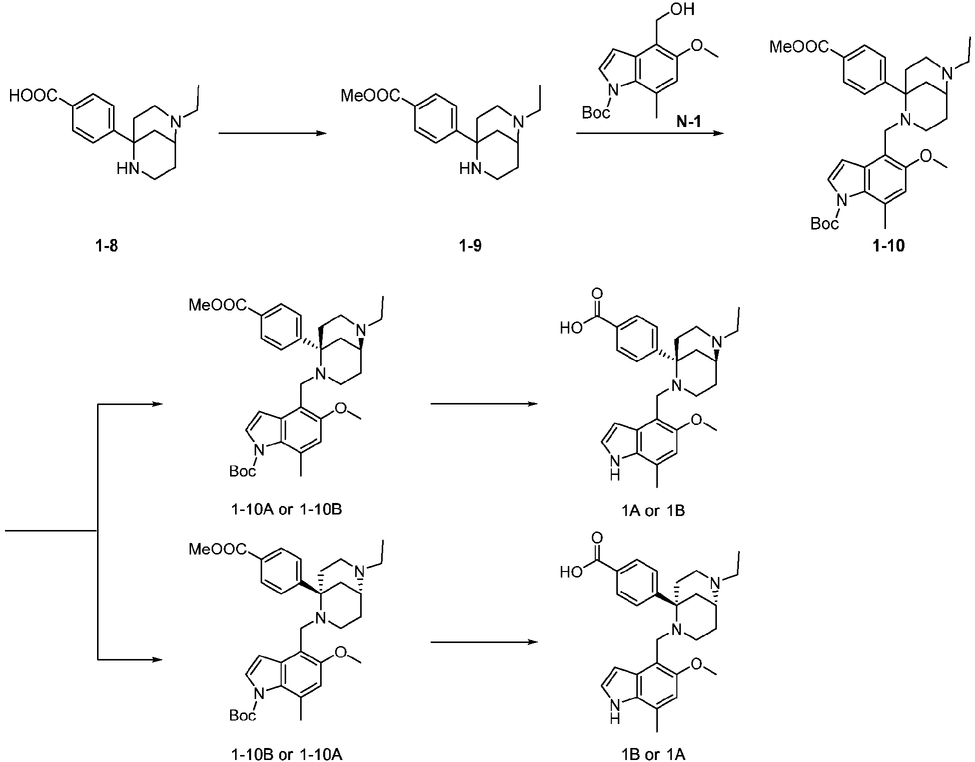

### Step 1

*p*-Toluenesulfonic acid monohydrate (295.83 mg, 1.56 mmol, 0.2 eq) was added to a solution of compound **1-1** (2.6 g, 7.78 mmol, 1 eq) and ethylene glycol (965.26 mg, 15.55 mmol, 869.61 µL, 2 eq) in toluene (40 mL) at 25 °C. The reaction solution was warmed to 100 °C and stirred for another 6 h. The reaction solution was cooled to room temperature, and ethyl acetate (15 mL) was added. The mixture was washed with a saturated aqueous sodium bicarbonate solution (15 mL) and saturated brine (15 mL), respectively. The organic layer was dried over anhydrous sodium sulfate and filtered, and the filtrate was concentrated under reduced pressure. The residue was separated and purified by silica gel column chromatography (ethyl acetate:petroleum ether = 3:1 to 2:1) to give compound **1-2.** LC-MS: *m*/*z* = 379.1 [M+H]⁺.

### Step 2

*n*-Butyllithium (2.5 M, 2.70 mL, 1.5 eq) was slowly added dropwise to a solution of compound **1-2** (1.7 g, 4.49 mmol, 1 eq) in tetrahydrofuran (25 mL) at -70 °C under nitrogen atmosphere. After the addition, the reaction solution was stirred at -70 °C for 0.5 h, and then allyl bromide (1.63 g, 13.48 mmol, 3 eq) was added. After the addition, the reaction solution was warmed to 10-15 °C and stirred for 8 h. The reaction solution was quenched with a saturated aqueous ammonium chloride solution (20 mL), and the mixture was extracted twice with ethyl acetate (15 mL). The combined organic phases were washed with saturated brine (15 mL), dried over anhydrous sodium sulfate, and filtered. The filtrate was concentrated under reduced pressure, and the residue was separated and purified by silica gel column chromatography (ethyl acetate:petroleum ether = 1:2 to 1:4) to give compound **1-3.**

¹H NMR (400 MHz, CDCl₃) δ ppm 7.44 (d, J--8.4 Hz, 2H), 7.26 - 7.20 (m, 5H), 7.05 (brs, 2H), 5.78 - 5.60 (m, 1H), 5.09 - 4.85 (m, 4H), 3.99 - 3.87 (m, 1H), 3.84 - 3.62 (m, 3H), 3.57 - 3.43 (m, 2H), 3.20 (brdd, J=5.8, 13.8 Hz, 1H), 2.64 (dd, J=8.0, 14.0 Hz, 1H), 2.29 (d, *J*=14.8 Hz, 1H), 1.88 - 1.78 (m, 3H); LC-MS: *m*/*z* = 419.1 [M+H]⁺.

### Step 3

Ozone was slowly bubbled into a solution of compound 1-3 (1.6 g, 3.82 mmol, 1 eq) in dichloromethane (32 mL) at -70 °C until the solution was pale blue, and the mixture was stirred at -70 °C for another 10 min. The mixture was purged with nitrogen 3 times, then triphenylphosphine (2.01 g, 7.65 mmol, 2 eq) was added, and the mixture was warmed to room temperature and stirred for another 15 min under nitrogen atmosphere. The reaction solution was concentrated under reduced pressure, and the residue was separated and purified by silica gel column chromatography (ethyl acetate:petroleum ether = 1:2 to 1:3) to give compound **1-4.**

¹H NMR (400 MHz, CDCl₃) δ ppm 9.67 (s, 1H), 7.56 (d, J--8.4 Hz, 2H), 7.37 - 7.27 (m, 5H), 7.18 (brs, 2H), 5.08 - 4.94 (m, 2H), 4.06 - 3.99 (m, 1H), 3.96 - 3.71 (m, 5H), 3.47 - 3.22 (m, 2H), 2.50 - 2.13 (m, 2H), 2.04 - 1.92 (m, 2H); LC-MS: *m*/*z* = 421.1 [M+H]⁺.

### Step 4

Ethylamine hydrochloride (465.46 mg, 5.71 mmol, 4 eq), potassium acetate (560.20 mg, 5.71 mmol, 4 eq), and sodium cyanoborohydride (358.71 mg, 5.71 mmol, 4 eq) were added to a solution of compound **1-4** (0.6 g, 1.43 mmol, 1 eq) in methanol (10 mL) at 0 °C. The mixture was stirred at 0 °C for 3 h. The reaction solution was concentrated under reduced pressure, then water (20 mL) was added at 15 °C, and the mixture was extracted 3 times with ethyl acetate (20 mL). The organic phases were combined, washed with saturated brine (20 mL), dried over anhydrous sodium sulfate, and filtered. The filtrate was concentrated under reduced pressure to give a crude product of compound **1-5,** which was directly used in the next step. LC-MS: *m*/*z* = 450.1 [M+H]⁺.

### Step 5

Hydrochloric acid (2 M, 14 mL, 17.48 eq) was added to a solution of compound **1-5** (0.72 g, 1.60 mmol, 1 eq) in tetrahydrofuran (14 mL) at 25 °C, and the mixture was stirred at 25 °C for 8 h. The reaction solution was concentrated under reduced pressure to give a crude hydrochloride of compound **1-6**, which was directly used in the next step. LC-MS: *m*/*z* = 406.1 [M+H]⁺.

### Step 6

Potassium acetate (907.63 mg, 9.25 mmol) and sodium cyanoborohydride (581.17 mg, 9.25 mmol) were added to a solution of compound **1-6** (0.75 g, hydrochloride) in methanol (20 mL) at 15 °C. The mixture was stirred at 15 °C for 4 h and then stirred at 45 °C for another 4 h. The reaction solution was concentrated under reduced pressure, then water (20 mL) was added at 15 °C, and the mixture was extracted 3 times with ethyl acetate (20 mL). The organic phases were combined, washed with saturated brine (20 mL), dried over anhydrous sodium sulfate, and filtered. The filtrate was concentrated under reduced pressure to give a crude product of compound **1-7**, which was directly used in the next step. LC-MS: *m*/*z* = 390.1 [M+H]⁺.

### Step 7

Hydrobromic acid (5.96 g, 35.36 mmol, 4.00 mL, 48% content, 34.43 eq) was added to compound **1-7** (0.4 g, 1.03 mmol, 1 eq) at 15 °C. The mixture was warmed to 100 °C and stirred for 8 h. The reaction solution was cooled to room temperature and then concentrated under reduced pressure to give a crude hydrobromide of compound **1-8**, which was directly used in the next step. LC-MS: *m*/*z* = 275.2[M+H]⁺.

### Step 8

Hydrochloric acid methanol (4 M, 30.00 mL) was added to compound **1-8** (0.6 g, hydrobromide) at 15 °C. The mixture was warmed to 80 °C and stirred for 3 h. The reaction solution was cooled to room temperature and then concentrated under reduced pressure to give a crude hydrochloride of compound **1-9**, which was directly used in the next step. LC-MS: *m*/*z* = 289.1 [M+H]⁺

### Step 9

Dibromobis(triphenyl)phosphine (37.67 mg, 89.24 µmol, 1.3 eq) was added to a solution of compound N-1 (0.02 g, 68.65 µmol, 1 eq) in dichloromethane (5 mL) at 0 °C. After the reaction solution was stirred at 0 °C for 1 h, *N*,*N*-diisopropylethylamine (26.62 mg, 205.94 µmol, 3 eq) and compound 1-9 (21.78 mg, hydrochloride) were added. The reaction solution was stirred at 0 °C for another 1 h and concentrated under reduced pressure to remove the solvent. The residue was separated and purified by silica gel column chromatography (dichloromethane:methanol = 10:1) and further purified by high performance liquid chromatography (column: Phenomenex Gemini-NX C18 75 × 30 mm, 3 µm; mobile phase: A: water (0.225% formic acid), B: acetonitrile; method: B: 4%-15%, 5 min) to give compound 1-10, which was then separated by chiral column chromatography (column: ChiralPak AD 250 × 30 mm, 10 µm; mobile phase: A: carbon dioxide, B: ethanol (0.1% ammonium hydroxide); method: B: 40%-40%, 4 min, then maintaining B at 40% for 20 min) to give compound **1-10A** (retention time: 1.614 min) and compound **1-10B** (retention time: 2.141 min). SFC analytical method: column: Chiralpak AD 50 × 4.6 mm I.D., 3 µm; mobile phase: A: CO₂, B: ethanol (0.05% diethylamine); gradient: B%: 5%-40%. LC-MS: m/z = 562.1 [M+H]⁺.

### Step 10

An aqueous lithium hydroxide solution (1 M, 115.72 µL, 5 eq) was added to a mixed solution of compound **1-10A** (0.013 g, 23.14 µmol, 1 eq) in tetrahydrofuran (0.5 mL) and methanol (0.5 mL) at 25 °C under nitrogen atmosphere. The reaction solution was warmed to 40 °C and stirred for 8 h. The reaction solution was cooled to room temperature and adjusted to pH 6 with 0.5 M diluted hydrochloric acid. The mixture was diluted with water (2 mL) and concentrated under reduced pressure to give a crude product, which was purified by high performance liquid chromatography (column: Phenomenex Gemini-NX C18 75 × 30 mm, 3 µm; mobile phase: A: water (0.225% formic acid), B: acetonitrile; method: B: 1%-30%, 7 min) to give compound **1A** (ee = 100%, retention time: 1.718 min. SFC analytical method: column: Chiralpak AD 50 × 4.6 mm I.D., 3 µm; mobile phase: A: CO₂, B: ethanol (0.05% diethylamine); gradient: B%: 5%-40%). ¹H NMR (400 MHz, CD₃OD) δ ppm 8.51 (br s, 1H), 8.11 (d, *J*=8.2 Hz, 2H), 7.69 (d, *J*=8.0 Hz, 2H), 7.23 (d, *J*=2.8 Hz, 1H), 6.70 (s, 1H), 6.27 (d, *J*=3.2Hz, 1H), 3.73 (br s, 5H), 3.22 - 2.95 (m, 5H), 2.79 - 2.65 (m, 1H), 2.48 (m, 4H), 2.06 (m, 3H), 1.41 - 1.20 (m, 6H); LC-MS: *m*/*z* = 448.2 [M+H]⁺.

An aqueous lithium hydroxide solution (1 M, 160.23 µL, 5 eq) was added to a mixed solution of compound 1-10B (0.018 g, 32.05 µmol, 1 eq) in tetrahydrofuran (0.5 mL) and methanol (0.5 mL) at 25 °C under nitrogen atmosphere. The reaction solution was warmed to 40 °C and stirred for 8 h. The reaction solution was cooled to room temperature and adjusted to pH 6 with 0.5 M diluted hydrochloric acid. The mixture was diluted with water (2 mL) and concentrated under reduced pressure to give a crude product, which was purified by high performance liquid chromatography (column: Phenomenex Gemini-NX C18 75 × 30 mm, 3 µm; mobile phase: A: water (0.225% formic acid), B: acetonitrile; method: B: 0%-30%, 7 min) to give compound **1B** (ee = 98.83%, retention time: 2.197 min. SFC analytical method: column: Chiralpak AD 50 × 4.6 mm I.D., 3 µm; mobile phase: A: CO₂, B: ethanol (0.05% diethylamine); gradient: B%: 5%-40%). ¹H NMR (400 MHz, CD₃OD) δ ppm 8.51 (br s, 1H), 8.11 (d, *J*=8.2 Hz, 2H), 7.69 (d, *J*=8.0 Hz, 2H), 7.23 (d, *J*=2.8 Hz, 1H), 6.70 (s, 1H), 6.27 (d, *J*=3.2Hz, 1H), 3.73 (br s, 5H), 3.22 - 2.95 (m, 5H), 2.79 - 2.65 (m, 1H), 2.48 (m, 4H), 2.06 (m, 3H), 1.41 - 1.20 (m, 6H); LC-MS: *m*/*z* = 448.2 [M+H]⁺.

### Example 2

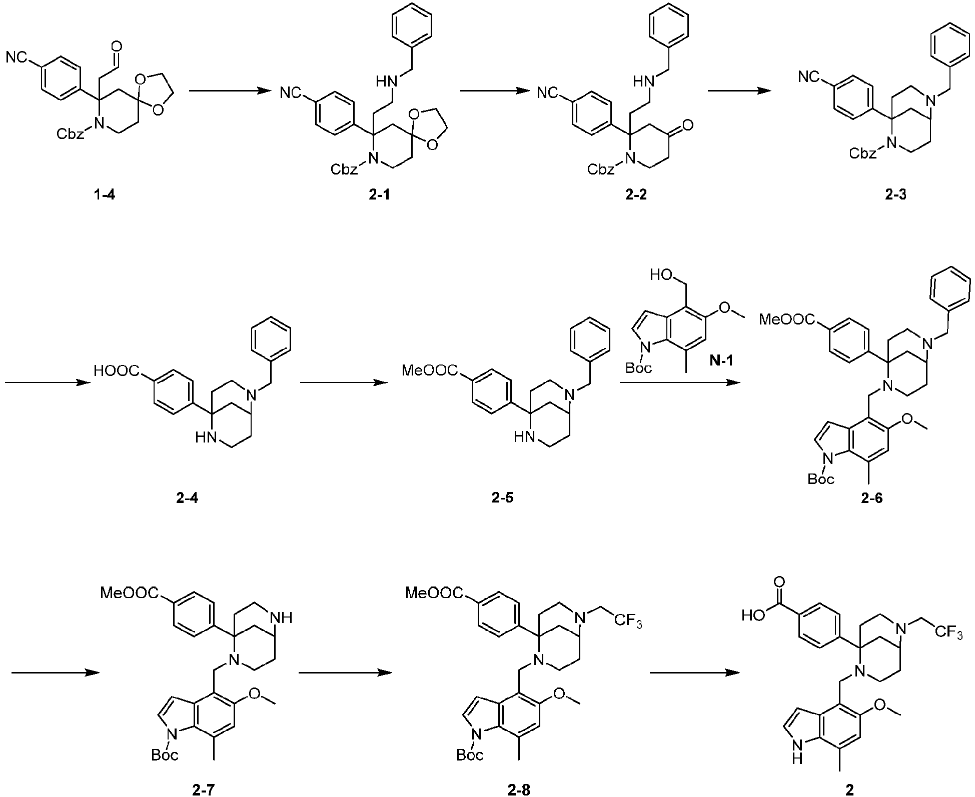

### Step 1

Benzylamine (1.30 g, 12.13 mmol, 1.32 mL, 3 eq), potassium acetate (1.19 g, 12.13 mmol, 3 eq), and sodium cyanoborohydride (762.25 mg, 12.13 mmol, 3 eq) were added to a solution of compound **1-4** (1.7 g, 4.04 mmol, 1 eq) in methanol (30 mL) at 0 °C. The mixture was warmed to 20 °C and stirred for 12 h. The reaction solution was concentrated under reduced pressure, then water (100 mL) was added at 20 °C, and the mixture was extracted twice with ethyl acetate (100 mL). The organic phases were combined, dried over anhydrous sodium sulfate, and filtered. The filtrate was concentrated under reduced pressure, and the residue was purified by silica gel column chromatography (ethyl acetate:petroleum ether = 1:1 to 1:0) to give compound **2-1.** LC-MS: *m*/*z* = 512.2 [M+H]⁺.

### Step 2

Hydrochloric acid (2 M, 24 mL, 20.46 eq) was added to a solution of compound **2-1** (1.2 g, 2.35 mmol, 1 eq) in tetrahydrofuran (1 mL) at 20 °C, and the mixture was stirred at 20 °C for 12 h. The reaction solution was concentrated under reduced pressure to give a crude hydrochloride of compound **2-2,** which was directly used in the next step. LC-MS: *m*/*z* = 468.2[M+H]⁺.

### Step 3

potassium acetate (1.05 g, 10.69 mmol, 5.34 eq) and sodium cyanoborohydride (672.02 mg, 10.69 mmol, 5.34 eq) were added to a solution of compound **2-2** (1.0 g hydrochloride) in methanol (20 mL) at 20 °C. The mixture was stirred at 20 °C for 12 h. Water (50 mL) was added to the reaction solution, and the mixture was extracted 3 times with ethyl acetate (50 mL). The organic phases were combined, dried over anhydrous sodium sulfate, and filtered. The filtrate was concentrated under reduced pressure to give a crude product of compound **2-3,** which was directly used in the next step. LC-MS: *m*/*z* = 452.3 [M+H]⁺.

### Step 4

Hydrobromic acid (40.23 g, 198.88 mmol, 27.00 mL, 40% content, 99.79 eq) was added to compound **2-3** (900 mg, 1.99 mmol, 1 eq) at 0 °C. The mixture was warmed to 100 °C and stirred for 2 h. The reaction solution was cooled to room temperature and then concentrated under reduced pressure to give a crude hydrobromide of compound **2-4,** which was directly used in the next step. LC-MS: m/z = 337.1 [M+H]⁺.

### Step 5

Hydrochloric acid methanol (4 M, 6 mL) was added to compound **2-4** (600 mg hydrobromide) at 15 °C. The mixture was warmed to 80 °C and stirred for 12 h. The reaction solution was cooled to room temperature and then concentrated under reduced pressure to give a crude hydrochloride of compound **2-5,** which was directly used in the next step. LC-MS: *m*/*z* = 351.4 [M+H]⁺.

### Step 6

Dibromobis(triphenyl)phosphine (578.12 mg, 1.37 mmol, 1.23 eq) was added to a solution of compound **N-1** (324.22 mg, 1.11 mmol, 1.0 eq) in dichloromethane (7 mL) at 0 °C. After the reaction solution was stirred at 0 °C for 1 h, *N*,*N*-diisopropylethylamine (442.54 mg, 3.42 mmol, 596.41 µL, 3.08 eq) and compound **2-5** (0.3 g, hydrochloride) were added. The reaction solution was stirred at 0 °C for 1 h, then warmed to 15 °C, and stirred for another 12 h. The reaction solution was concentrated under reduced pressure to remove the solvent, and the residue was separated and purified by silica gel column chromatography (ethyl acetate:petroleum ether = 1:5 to 1:10) to give compound **2-6.** LC-MS: *m*/*z* = 624.2 [M+H]⁺.

### Step 7

1-Chloroacetyl chloride (81.82 mg, 572.32 µmol, 3 eq) and *N*,*N*-diisopropylethylamine (86.30 mg, 667.70 µmol, 116.30 µL, 3.5 eq) were added to a solution of compound **2-6** (0.119 g, 190.77 µmol, 1 eq) in dichloromethane (5 mL) at 15 °C under nitrogen atmosphere. The mixture was warmed to 85 °C and stirred for 30 min. The mixture was concentrated under reduced pressure at 45 °C, and the residue was added with methanol (5 mL) at 15 °C, warmed to 65 °C, and stirred for 1 h. The mixture was cooled to 45 °C and concentrated under reduced pressure. The residue was purified by high performance liquid chromatography (column: Unisil 3-100 C18 Mltra 150 × 50 mm, 3 µm; mobile phase: A: water (0.225% trifluoroacetic acid), B: acetonitrile; method: B: 15%-45%, 10 min) to give compound **2-7.** LC-MS: *m*/*z* = 534.2 [M+H]⁺.

### Step 8

2,2,2-Trifluoroethyl trifluoromethanesulfonate (39.14 mg, 168.65 µmol, 2.21 µL, 3 eq) and *N*,*N*-diisopropylethylamine (21.80 mg, 168.65 µmol, 29.38 µL, 3 eq) were added to a solution of compound 2-7 (30 mg, 56.22 µmol, 1 eq) in acetonitrile (1 mL) at 15 °C. The reaction solution was stirred at 30 °C for 1 h and concentrated under reduced pressure to remove the solvent. The residue was separated and purified by silica gel column chromatography (ethyl acetate:petroleum ether = 1:2) to give compound 2-8. LC-MS: m/z = 616.2[M+H]⁺.

### Step 9

An aqueous lithium hydroxide solution (1 M, 73.09 µL, 5 eq) was added to a mixed solution of compound **2-8** (9 mg, 14.62 µmol, 1 eq) in tetrahydrofuran (0.5 mL) and methanol (0.5 mL) at 15 °C under nitrogen atmosphere. The reaction solution was warmed to 40 °C and stirred for 12 h. The reaction solution was cooled to room temperature and adjusted to pH 7 with glacial acetic acid. The mixture was concentrated under reduced pressure to give a crude product, which was purified by high performance liquid chromatography (column: Unisil 3-100 C18 Mltra 150 × 50 mm, 3 µm; mobile phase: A: water (0.225% formic acid), B: acetonitrile; method: B: 16%-36%, 10 min) to give compound 2.

¹H NMR (400 MHz, DMSO-*d*₆) δ ppm 10.86-10.72 (m, 1H), 7.97-7.89 (m, 2H), 7.81-7.69 (m, 2H), 7.26-7.17 (m, 1H), 6.65-6.57 (m, 1H), 6.36-6.28 (m, 1H), 3.65-3.62 (m, 3H), 3.12-3.00 (m, 2H), 3.00-2.87 (m, 2H), 2.66 (brs, 3H), 2.39 (s, 3H), 2.35-2.28 (m, 2H), 2.12-2.00 (m, 1H), 1.95-1.83 (m, 1H), 1.73-1.55 (m, 2H), 1.30-1.16 (m, 1H); LC-MS: *m*/*z* = 502.2 [M+H]⁺.

### Example 3

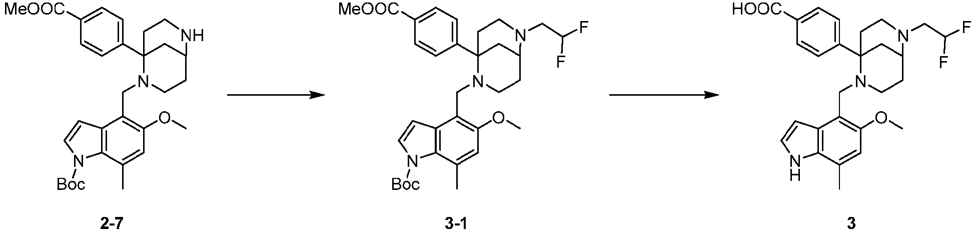

### Step 1

1,1-Difluoro-2-iodoethane (30.21 mg, 157.40 µmol, 2.21 µL, 3 eq) and *N*,*N*-diisopropylethylamine (27.12 mg, 209.87 µmol, 36.56 µL, 4 eq) were added to a solution of compound **2-7** (28 mg, 52.47 µmol, 1 eq) in acetonitrile (1 mL) at 15 °C. The reaction solution was stirred at 40 °C for 12 h and concentrated under reduced pressure to remove the solvent. The residue was separated and purified by silica gel column chromatography (ethyl acetate:petroleum ether = 1:1) to give compound **3-1.** LC-MS: m/z = 598.2[M+H]⁺.

### Step 2

An aqueous lithium hydroxide solution (1 M, 75.29 µL, 5 eq) was added to a mixed solution of compound **3-1** (9 mg, 15.06 µmol, 1 eq) in tetrahydrofuran (0.2 mL) and methanol (0.2 mL) at 15 °C under nitrogen atmosphere. The reaction solution was warmed to 40 °C and stirred for 12 h. The reaction solution was cooled to room temperature and adjusted to pH 7 with glacial acetic acid. The mixture was concentrated under reduced pressure to give a crude product, which was purified by high performance liquid chromatography (column: Unisil 3-100 C18 Mltra 150 × 50 mm, 3 µm; mobile phase: A: water (0.225% formic acid), B: acetonitrile; method: B: 10%-40%, 10 min) to give compound **3.**

¹H NMR (400 MHz, DMSO-*d*₆) δ ppm 10.84-10.67 (m, 1H), 8.27-8.17 (m, 1H), 7.97-7.90 (m, 2H), 7.79-7.71 (m, 2H), 7.23-7.16 (m, 1H), 6.67-6.55 (m, 1H), 6.37-6.29 (m, 1H), 6.23-5.94 (m, 1H), 3.65-3.63 (m, 3H), 3.08-2.96 (m, 4H), 2.70-2.64 (m, 3H), 2.43-2.37 (m, 4H), 2.34-2.31 (m, 1H), 2.08-2.03 (m, 1H), 1.87-1.81 (m, 1H), 1.68-1.61 (m, 2H); LC-MS: *m*/*z* = 484.1 [M+H]⁺.

### Example 4

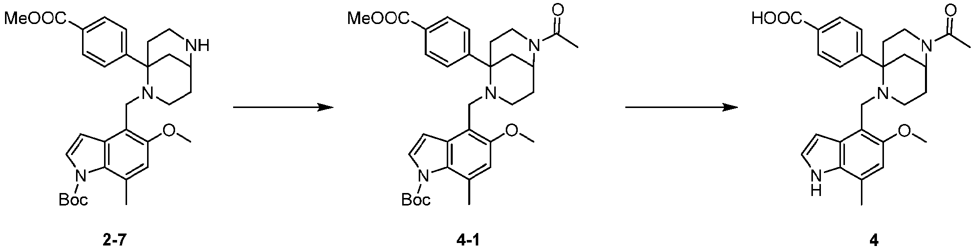

### Step 1

Acetyl chloride (20.59 mg, 262.34 µmol, 18.72 µL, 5 eq) was added to a solution of compound **2-7** (28 mg, 52.47 µmol, 1 eq) and *N*,*N*-diisopropylethylamine (27.12 mg, 209.87 µmol, 36.56 µL, 4 eq) in acetonitrile (0.5 mL) at 0 °C. The reaction solution was stirred at 15 °C for 0.5 h and concentrated under reduced pressure to remove the solvent. The residue was separated and purified by silica gel column chromatography (ethyl acetate:methanol = 10:1) to give compound **4-1.** LC-MS: m/z = 576.2[M+H]⁺.

### Step 2

An aqueous lithium hydroxide solution (1 M, 208.44 µL, 5 eq) was added to a mixed solution of compound **4-1** (24 mg, 41.69 µmol, 1 eq) in tetrahydrofuran (0.2 mL) and methanol (0.2 mL) at 15 °C under nitrogen atmosphere. The reaction solution was warmed to 40 °C and stirred for 8 h. The reaction solution was cooled to room temperature and adjusted to pH 7 with glacial acetic acid. The mixture was concentrated under reduced pressure to give a crude product, which was purified by high performance liquid chromatography (column: Unisil 3-100 C18 Mltra 150 × 50 mm, 3 µm; mobile phase: A: water (0.225% formic acid), B: acetonitrile; method: B: 5%-35%, 10 min) to give compound **4.**

¹H NMR (400 MHz, CD₃OD) δ ppm 8.21-8.15 (m, 2H), 7.81-7.73 (m, 2H), 7.29-7.27 (m, 1H), 6.74-6.71 (m, 1H), 6.25-6.18 (m, 1H), 4.47-4.18 (m, 2H), 4.18-3.92 (m, 2H), 3.88-3.75 (m, 1H), 3.69-3.65 (m, 3H), 3.29-3.21 (m, 1H), 3.19-3.05 (m, 1H), 2.89-2.71 (m, 1H), 2.69-2.57 (m, 1H), 2.51-2.45 (m, 3H), 2.18-2.15 (m, 3H), 1.35-1.27 (m, 4H); LC-MS: *m*/*z* = 462.2 [M+H]⁺.

### Example 5

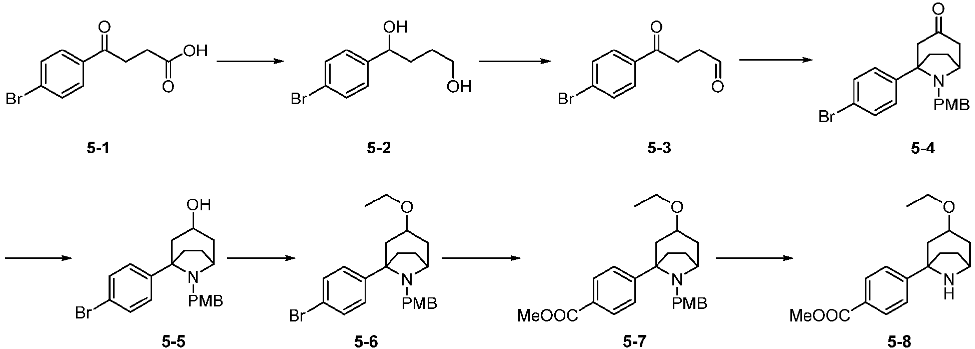

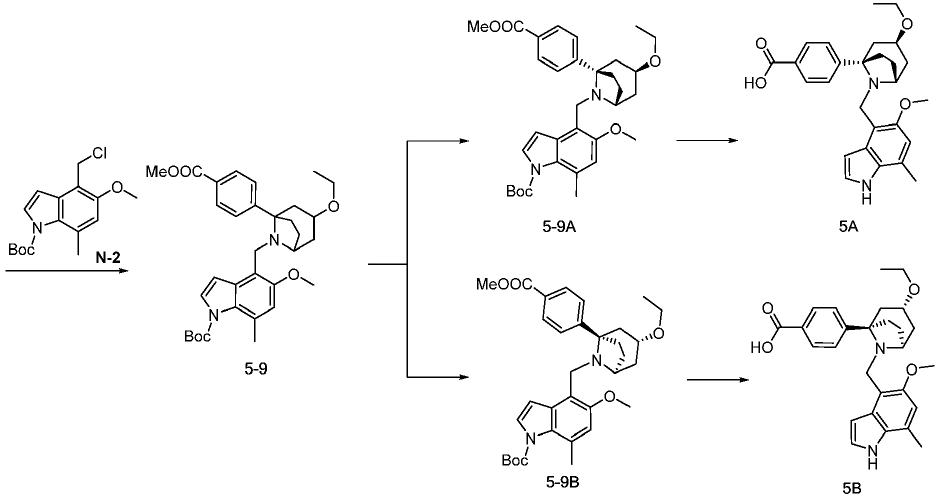

### Step 1

A solution of lithium aluminum hydride (2.5 M, 953 mmol, 3.5 eq) in THF (381.20 mL) was added to a solution of compound **5-1** (70 g, 272.29 mmol, 1 eq) in tetrahydrofuran (700 mL) at 0 °C. The reaction system was stirred at 0 °C for 1 h, and then 36 mL of water, 36 mL of a 15% aqueous sodium hydroxide solution, and 108 mL of water were successively slowly added to quench the reaction system. The mixture was filtered, and the filtrate was dried over anhydrous sodium sulfate and concentrated under reduced pressure to give a crude product of compound **5-2,** which was directly used in the next step.

¹H NMR (400 MHz, CDCl₃) δ ppm 7.46 (d, J=8.28 Hz, 2H), 7.22 (brd, J=8.28 Hz, 2H), 4.63-4.73 (m, 1H), 3.59-3.73 (m, 2H), 1.77-1.90 (m, 2H), 1.58-1.72 (m, 2H).

### Step 2

Oxalyl chloride (33.14 g, 261.10 mmol, 22.86 mL, 4 eq) was added to a solution of dimethyl sulfoxide (30.60 g, 391.66 mmol, 30.60 mL, 6 eq) in dichloromethane (375 mL) at -78 °C under nitrogen atmosphere. The mixture was stirred for 10 min, and then a solution of compound **5-2** (16 g, 65.28 mmol, 1 eq) in dichloromethane (125 mL) was added. The mixture was stirred for another 10 min, then triethylamine (66.05 g, 652.76 mmol, 90.86 mL, 10 eq) was added, and the mixture was stirred at -78 °C for 10 min. Then the reaction system was warmed to 25 °C and stirred for 1 h. The reaction solution was quenched with diluted hydrochloric acid (1 M, 100 mL), the phases were separated, and the organic phase was washed with a saturated aqueous sodium chloride solution (100 mL). The organic phase was dried over anhydrous sodium sulfate, filtered, and concentrated under reduced pressure. The residue was purified by column chromatography (ethyl acetate:petroleum ether = 1:3) to give

### compound 5-3.

¹H NMR (400 MHz, CDCl₃) δ ppm 9.92 (s, 1H), 7.87 (d, *J*=8.8 Hz, 2H), 7.64 (d, *J*=8.8 Hz 2H), 3.31 (t, *J*=6.27 Hz, 2H), 2.97 (t, *J*=6.27 Hz, 2H); LC-MS: *m*/*z* = 240.7/242.7 [M+H]⁺.

### Step 3

4-Methoxybenzylamine (4.48 g, 32.62 mmol, 4.22 mL, 1.1 eq) and sodium acetate (15.40 g, 187.74 mmol, 6.33 eq) were added to 80 mL of water, and then the reaction system was placed in an ice-water bath. 3-Oxopentadienoic acid (5.42 g, 37.07 mmol, 1.25 eq) and diluted hydrochloric acid (1 M, 37.07 mL, 1.25 eq) were added to the reaction solution, and the mixture was stirred under nitrogen atmosphere for 1 h. Then a solution of compound **5-3** (7.15 g, 29.66 mmol, 1 eq) in tetrahydrofuran (80 mL) was added to the reaction solution. The reaction system was warmed to 40 °C and stirred for 15 h. The reaction solution was diluted with 50 mL of ethyl acetate and washed with water (50 mL × 3). The organic phase was dried over anhydrous sodium sulfate, filtered, and concentrated under reduced pressure. The residue was purified by column chromatography (ethyl acetate:petroleum ether = 1:4 to 1:3) to give compound **5-4.**

¹H NMR (400 MHz, CDCl₃) δ ppm 7.53 (d, J=8.8 Hz, 2H), 7.44 (d, J=8.8Hz, 2H), 7.29 (d, J--7.2 Hz 2H), 6.90 (d, *J*=8.53 Hz, 2H), 3.83 (s, 3H), 3.64 (brt, *J*=5.02 Hz, 1H), 3.55 (d, *J*=14Hz, 1H), 3.50 (d, *J*=14Hz, 1H), 2.79-2.88 (m, 3H), 1.98-2.26 (m, 4H), 1.57-1.66 (m, 1H); LC-MS: *m*/*z* = 399.9/401.9 [M+H]⁺.

### Step 4

A solution of lithium tri-sec-butylborohydride (1 M, 6.89 mL, 1.2 eq) in tetrahydrofuran was slowly added to a solution of compound **5-4** (2.3 g, 5.75 mmol, 1 eq) in tetrahydrofuran (30 mL) at -78 °C under nitrogen atmosphere. The reaction system was reacted at -78 °C for 1 h. 10 mL of methanol was added to the reaction system at -78 °C, and the mixture was slowly warmed to room temperature. The reaction solution was concentrated under reduced pressure, 10 mL of ethyl acetate was added to the residue, and the mixture was washed with 10 mL of water. The phases were separated, and the organic phase was dried over anhydrous sodium sulfate, filtered, and concentrated under reduced pressure. The residue was purified by column chromatography (ethyl acetate:petroleum ether = 1:1 to 1:3) to give compound **5-5**.

¹H NMR (400 MHz, CDCl₃) δ ppm 7.32-7.41 (m, 4H), 7.17 (br d, *J*=8.75 Hz, 2H), 6.77 (d, *J*=8.38 Hz, 2H), 3.72 (s, 3H), 3.32-3.40 (m, 1H), 3.19 (brs, 1H), 3.08 (br d, *J*=13.63 Hz, 1H), 2.39-2.55 (m, 1H), 1.82-2.23 (m, 8H); LC-MS: *m*/*z* = 401.9/403.9 [M+H]⁺.

### Step 5

Sodium hydride (695.89 mg, 17.40 mmol, 60% content, 10 eq) was slowly added to a solution of compound **5-5** (700 mg, 1.74 mmol, 1 eq) and iodoethane (2.71 g, 17.40 mmol, 1.39 mL, 10 eq) in *N*,*N*-dimethylformamide (20 mL) at 0 °C under nitrogen atmosphere. The reaction system was stirred at 25 °C for 16 h. The reaction system was cooled to 0 °C, and 10 mL of a saturated aqueous ammonium chloride solution was slowly added to quench the reaction system. The aqueous phase was extracted three times with 30 mL of ethyl acetate. The organic phases were combined, dried over anhydrous sodium sulfate, filtered, and concentrated under reduced pressure. The residue was purified by column chromatography (ethyl acetate:petroleum ether = 1:3) to give compound **5-6**.

¹H NMR (400 MHz, CDCl₃) δ ppm 7.32-7.50 (m, 4H), 7.13-7.19 (m, 2H), 6.76 (d, J=8.53 Hz, 2H), 3.69-3.74 (m, 3H), 3.65 (brs, 1H), 3.32-3.41 (m, 3H), 3.14 (brs, 1H), 3.02-3.09 (m, 1H), 2.34-2.44 (m, 1H), 1.81-2.08 (m, 6H), 1.49-1.60 (m, 1H), 1.11 (t, *J*=7.03 Hz, 3H); LC-MS: *m*/*z* = 430.0/432.0[M+H]⁺.

### Step 6

Pd(dppf)Cl₂ (758.95 mg, 1.04 mmol, 0.8 eq) was added to a mixed solution of compound **5-6** (558 mg, 1.30 mmol, 1 eq) and triethylamine (131.20 mg, 1.30 mmol, 180.46 µL, 1 eq) in *N*,*N*-dimethylformamide (10 mL) and methanol (10 mL) at room temperature. The reaction system was purged with carbon monoxide atmosphere (15 psi) and warmed to 80 °C to react for 15 h. The reaction solution was cooled to room temperature and filtered to remove the solid. The filtrate was concentrated under reduced pressure, and the residue was purified by column chromatography (ethyl acetate:petroleum ether = 1:2) to give compound **5-7**.

¹H NMR (400 MHz, CDCl₃) δ ppm 7.93 (br d, *J*=8.50 Hz, 2H), 7.56 (br d, *J*=8.25 Hz, 2H), 7.16-7.23 (m, 2H), 6.77 (brd, *J*=8.50 Hz, 2H), 4.02-3.88 (m, 1H), 3.83 (s, 3H), 3.72 (s, 3H), 3.67 (br s, 1H), 3.33-3.43 (m, 3H), 3.17 (br s, 1H), 3.06 (brd, *J*=13.76 Hz, 1H), 2.38-2.46 (m, 1H), 1.87-2.04 (m, 6H), 1.19 (t, *J*=7.13 Hz, 3H); LC-MS: *m*/*z* = 410.2[M+H]⁺.

### Step 7

Pd/C (0.05 g, 439.54 µmol, 10% content, 1 eq) was added to a mixed solution of compound **5-7** (180 mg, 0.44 mmol, 1 eq) and formic acid (422.32 mg, 8.79 mmol, 20 eq) in methanol (15 mL) at room temperature. The reaction system was warmed to 50 °C to react for 1.5 h. The reaction solution was cooled to room temperature and filtered to remove the solid. The filtrate was concentrated under reduced pressure to give a crude product of compound **5-8**, which was directly used in the next step.

LC-MS: *m*/*z* = 290.2[M+H]⁺.

### Step 8

Cesium carbonate (630.54 mg, 1.94 mmol, 4 eq) and potassium iodide (160.63 mg, 967.62 µmol, 2 eq) were added to a mixed solution of compound **5-8** (0.14 g, 483.81 µmol, 1 eq) and compound **N-2** (284.77 mg, 919.24 µmol, 1.9 eq) in *N*,*N*-dimethylformamide (5 mL) at room temperature, and the reaction system was reacted at 15 °C for 1 h. The reaction solution was quenched by slowly adding to 20 mL of ice water. The aqueous phase was extracted three times with 30 mL of ethyl acetate. The organic phases were combined, dried over anhydrous sodium sulfate, filtered, and concentrated under reduced pressure. The residue was separated and purified by silica gel column chromatography (dichloromethane:methanol = 10:1) to give compound **5-9,** which was then separated by chiral column chromatography (column: Daicel ChiralPak IG 250 × 30 mm, 10 µm; mobile phase: A: carbon dioxide, B: isopropanol (0.1% ammonium hydroxide); method: B: 40%-40%, 5.8 min, then maintaining B at 40%, 80 min) to give compound **5-9A** (retention time: 2.088 min, ee = 100%) and compound **5-9B** (retention time: 2.258 min, 100% ee). SFC analytical method: column: Chiralpak AD 50 × 4.6 mm I.D., 3 µm; mobile phase: A: CO₂, B: isopropanol (0.05% diethylamine); gradient: B%: 5%-40%. LC-MS: m/z = 563.4[M+H]⁺.

### Step 9

An aqueous lithium hydroxide solution (1 M, 4.5 mL, 56.27 eq) was added to a mixed solution of compound **5-9A** (0.045 g, 79.97 µmol, 1 *eq*) in tetrahydrofuran (4.5 mL) and methanol (4.5 mL) at 15 °C under nitrogen atmosphere. The reaction solution was warmed to 45 °C and stirred for 16 h. The reaction solution was cooled to room temperature and adjusted to pH 6-7 with glacial acetic acid. The mixture was concentrated under reduced pressure to give a crude product, which was purified by high performance liquid chromatography (column: Phenomenex Synergi C18 Mltra 150 × 25 mm, 3 µm; mobile phase: A: water (0.225% formic acid), B: acetonitrile; method: B: 14%-44%, 10 min) to give a formate salt of compound **5A**. ¹H NMR (400 MHz, DMSO-*d*₆) δ ppm 10.81 (brs, 1H), 8.24 (s, 1H), 7.96 (d, *J=* 8.4 Hz, 2H), 7.74 (d, *J*= 8.4 Hz, 2H), 7.26 (t, *J*= 2.8 Hz, 1H), 6.66 (s, 1H), 6.53 (brs, 1H), 3.72 (s, 3H), 3.70 (brd, *J=* 3.5 Hz, 2H), 3.18 (brs, 1H), 3.06 (brd, *J*= 2.0 Hz, 2H), 2.42 (s, 3H), 2.25 - 2.15 (m, 1H), 2.12 - 1.86 (m, 6H), 1.55 - 1.48 (m, 1H), 1.11 (t, *J*= 6.8 Hz, 3H); LC-MS: m/z =449.3 [M+H]⁺.

An aqueous lithium hydroxide solution (1 M, 213.26 µL, 10 *eq)* was added to a mixed solution of compound **5-9B** (12 mg, 21.33 µmol, 1 *eq*) in tetrahydrofuran (0.5 mL) and methanol (0.5 mL) at 15 °C under nitrogen atmosphere. The reaction solution was warmed to 45 °C and stirred for 12 h. The reaction solution was cooled to room temperature and adjusted to pH 6-7 with glacial acetic acid. The mixture was concentrated under reduced pressure to give a crude product, which was purified by high performance liquid chromatography (column: Unisil 3-100 C18 Mltra 150 × 50 mm, 3 µm; mobile phase: A: water (0.225% formic acid), B: acetonitrile; method: B: 15%-45%, 10 min) to give a formate salt of compound **5B**. ¹H NMR (400 MHz, DMSO-*d*₆) δ ppm 10.82 (brs, 1H), 8.24 (s, 1H), 7.96 (d, *J*= 8.4 Hz, 2H), 7.75 (d, *J*= 8.4 Hz, 2H), 7.26 (t, *J*= 2.8 Hz, 1H), 6.66 (s, 1H), 6.53 (brs, 1H), 3.72 (s, 3H), 3.70 (brd, *J*= 3.5 Hz, 2H), 3.18 (brs, 1H), 3.06 (brd, *J*= 2.0 Hz, 2H), 2.42 (s, 3H), 2.26 - 2.15 (m, 1H), 2.12 - 1.86 (m, 6H), 1.54 - 1.48 (m, 1H), 1.13 (t, *J*= 6.8 Hz, 3H); LC-MS: m/z =449.3[M+H]⁺.

### Example 6

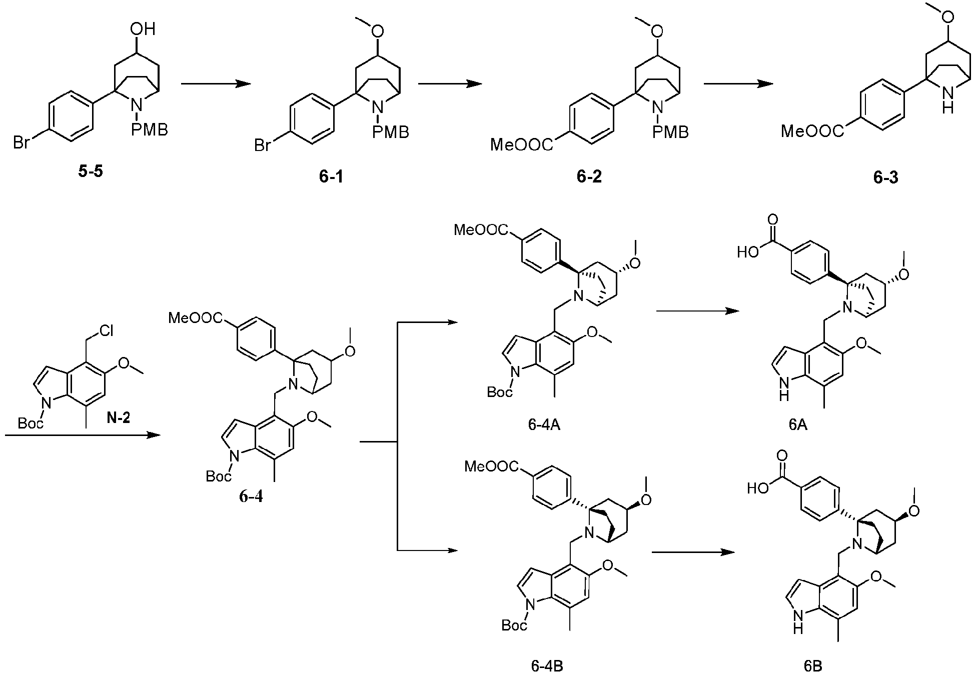

### Step 1

Sodium hydride (208.77 mg, 5.22 mmol, 60% content, 3 *eq)* was slowly added to a solution of compound **5-5** (700 mg, 1.74 mmol, 1 *eq)* and iodomethane (493.91 mg, 3.48 mmol, 0.21 mL, 2 *eq)* in *N*,*N*-dimethylformamide (4 mL) at 0 °C under nitrogen atmosphere. The reaction system was stirred at 20 °C for 12 h. The reaction system was cooled to 0 °C, 10 mL of a saturated aqueous ammonium chloride solution was slowly added to quench the reaction, and 10 mL of water was added to dilute the mixture. The aqueous phase was extracted twice with ethyl acetate (30 mL). The organic phases were combined, washed twice with 20 mL of a saturated aqueous sodium chloride solution, dried over anhydrous sodium sulfate, filtered, and concentrated under reduced pressure. The residue was purified by column chromatography (ethyl acetate:petroleum ether = 1:3) to give compound 6-1. LC-MS:*m*/*z*-416.4/417.0[M+H]^{*}.

### Step 2

Palladium acetate (269.61 mg, 1.20 mmol, 1 *eq)* and 1,3-bis(diphenylphosphino)propane (495.31 mg, 1.20 mmol, 1 *eq*) were added to a mixed solution of compound **6-1** (500 mg, 1.20 mmol, 1 *eq*) and triethylamine (364.56 mg, 3.60 mmol, 501.45 µL, 3 *eq)* in *N*,*N*-dimethylformamide (10 mL) and methanol (10 mL) at room temperature. The reaction system was purged with carbon monoxide atmosphere (15 psi) and warmed to 80 °C to react for 12 h. The reaction solution was cooled to room temperature, concentrated under reduced pressure, diluted with water (50 mL), and extracted twice with ethyl acetate (100 mL). The organic phases were combined, washed twice with a saturated aqueous sodium chloride solution (100 mL), dried over anhydrous sodium sulfate, filtered, and concentrated under reduced pressure. The residue was purified by column chromatography (ethyl acetate:petroleum ether = 1:3) to give compound **6-2**. LC-MS: m/z = 396.0[M+H]⁺.

### Step 3

Wet palladium on carbon (0.2 g, 10% content) was added to a mixed solution of compound **6-2** (300 mg, 758.55 µmol, 1 *eq*) and formic acid (728.84 mg, 15.17 mmol, 20 *eq*) in methanol (30 mL) at room temperature. The reaction system was warmed to 50 °C to react for 2 h. The reaction solution was cooled to room temperature and filtered to remove the solid. The filtrate was concentrated under reduced pressure to give a crude product of compound **6-3**, which was directly used in the next step. LC-MS: m/z = 276.0[M+H]⁺.

### Step 4

Cesium carbonate (946.66 mg, 2.91 mmol, 4 *eq)* and potassium iodide (482.31 mg, 2.91 mmol, 4 *eq)* were added to a mixed solution of compound **6-3** (200 mg, 726.37 µmol, 1 *eq)* and compound **N-2** (247.52 mg, 799.01 µmol, 1.1 *eq)* in *N*,*N-*dimethylformamide (2 mL) at room temperature, and the reaction system was reacted at 20 °C for 1 h. The reaction solution was quenched by slowly adding to 5 mL of ice water. The aqueous phase was extracted twice with 10 mL of ethyl acetate. The organic phases were combined, washed twice with 20 mL of a saturated aqueous sodium chloride solution, dried over anhydrous sodium sulfate, filtered, and concentrated under reduced pressure. The resulting crude product was purified by high performance liquid chromatography (column: Phenomenex luna C18 150 × 25 mm, 10 µm; mobile phase: A: water (0.225% formic acid), B: acetonitrile; method: B: 25%-55%) to give compound **6-4,** which was then separated by chiral column chromatography (column: Daicel ChiralPak AD 250 × 30 mm, 10 µm; mobile phase: A: carbon dioxide, B: isopropanol (0.1% ammonium hydroxide); method: B: 35%-35%, 7.2 min, then maintaining B at 35%, 100 min) to give compound **6-4A** (ee = 100%, retention time: 1.778 min) and compound **6-4B** (ee = 100%, retention time: 1.960 min). SFC analytical method: column: OZ-3 50 × 4.6 mm I.D., 3 µm; mobile phase: A: n-heptane (0.05%), B: ethanol (0.05% diethylamine); gradient B%: 40%. LC-MS: *m*/*z* = 549.3 [M+H]⁺.

### Step 5

An aqueous lithium hydroxide solution (1 M, 2 mL, 10 *eq)* was added to a mixed solution of compound **6-4A** (100 mg, 182.26 µmol, 1 *eq*) in tetrahydrofuran (1 mL) and methanol (1 mL) at 15 °C under nitrogen atmosphere. The reaction solution was warmed to 45 °C and stirred for 12 h. The reaction solution was cooled to room temperature and adjusted to pH 6-7 with glacial acetic acid. The mixture was concentrated under reduced pressure to give a crude product, which was purified by high performance liquid chromatography (column: Phenomenex Synergi C18 Mltra 150 × 25 mm, 10 µm; mobile phase: A: water (0.225% formic acid), B: acetonitrile; method: B: 8%-38%) to give a formate salt of compound **6A.**

¹H NMR (400 MHz, DMSO-*d*₆) δ ppm 10.83 (br s, 1H), 8.18(s, 1H), 7.98 (d, *J*=8.4 Hz, 2H), 7.76 (br d, *J*=8.4 Hz, 2H), 7.27 (br s, 1H), 6.67 (s, 1H), 6.51 (br s, 1H), 3.74 (s, 3H), 3.70 (s, 1H), 3.60 (br s, 1H), 3.23 (s, 3H), 3.09 (br s, 1H), 2.43 (s, 3H), 2.26 - 2.18 (m, 1H), 2.04-2.01 (br d, *J*=14.7 Hz, 6H), 1.56 (br d, *J*=14.2 Hz, 1H), 1.45 - 1.35 (m, 1H); LC-MS: *m*/*z* =435.2[M+H]⁺.

An aqueous lithium hydroxide solution (1 M, 2 mL, 10 *eq*) was added to a mixed solution of compound **6-4B** (110 mg, 200.49 µmol, 1 *eq*) in tetrahydrofuran (1 mL) and methanol (1 mL) at 15 °C under nitrogen atmosphere. The reaction solution was warmed to 45 °C and stirred for 12 h. The reaction solution was cooled to room temperature and adjusted to pH 6-7 with glacial acetic acid. The mixture was concentrated under reduced pressure to give a crude product, which was purified by high performance liquid chromatography (column: Phenomenex Synergi C18 Mltra 150 × 25 mm, 10 µm; mobile phase: A: water (0.225% formic acid), B: acetonitrile; method: B: 8%-38%) to give a formate salt of compound **6B.**

¹H NMR (400 MHz, DMSO-*d*₆) δ ppm 10.81 (br s, 1H), 8.18 (s, 8.18, 1H), 7.98 (d, *J*=8.4 Hz, 2H), 7.78 (br d, *J*=8.4 Hz, 2H), 7.28 (br s, 1H), 6.69 (s, 1H), 6.51 (br s, 1H), 3.74 (s, 3H), 3.70 (s, 1H), 3.62 (br s, 1H), 3.25 (s, 3H), 3.08 (br s, 1H), 2.45 (s, 3H), 2.26 - 2.19 (m, 1H), 2.04-2.01 (br d, *J*=14.7 Hz, 6H), 1.58 (br d, *J*=14.2 Hz, 1H), 1.45 - 1.38 (m, 1H); LC-MS: *m*/*z* =435.2[M+H]⁺.

### Example 7

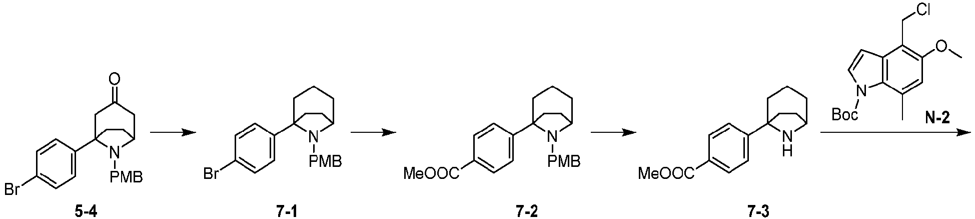

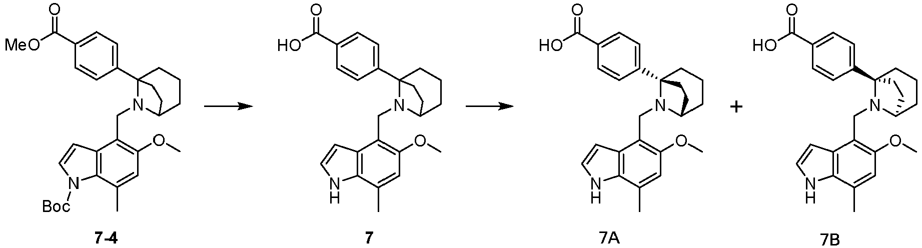

### Step 1

Hydrazine hydrate (354 mg, 6.01 mmol, 343.69 µL, 85% purity, 3.01 *eq*) was added to a solution of compound **5-4** (800.00 mg, 2.00 mmol, 1 *eq*) in ethylene glycol (8 mL) at room temperature, and the reaction system was stirred at 80 °C for 2 h. Then potassium hydroxide (560.62 mg, 9.99 mmol, 5 *eq*) was added to the reaction system, and the mixture was warmed to 180 °C and stirred at this temperature for another 2 h. The reaction solution was cooled to 15 °C and slowly poured into 30 mL of ice water, and then the mixture was extracted twice with 80 mL of ethyl acetate. The organic phases were combined, washed with 20 mL of a saturated aqueous sodium chloride solution, dried over anhydrous sodium sulfate, filtered, and concentrated under reduced pressure. The residue was purified by column chromatography (ethyl acetate:petroleum ether = 1:1) to give compound **7-1.** LC-MS: *m*/*z* = 386.0/388.0[M+H]⁺.

### Step 2

Palladium acetate (406.80 mg, 1.81 mmol, 1 *eq*) and 1,3-bis(diphenylphosphino)propane (747.32 mg, 1.81 mmol, 1 *eq*) were added to a mixed solution of compound **7-1** (700 mg, 1.81 mmol, 1 *eq*) and triethylamine (550.05 mg, 5.44 mmol, 756.61 µL, 3 *eq*) in *N,N*-dimethylformamide (12 mL) and methanol (12 mL) at room temperature. The reaction system was purged with carbon monoxide atmosphere (15 psi) and warmed to 80 °C to react for 12 h. The reaction solution was cooled to 15 °C and filtered through diatomite, the filter cake was washed with 50 mL of ethyl acetate, and the filtrate was collected. The filtrate was washed successively with 10 mL of water and 20 mL of a saturated aqueous sodium chloride solution, and the organic phase was dried over anhydrous sodium sulfate, filtered, and concentrated under reduced pressure. The residue was purified by column chromatography (ethyl acetate:petroleum ether = 1:1) to give compound **7-2.** LC-MS: *m*/*z* = 366.1[M+H]⁺.

### Step 3

Wet palladium on carbon (60 mg, 10% content) was added to a mixed solution of compound **7-2** (600 mg, 1.64 mmol, 1 *eq*) and formic acid (1.51 g, 32.83 mmol, 1.24 mL, 20 *eq*) in methanol (30 mL) at room temperature. The reaction system was warmed to 50 °C to react for 1.5 h. The reaction solution was cooled to 15 °C and filtered to remove the solid. The filtrate was concentrated under reduced pressure to give a crude product of compound **7-3,** which was directly used in the next step. LC-MS: *m*/*z* = 246.1 [M+H]⁺.

### Step 4

Cesium carbonate (1.06 g, 3.26 mmol, 4 *eq*) and potassium iodide (270.67 mg, 1.63 mmol, 2 *eq*) were added to a mixed solution of compound **7-3** (200 mg, 815.27 µmol, 1 *eq*) and compound **N-2** (310 mg, 1.00 mmol, 1.23 *eq*) in *N,N*-dimethylformamide (10 mL) at room temperature, and the reaction system was reacted at 20 °C for 1 h. The reaction solution was quenched by slowly adding to 30 mL of water. The aqueous phase was extracted twice with 30 mL of ethyl acetate. The organic phases were combined, washed with 15 mL of a saturated aqueous sodium chloride solution, dried over anhydrous sodium sulfate, filtered, and concentrated under reduced pressure. The resulting crude product was purified by high performance liquid chromatography (column: Phenomenex luna C18 150 × 25 mm, 10 µm; mobile phase: A: water (0.225% formic acid), B: acetonitrile; method: B: 25%-55%) to give compound **7-4.** LC-MS: *m*/*z* = 519.3[M+H]⁺.

### Step 5

An aqueous lithium hydroxide solution (1 M, 2.4 mL, 4.15 *eq*) was added to a mixed solution of compound **7-4** (300 mg, 578.43 µmol, 1 *eq*) in tetrahydrofuran (6 mL) and methanol (6 mL) at 15 °C under nitrogen atmosphere. The reaction solution was warmed to 45 °C and stirred for 24 h. The reaction solution was cooled to 15 °C and adjusted to pH 5 with glacial acetic acid. The mixture was concentrated under reduced pressure to give a crude product, which was purified by high performance liquid chromatography (column: Phenomenex Synergi C18 Mltra 150 × 25 mm, 10 µm; mobile phase: A: water (0.225% formic acid), B: acetonitrile; method: B: 9%-39%) to give compound **7.** LC-MS: *m*/*z* = 405.1[M+H]⁺.

### Step 6

Compound **7** was separated by chiral column chromatography (column: Daicel ChiralPak IE 250 × 30 mm, 10 µm; mobile phase: A: *n*-hexane, B: ethanol (0.1% ammonium hydroxide); method: B: 55%-55%, 20 min, then maintaining B at 55%, 480 min) to give compound **7A** (retention time: 3.852 min, ee = 100%; analytical method: column: Chiralpak IE 50 × 4.6 mm I.D., 3 µm; mobile phase: A: *n*-heptane, B: ethanol (0.05% diethylamine), gradient B%: 0%-40%), ¹H NMR (400 MHz, DMSO-*d₆*): δ ppm 13.71 - 12.63 (m, 1H), 11.35 - 11.04 (m, 1H), 9.35 (br d, *J* = 5.8 Hz, 1H), 8.18 - 7.91 (m, 2H), 7.90 - 7.72 (m, 1H), 7.46 - 7.26 (m, 1H), 6.73 (s, 1H), 6.40 - 6.25 (m, 1H), 4.10 - 3.91 (m, 3H), 3.90 - 3.81 (m, 3H), 2.94 - 2.87 (m, 1H), 2.62 (br s, 2H), 2.50 (br s, 3H), 2.44 - 2.25 (m, 1H), 2.20 - 1.99 (m, 2H), 1.97 - 1.76 (m, 1H), 1.74 - 1.53 (m, 3H); LC-MS: *m*/*z* =405.1[M+H]⁺; and compound **7B** (retention time: 5.651 min, ee = 100%; analytical method: column: Chiralpak IE 50 × 4.6 mm I.D., 3 µm; mobile phase: A: *n*-heptane, B: ethanol (0.05% diethylamine), gradient B%: 0%-40%). ¹H NMR (400 MHz, DMSO-*d₆*): δ ppm 13.64 - 12.44 (m, 1H), 11.30 - 11.11 (m, 1H), 9.62 - 9.34 (m, 1H), 8.18 - 7.92 (m, 2H), 7.91 - 7.69 (m, 1H), 7.45 - 7.27 (m, 1H), 6.85 - 6.75 (m, 1H), 6.41 - 6.32 (m, 1H), 4.09 - 3.90 (m, 3H), 3.88 - 3.81 (m, 3H), 2.97 - 2.85 (m, 1H), 2.75 - 2.57 (m, 2H), 2.49 - 2.48 (m, 3H), 2.43 - 2.31 (m, 1H), 2.19 - 2.05 (m, 2H), 1.94 - 1.77 (m, 1H), 1.73 - 1.54 (m, 3H); LC-MS: *m*/*z* =405.1[M+H]⁺.

### Example 8

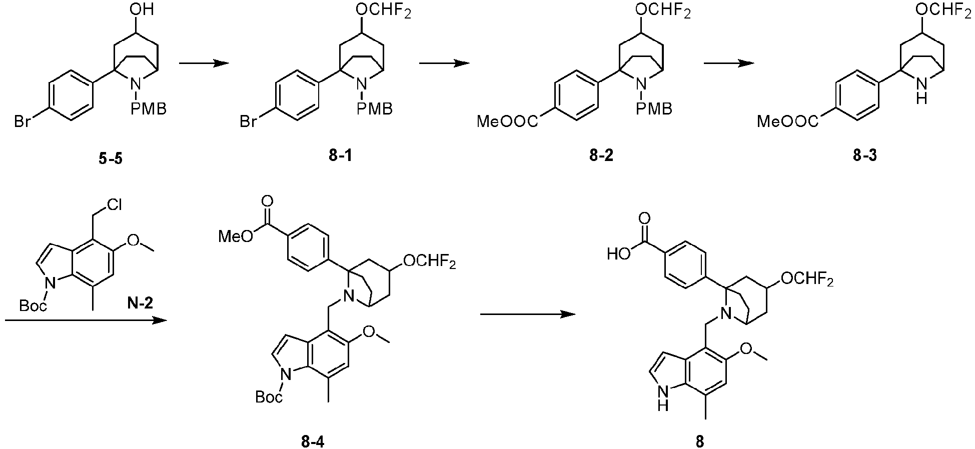

### Step 1

A solution of 2-(fluorosulfonyl)difluoroacetic acid (1.33 g, 7.46 mmol,772.06 µL, 3 *eq*) in acetonitrile (4 mL) was added to a solution of compound **5-5** (1 g, 2.49 mmol, 1 *eq*) and copper(I) iodide (47.34 mg, 248.56 µmol, 0.1 *eq*) in acetonitrile (10 mL) at room temperature, and the reaction system was stirred at 60 °C for 1 h. The reaction solution was cooled to 20 °C and slowly added to 15 mL of water, and then the mixture was extracted twice with 60 mL of ethyl acetate. The organic phases were combined, washed with 10 mL of a saturated aqueous sodium chloride solution, dried over anhydrous sodium sulfate, filtered, and concentrated under reduced pressure. The residue was purified by column chromatography (ethyl acetate:petroleum ether:methanol = 1:15:2) to give compound **8-1.** LC-MS: *m*/*z* = 452.1/454.1[M+H]⁺.

### Step 2

Palladium acetate (74.45 mg, 331.62 µmol, 1 *eq*) and 1,3-bis(diphenylphosphino)propane (136.77 mg, 331.62 µmol, 1 *eq*) were added to a mixed solution of compound **8-1** (150 mg, 331.62 µmol, 1 *eq*) and diisopropylethylamine (128.58 mg, 331.62 µmol, 173.28 µL, 3 *eq*) in *N,N*-dimethylformamide (1.5 mL) and methanol (1.5 mL) at room temperature. The reaction system was purged with carbon monoxide atmosphere (15 psi) and warmed to 75 °C to react for 24 h. The reaction solution was cooled to 20 °C and added into 10 mL of water, and the mixture was extracted with 20 mL of ethyl acetate. Then the mixture was washed with 10 mL of a saturated aqueous sodium chloride solution, and the organic phase was dried over anhydrous sodium sulfate, filtered, and concentrated under reduced pressure. The residue was purified by column chromatography (ethyl acetate:petroleum ether = 1:5) to give compound **8-2.** LC-MS: *m*/*z* = 432.1[M+H]⁺.

### Step 3

Wet palladium on carbon (15 mg, 10% content) was added to a mixed solution of compound **8-2** (70 mg, 162.24 µmol, 1 *eq*) and formic acid (155.88 mg, 3.24 mmol, 20 *eq*) in methanol (2 mL) at room temperature. The reaction system was warmed to 50 °C to react for 1 h. The reaction solution was cooled to 20 °C and filtered to remove the solid. The filtrate was concentrated under reduced pressure to give a crude product of a formate salt of compound **8-3,** which was directly used in the next step. LC-MS: *m*/*z* = 312.1[M+H]⁺.

### Step 4

Cesium carbonate (200.59 mg, 615.65 µmol, 4 *eq*) and potassium iodide (51.10 mg, 307.82 µmol, 2 *eq*) were added to a mixed solution of compound **8-3** (55 mg, crude product of formate salt) and compound **N-2** (71.52 mg, 230.87 µmol, 1.5 *eq*) in *N,N-*dimethylformamide (1 mL) at room temperature, and the reaction system was reacted at 20 °C for 24 h. The reaction solution was quenched by slowly adding to 10 mL of water. The aqueous phase was extracted twice with 16 mL of ethyl acetate. The organic phases were combined, washed with 5 mL of a saturated aqueous sodium chloride solution, dried over anhydrous sodium sulfate, filtered, and concentrated under reduced pressure. The resulting crude product was purified by preparative plate chromatography (petroleum ether:ethyl acetate = 8:1) to give compound **8-4.** LC-MS: *m*/*z* = 585.3[M+H]⁺.

### Step 5

An aqueous lithium hydroxide solution (1 M, 598.65 µL, 10 *eq*) was added to a mixed solution of compound **8-4** (35 mg, 59.86 µmol, 1 *eq*) in tetrahydrofuran (1 mL) and methanol (1 mL) at 15 °C under nitrogen atmosphere. The reaction solution was warmed to 50 °C and stirred for 12 h. The reaction solution was cooled to 20 °C and adjusted to pH 7 with glacial acetic acid. The mixture was concentrated under reduced pressure to give a crude product, which was purified by high performance liquid chromatography (column: Phenomenex Luna C18 75 × 30 mm, 3 µm; mobile phase: A: water (0.05% hydrochloric acid), B: acetonitrile; method: B: 26%-45%) to give a hydrochloride of compound **8.**

¹H NMR (400 MHz, DMSO-*d₆*): δ ppm 11.24 (br s, 1H), 9.96 - 9.71 (m, 1H), 8.14 (br d, *J* = 7.9 Hz, 2H), 8.02 - 7.73 (m, 2H), 7.47 - 7.37 (m, 1H), 6.81 (s, 1H), 6.99 - 6.48 (t, J=7.2 Hz, 1H), 6.40 - 6.33 (m, 1H), 4.52 - 4.43 (m, 1H), 4.12 - 4.02 (m, 2H), 4.00 - 3.92 (m, 1H), 3.90 - 3.83 (m, 3H), 3.04 - 2.88 (m, 2H), 2.57 (br s, 4H), 2.49 - 2.48 (m, 3H), 2.13 - 2.01 (m, 2H); LC-MS: *m*/*z* =471.2[M+H]⁺.

### Example 9

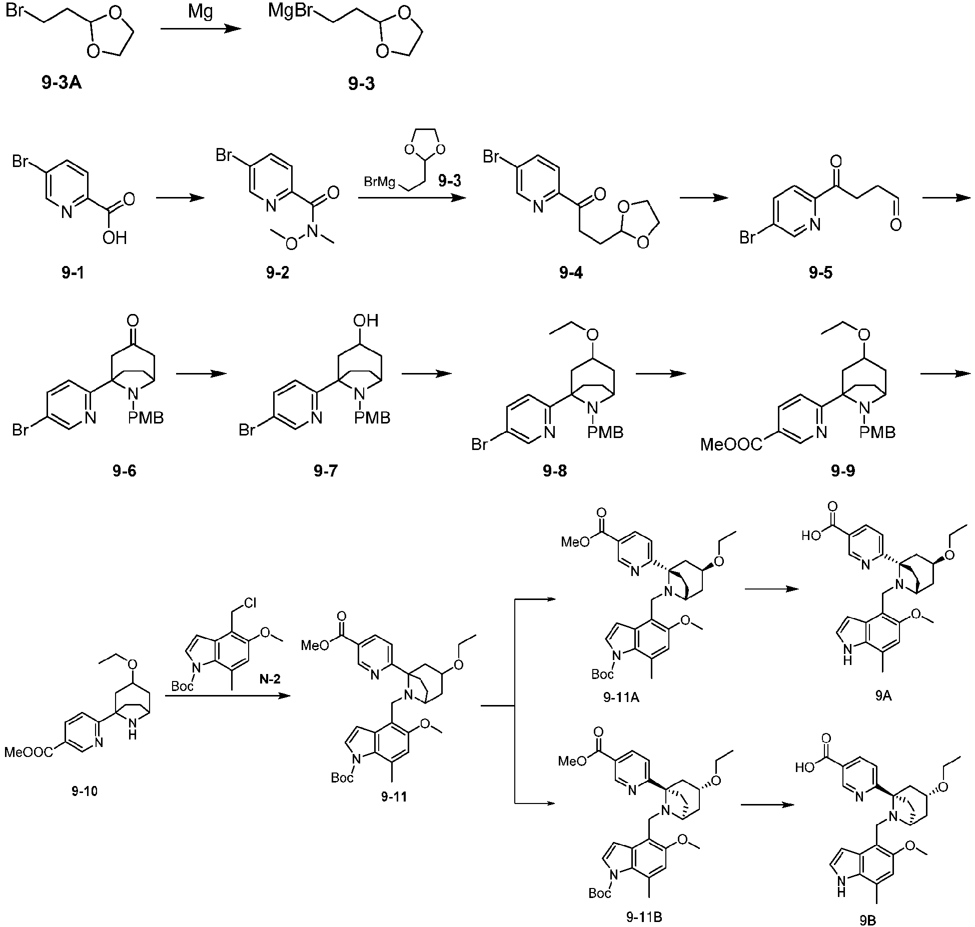

### Step 1

Magnesium chips (402.78 g, 16.57 mol, 5 *eq)* were added to tetrahydrofuran (3000 mL) at 30 °C under nitrogen atmosphere, compound **9-3A** (60 g, 40 mL, 0.1 *eq*) was added slowly, and then iodine (3.0 g) was added to initiate the reaction. The reaction solution was stirred at 30 °C for 0.5 h under nitrogen atmosphere. Then the remaining compound **9-3A** (540.0 g, 360 mL, 0.9 *eq*) was added dropwise to the reaction solution over a period of about 2 h.

After the dropwise addition, the reaction solution was stirred at 30 °C for 0.5 h under nitrogen atmosphere to give a solution of compound 9-3 (concentration: 0.66 M by iodometric titration) in tetrahydrofuran, which was directly used in the next step.

### Step 2

Triethylamine (6.01 g, 59.40 mmol, 8.27 mL, 1.2 *eq*) and 1-(3-dimethylaminopropyl)-3-ethylcarbodiimide hydrochloride (11.39 g, 59.40 mmol, 1.2 *eq*) were added to a solution of compound **9-1** (10 g, 49.50 mmol, 1 *eq*) and methoxymethylamine hydrochloride (5.79 g, 59.40 mmol, 1.2 *eq*) in dichloromethane (100 mL) at 0 °C, and the reaction solution was reacted at 25 °C for 1 h. Water (100 mL) was added to the reaction solution, and the mixture was extracted 3 times with dichloromethane (300 mL). The combined organic layers were washed twice with saturated brine (300 mL), dried over anhydrous sodium sulfate, and filtered, and the filtrate was concentrated under reduced pressure to give compound **9-2.** LCMS: *m*/*z* = 243.4/245.4[M+H]⁺.

### Step 3

A solution of compound **9-3** in tetrahydrofuran (0.66 M, 40.80 mL, 1.1 *eq*) was slowly added dropwise to a solution of compound **9-2** (6 g, 24.48 mmol, 1 *eq*) in tetrahydrofuran (100 mL) at -20 °C under nitrogen atmosphere. After the addition, the reaction solution was stirred at -20 °C for 0.5 h. A saturated ammonium chloride solution (100 mL) was added dropwise to the reaction solution, the aqueous phase was extracted twice with ethyl acetate (100 mL), and the combined organic phases were washed twice with saturated brine (100 mL), dried over anhydrous sodium sulfate, and filtered. The filtrate was concentrated under reduced pressure, and the residue was separated and purified by silica gel column chromatography (ethyl acetate:petroleum ether = 1:5) to give compound **9-4.** LCMS: *m*/*z* = 284.2/286.2[M+H]⁺.

### Step 4

Compound **9-4** (6 g, 20.97 mmol, 1 *eq*) was dissolved in formic acid (48 mL) and water (12 mL), and the mixture was stirred at 45 °C for 1 h under nitrogen atmosphere. To the reaction solution was added 100 mL of water, and the pH was adjusted to 7 with potassium carbonate. The mixture was extracted 3 times with ethyl acetate (60 mL), and the combined organic phases were washed 3 times with a saturated sodium bicarbonate solution (300 mL), dried over anhydrous sodium sulfate, and filtered. The filtrate was concentrated under reduced pressure, the residue was dispersed in 50 mL of water, and the mixture was stirred at 25 °C for 30 min and then filtered to give compound **9-5.** LCMS: *m*/*z* = 242.3/244.3[M+H]⁺.

### Step 5

1,3-Acetonedicarboxylic acid (10.14 g, 69.40 mmol, 4 *eq*) and diluted hydrochloric acid (1 M, 21.69 mL, 1.25 *eq*) were added to a solution of *p*-methoxybenzylamine (4.76 g, 34.70 mmol, 4.49 mL, 2 *eq*) and sodium acetate (9.01 g, 109.83 mmol, 6.33 *eq*) in water (40 mL) at 0 °C, and the mixture was stirred at the temperature for 1 h under nitrogen atmosphere. Then a solution of compound **9-5** (4.2 g, 17.35 mmol, 1 *eq*) in tetrahydrofuran (40 mL) was added, and the reaction solution was stirred at 40 °C for 3 h. The reaction solution was diluted with water (40 mL), the mixture was extracted 3 times with ethyl acetate (300 mL), and the combined organic phases were washed 3 times with saturated brine (300 mL), dried over anhydrous sodium sulfate, and filtered. The filtrate was concentrated under reduced pressure, and the residue was separated and purified by silica gel column chromatography (ethyl acetate:petroleum ether = 1:10 to 1:5) to give compound **9-6.** LCMS: *m*/*z* = 399.2/401.2[M+H]⁺.

### Step 6

Lithium tri-*sec*-butylborohydride (1 M, 14.35 mL, 1.2 *eq*) was added to a solution of compound **9-6** (4.80 g, 11.96 mmol, 1 *eq*) in tetrahydrofuran (50 mL) at -70 °C, and the reaction solution was stirred at the temperature for 1 h. Methanol (50 mL) was slowly added dropwise to quench the reaction solution, then the reaction solution was concentrated under reduced pressure, and the residue was separated and purified by silica gel column chromatography (ethyl acetate:petroleum ether = 1:5 to 1:3) to give compound **9-7.** LCMS: *m*/*z* = 401.2/4032[M+H]⁺.

### Step 7

Sodium hydride (2.98 g, 74.38 mmol, 60% purity, 10 *eq*) was added to a solution of compound **9-7** (3 g, 7.44 mmol, 1 *eq*) in *N*,*N*-dimethylformamide (50 mL) at 40 °C, the reaction solution was stirred at the temperature for 1 h, then iodoethane (3.48 g, 22.32 mmol, 1.78 mL, 3 *eq*) was added, and the mixture was stirred for another 12 h. The reaction solution was cooled to room temperature and slowly added to water (200 mL), the mixture was extracted twice with ethyl acetate (400 mL), and the combined organic phases were washed twice with saturated brine (400 mL), dried over anhydrous sodium sulfate, and filtered. The filtrate was concentrated under reduced pressure, and the residue was separated and purified by silica gel column chromatography (ethyl acetate:petroleum ether = 1:10 to 1:3) to give compound **9-8.** LCMS: *m*/*z* = 429.2/431.2[M+H]⁺.

### Step 8

Palladium acetate (572.51 mg, 2.55 mmol, 1 *eq*)*,* 1,3-bis(diphenylphosphino)propane (1.05 g, 2.55 mmol, 1 eq), and *N,N-*diisopropylethylamine (988.70 mg, 7.65 mmol, 1.33 mL, 3 *eq*) were added to a solution of compound **9-8** (1.1 g, 2.55 mmol, 1 *eq*) in *N*,*N*-dimethylformamide (10 mL) and methanol (10 mL). The reaction solution was purged with carbon monoxide several times and reacted at 75 °C for 16 h under carbon monoxide atmosphere. The reaction solution was diluted with water (4 mL), then the mixture was extracted 3 times with ethyl acetate (15 mL), and the combined organic phases were washed twice with saturated brine (20 mL), dried over anhydrous sodium sulfate, and filtered. The filtrate was concentrated under reduced pressure, and the residue was separated and purified by preparative plate chromatography (ethyl acetate:petroleum ether = 1:5) to give compound **9-9.** LCMS: *m*/*z* = 411.2[M+H]⁺.

### Step 9

Compound **9-9** (0.78 g, 1.90 mmol, 1 *eq*)*,* wet palladium on carbon (100 mg, 10% content), and ammonium formate (1.20 g, 19.00 mmol, 10 *eq*) were dissolved in methanol (10 mL) and purged with nitrogen 3 times, and the reaction solution was stirred at 70 °C for 1 h under nitrogen atmosphere. The reaction solution was cooled and filtered, and the resulting filtrate was concentrated under reduced pressure to give compound **9-10.** LCMS: *m*/*z* = 291.2[M+H]⁺.

### Step 10

Potassium iodide (228.69 mg, 1.38 mmol, 2 *eq*) and cesium carbonate (897.71 mg, 2.76 mmol, 4 *eq*) were added to a solution of compound **9-10** (0.2 g, 688.81 µmol, 1 *eq*) and compound **N-2** (256.06 mg, 826.57 µmol, 1.2 *eq*) in *N,N-*dimethylformamide (4 mL) at 25 °C, and the mixture was stirred at the temperature for 2 h. Water (10 mL) was added to the reaction solution, then the mixture was extracted 3 times with ethyl acetate (30 mL), and the combined organic phases were washed twice with saturated brine (30 mL), dried over anhydrous sodium sulfate, and filtered. The filtrate was concentrated under reduced pressure, and the residue was separated and purified by preparative plate chromatography (ethyl acetate:petroleum ether = 1:2) and then purified by high performance liquid chromatography (column: Phenomenex C18 75 × 30 mm × 3 µm; mobile phase: A: water (formic acid), B: acetonitrile; method: B: 28%-58%) to give compound **9-11,** which was then separated by chiral column chromatography (column: DAICEL CHIRALPAK AD (250 mm × 30 mm, 10 µm), mobile phase: A: CO₂, B: [0.1% ammonium hydroxide-isopropanol]; gradient B%: 35%-35%, 70 min) to give compound **9-11A** (retention time: 1.86 min, ee = 99.23%) and **9-11B** (retention time: 1.96 min, ee = 100%), respectively. SFC analytical method: column: Chiralpak AS-3 50 × 4.6 mm I.D., 3 µm; mobile phase: A: CO₂, B: methanol (0.05% diethylamine); gradient: B%: 5%-40%. LCMS: *m*/*z* = 564.4[M+H]⁺.

### Step 11

Lithium hydroxide (1 M, 443.51 µL, 10 *eq*) was added to a solution of compound **9-11A** (25 mg, 44.35 µmol, 1 *eq*) in tetrahydrofuran (2 mL) and methanol (2 mL), and the mixture was stirred at 45 °C for 12 h. The reaction solution was concentrated, and the resulting residue was adjusted to pH 7 with acetic acid, and then concentrated under reduced pressure to give a residue, which was purified by high performance liquid chromatography (column: Phenomenex C18 75 × 30 mm × 3 µm; mobile phase: A: water (0.225% formic acid), B: acetonitrile; method: B: 15%-45%) to give compound **9A.**

¹H NMR (400 MHz, CD₃OD) δ ppm 9.23 (s, 1H), 8.35 (t, *J* = 1.0 Hz, 1H), 7.59 (d, *J* = 1.0 Hz, 1H), 7.28 (s, 1H), 6.74 (s, 1H), 6.59 - 6.28 (m, 1H), 4.65 - 4.50 (m, 2H), 4.03 - 3.88 (m, 2H), 3.88 - 3.82 (m, 3H), 3.55 - 3.38 (m, 2H), 3.27 (td, *J* = 1.6, 3.2 Hz, 2H), 2.94 - 2.78 (m, 1H), 2.73 - 2.62 (m, 2H), 2.62 - 2.50 (m, 2H), 2.47 (s, 3H), 2.12 - 2.02 (m, 1H), 1.18 (t, *J* = 1.0 Hz, 3H); LCMS: *m*/*z*= 450.3[M+H]⁺.

### Step 12

Lithium hydroxide (1 M, 532.21 µL, 10 *eq*) was added to a solution of compound **9-11B** (30 mg, 53.22 µmol, 1 *eq*) in tetrahydrofuran (1 mL) and methanol (1 mL), and the mixture was stirred at 45 °C for 12 h. The reaction solution was concentrated, and the resulting residue was adjusted to pH 7 with acetic acid, and then concentrated under reduced pressure to give a residue, which was purified by high performance liquid chromatography (column: Phenomenex C18 75 × 30 mm × 3 µm; mobile phase: A: water (0.225% formic acid), B: acetonitrile; method: B: 15%-45%) to give compound **9B.**

¹H NMR (400 MHz, CD₃OD) δ ppm 9.30 (s, 1H), 8.41 (t, *J* = 1.0 Hz, 1H), 7.66 (d, *J* = 1.0 Hz, 1H), 7.33 (t, *J* = 1.0 Hz, 1H), 6.80 (s, 1H), 6.60 - 6.42 (m, 1H), 4.68 - 4.56 (m, 2H), 4.32 - 4.12 (m, 1H), 4.09 - 3.94 (m, 2H), 3.95 - 3.87 (m, 3H), 3.87 - 3.66 (m, 1H), 3.59 - 3.45 (m, 2H), 2.97 - 2.80 (m, 1H), 2.79 - 2.63 (m, 2H), 2.57 - 2.45 (m, 4H), 2.15 - 1.99 (m, 2H), 1.28 - 1.17 (m, 3H); LCMS: *m*/*z*= 450.3[M+H]⁺.

### Example 10

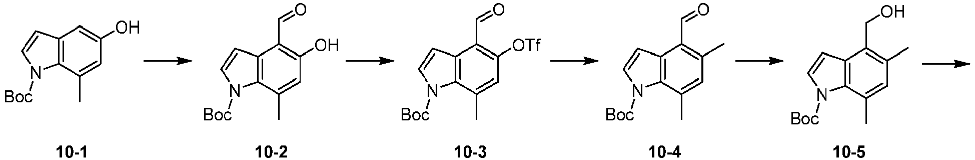

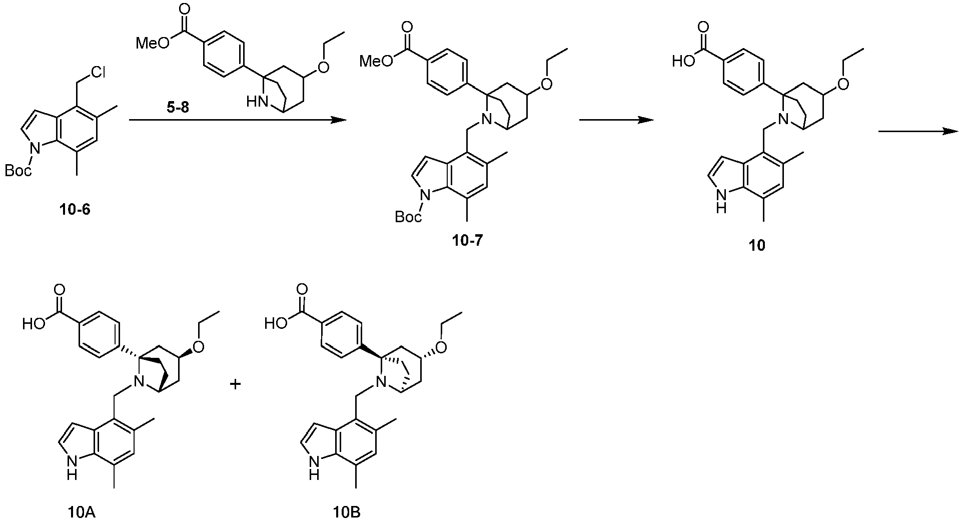

### Step 1

A Grignard reagent of methylmagnesium bromide (3 M, 5.39 mL, 1 *eq*) was added to a solution of compound **10-1** (4 g, 16.18 mmol, 1 *eq*) in tetrahydrofuran (80 mL) at 20 °C, then polyoxymethylene (0.5 g, 48.53 mmol, 3 *eq*) was added, and the reaction solution was warmed to 70 °C and stirred for another 2 h. After being cooled to room temperature, the reaction solution was quenched with a saturated aqueous ammonium chloride solution (100 mL) at 0 °C, and the mixture was extracted twice with ethyl acetate (100 mL). The organic layer was dried over anhydrous sodium sulfate and filtered, and the filtrate was concentrated under reduced pressure. The residue was slurried with ethyl acetate (20 mL) for 30 min and filtered, and the filter cake was collected to give compound **10-2.** LC-MS:*m*/*z* = 276.1 [M+H]⁺.

### Step 2

*N*-Phenylbis(trifluoromethanesulfonyl)imide (1.95 g, 5.45 mmol, 1.5 *eq*) and 4-dimethylaminopyridine (887.53 mg, 7.26 mmol, 2 *eq*) were added to a solution of compound **10-2** (1 g, 3.63 mmol, 1 *eq*) in dichloromethane (25 mL) at 0 °C. After the addition, the reaction solution was warmed to 25 °C and stirred for 12 h. Water (15 mL) was added to quench the reaction solution, and the mixture was extracted once with ethyl acetate (30 mL). The combined organic phases were dried over anhydrous sodium sulfate and filtered. The filtrate was concentrated under reduced pressure, and the residue was separated and purified by silica gel column chromatography (ethyl acetate:petroleum ether = 1:50) to give compound **10-3.**

¹H NMR (400 MHz, CDCl₃) δ ppm 10.52 - 10.37 (m, 1H), 7.78 - 7.65 (m, 1H), 7.50 - 7.40 (m, 1H), 7.08 - 6.97 (m, 1H), 2.72 - 2.57 (m, 3H), 1.58 (s, 9H).

### Step 3

Methylboronic acid (690.00 mg, 11.53 mmol, 4.08 *eq*)*,* [1,1-bis(diphenylphosphino)ferrocene]palladium chloride (206.56 mg, 282.30 µmol, 0.1 *eq*)*,* and potassium carbonate (1.17 g, 8.47 mmol, 3 *eq*) were added to a solution of compound **10-3** (1.15 g, 2.82 mmol, 1 *eq*) in tetrahydrofuran (10 mL) and water (2.5 mL) at 25 °C. The mixture was purged with nitrogen 3 times, warmed to 65 °C, and stirred for another 12 h under nitrogen atmosphere. Water (15 mL) was added to quench the reaction solution, and the mixture was extracted twice with ethyl acetate (30 mL). The organic phases were combined, washed with saturated brine (10 mL), dried over anhydrous sodium sulfate, and filtered. The filtrate was concentrated under reduced pressure, and the residue was separated and purified by silica gel column chromatography (ethyl acetate:petroleum ether = 1:50) to give compound **10-4.**

¹H NMR (400 MHz, CDCl₃) δ ppm 10.70 - 10.60 (m, 1H), 7.66 (d, *J* = 3.8 Hz, 1H), 7.47 - 7.36 (m, 1H), 7.04 - 6.89 (m, 1H), 2.75 - 2.73 (m, 3H), 2.68 (s, 3H), 1.70 - 1.64 (m, 9H).

### Step 4

Sodium borohydride (38 mg, 1.00 mmol, 2.75 *eq*) was added to a solution of compound **10-4** (100 mg, 365.86 µmol, 1 *eq*) in methanol (1.5 mL) and tetrahydrofuran (1.5 mL) at 0 °C. The mixture was stirred at 0 °C for 1 h. The reaction solution was quenched by adding a 0.5 M aqueous hydrochloric acid solution (3 mL) and then diluted with water (5 mL), and the mixture was extracted twice with ethyl acetate (8 mL). The organic phases were combined, washed with saturated brine (8 mL), dried over anhydrous sodium sulfate, and filtered. The filtrate was concentrated under reduced pressure to give compound **10-5,** which was directly used in the next step. LC-MS: *m*/*z* = 276.1 [M+H]⁺.

### Step 5

(Chloromethylene)dimethylammonium chloride (334.71 mg, 2.61 mmol, 1.5 *eq*) was added to a solution of compound **10-5** (480 mg, 1.74 mmol, 1 *eq*) in dichloromethane (10 mL) at 0 °C, and the reaction solution was stirred at 20 °C for 1 h. A 5% aqueous sodium bicarbonate solution (10 mL) was added to the reaction solution at 0 °C, and the mixture was stirred for 5 min. The mixture was extracted once with dichloromethane (10 mL). The combined organic phases were washed once successively with lithium chloride (10 mL) and saturated brine (10 mL), dried over anhydrous sodium sulfate, and filtered, and the filtrate was concentrated under reduced pressure to give compound **10-6,** which was directly used in the next step.

¹H NMR (400 MHz, CDCl₃) δ ppm 7.59 - 7.53 (m, 1H), 6.95 (s, 1H), 6.66 - 6.60 (m, 1H), 4.89 - 4.84 (m, 2H), 2.60 (s, 3H), 2.49 - 2.44 (m, 3H), 1.63 (s, 9H).

### Step 6

Intermediate **5-8** (280 mg, 967.62 µmol, 1 *eq*) was added to a solution of compound **10-6** (341.13 mg, 1.16 mmol, *1.2 eq*) in *N,N-*dimethylformamide (10 mL) at 25 °C, and potassium iodide (321.26 mg, 1.94 mmol, 2 *eq*) and cesium carbonate (1.26 g, 3.87 mmol, 4 *eq*) were added to the mixed solution successively. The mixture was stirred at 25 °C for 12 h under nitrogen atmosphere. The reaction solution was quenched with water (10 mL), the mixture was extracted once with ethyl acetate (30 mL), and the organic phases were combined, washed with saturated brine (10 mL), dried over anhydrous sodium sulfate, and filtered. The filtrate was concentrated under reduced pressure, and the residue was separated and purified by silica gel column chromatography (ethyl acetate:petroleum ether = 1:20) to give compound **10-7.** LC-MS: *m*/*z* = 547.3[M+H]⁺.

### Step 7

Lithium hydroxide monohydrate (1 M, 3.29 mL, 10 *eq*) was added to a solution of compound **10-7** (180 mg, 329.25 µmol, 1 *eq*) in tetrahydrofuran (5 mL) and methanol (5 mL) at 25 °C. The mixture was warmed to 50 °C and stirred for 12 h. The reaction solution was cooled to room temperature, then adjusted to pH 7 with an acetic acid solution, and concentrated under reduced pressure, and the residue was purified by high performance liquid chromatography (column: Phenomenex Gemini-NX C18 75 × 30 mm, 3 µm; mobile phase: A: water (0.225% formic acid), B: acetonitrile; method: B: 15%-45%) to give compound **10,** which was then separated by chiral column chromatography (column: ChiralPak AD 250 × 30 mm, 10 µm; mobile phase: A: carbon dioxide, B: 50% isopropanol and 50% acetonitrile (0.05% diethylamine); gradient B%: 50%-50%, 80 min) to give compound **10A** and compound **10B.** SFC analytical method: column: Chiralpak AD 50 × 4.6 mm I.D., 3 µm; mobile phase: A: CO₂, B: ethanol (0.05% diethylamine); gradient: B%: 0%-40%.

Compound **10A** (retention time: 1.1 min, ee = 98.5%): ¹H NMR (400 MHz, DMSO-*d₆*) δ ppm 10.83 (br s, 1H), 7.94 (d, *J* = 8.2 Hz, 2H), 7.68 (br d, *J* = 8.1 Hz, 2H), 7.22 (t, *J* = 2.6 Hz, 1H), 6.77 - 6.57 (m, 2H), 3.77 - 3.64 (m, 2H), 3.28 - 3.20 (m, 3H), 2.90 (br d, *J* = 4.4 Hz, 1H), 2.51 - 2.41 (m, 1H), 2.38 (d, *J* = 3.3 Hz, 6H), 2.34 - 2.22 (m, 3H), 2.08 - 1.91 (m, 3H), 1.85 - 1.69 (m, 1H), 1.49 (br d, *J* = 14.2 Hz, 1H), 1.10 (t, *J* = 6.9 Hz, 3H); LC-MS: *m*/*z* = 433.2[M+H]⁺.

Compound **10B** (retention time: 1.5 min, ee = 94.7%): ¹H NMR (400 MHz, DMSO-*d₆*) δ = 11.00 - 10.78 (m, 1H), 8.04 - 7.91 (m, 2H), 7.82 - 7.62 (m, 2H), 7.37 - 7.12 (m, 1H), 6.83 - 6.48 (m, 2H), 3.72 - 3.66 (m, 2H), 3.27 - 3.17 (m, 3H), 2.99 - 2.82 (m, 1H), 2.52 - 2.44 (m, 1H), 2.38 (d, J = 3.3 Hz, 6H), 2.31 - 2.20 (m, 3H), 2.18 - 1.87 (m, 3H), 1.68 (s, 1H), 1.57 - 1.37 (m, 1H), 1.10 (t, *J* = 7.0 Hz, 3H); LC-MS: *m*/*z* = 433.2[M+H]⁺.

### Example 11

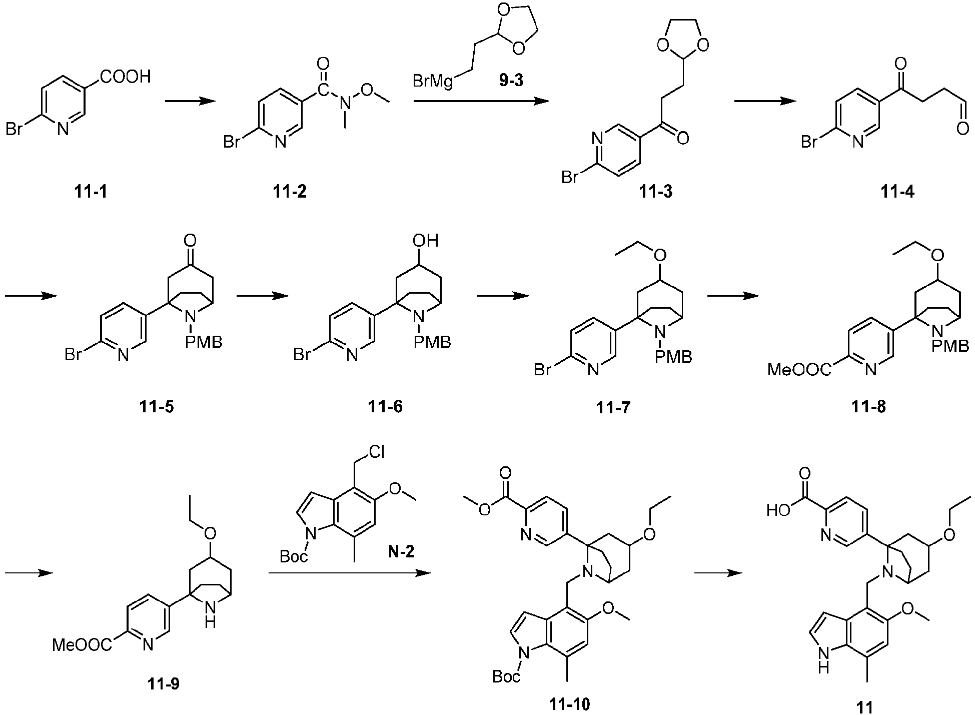

### Step 1

Triethylamine (7.81 g, 77.23 mmol, 10.75 mL, 1.2 *eq*) and 1-ethyl-(3-dimethylaminopropyl)carbodiimine hydrochloride (14.80 g, 77.23 mmol, 1.2 *eq*) were added to a solution of compound **11-1** (13 g, 64.35 mmol, 1 *eq*) and methoxymethylamine hydrochloride (7.53 g, 77.23 mmol, 1.2 *eq*) in dichloromethane (200 mL) at 0 °C, and the reaction solution was reacted at 25 °C for 1 h. Water (40 mL) was added to the reaction solution, and the mixture was extracted 3 times with dichloromethane (120 mL). The combined organic layers were washed twice with saturated brine (120 mL), dried over anhydrous sodium sulfate, and filtered, and the filtrate was concentrated under reduced pressure to give compound **11-2.** LCMS: *m*/*z* = 243.3/245.3 [M+H]⁺.

### Step 2

A solution of compound **9-3** in tetrahydrofuran (0.66 M, 91.04 mL, 1.2 *eq*) was slowly added dropwise to a solution of compound **11-2** (12.3 g, 50.19 mmol, 1 *eq*) in tetrahydrofuran (120 mL) at -20 °C under nitrogen atmosphere. After the addition, the reaction solution was stirred at -20 °C for 0.5 h. A saturated ammonium chloride solution (200 mL) was added dropwise to the reaction solution, the aqueous phase was extracted twice with ethyl acetate (400 mL), and the combined organic phases were washed twice with saturated brine (200 mL), dried over anhydrous sodium sulfate, and filtered. The filtrate was concentrated under reduced pressure, and the residue was separated and purified by silica gel column chromatography (ethyl acetate:petroleum ether = 1:5) to give compound **11-3.** LCMS: *m*/*z* = 286.3/288.3 [M+H]⁺.

### Step 3

Compound **11-3** (16.3 g, 56.97 mmol, 1 *eq*) was dissolved in formic acid (80 mL) and water (20 mL), and the mixture was stirred at 45 °C for 13 h under nitrogen atmosphere. To the reaction solution was added 100 mL of water, and the pH was adjusted to 7 with potassium carbonate. The mixture was extracted 3 times with ethyl acetate (300 mL), and the combined organic phases were washed with a saturated sodium bicarbonate solution (100 mL), dried over anhydrous sodium sulfate, and filtered. The filtrate was concentrated under reduced pressure, and the residue was separated and purified by silica gel column chromatography (ethyl acetate:petroleum ether = 1:3) to give compound **11-4.** LCMS: *m*/*z* = 242.3/244.3[M+H]⁺.

### Step 4

1,3-Acetonedicarboxylic acid (21.73 g, 148.72 mmol, 4 *eq*) and diluted hydrochloric acid (1 M, 46.47 mL, 1.25 eq) were added to a solution of *p*-methoxybenzylamine (10.20 g, 74.36 mmol, 9.62 mL, 2 *eq*) and sodium acetate (19.31 g, 235.35 mmol, 6.33 *eq*) in water (50 mL) at 0 °C, and the mixture was stirred at the temperature for 1 h under nitrogen atmosphere. Then a solution of compound **11-4** (9 g, 37.18 mmol, 1 *eq*) in tetrahydrofuran (50 mL) was added, and the reaction solution was stirred at 40 °C for 13 h. The reaction solution was diluted with water (100 mL), the mixture was extracted 3 times with ethyl acetate (300 mL), and the combined organic phases were washed 3 times with saturated brine (300 mL), dried over anhydrous sodium sulfate, and filtered. The filtrate was concentrated under reduced pressure, and the residue was separated and purified by high performance liquid chromatography (column: Kromasil Eternity XT 250 × 80 mm × 10 µm; mobile phase: water (formic acid)-acetonitrile]; acetonitrile%: 38%-58%) to give compound **11-5.** LCMS: *m*/*z* = 401.2/403.2[M+H]⁺.

### Step 5

Lithium tri-*sec*-butylborohydride (1 M, 11.96 mL, 1.2 *eq*) was added to a solution of compound **11-5** (4 g, 9.97 mmol, 1 *eq*) in tetrahydrofuran (50 mL) at -70 °C, and the reaction solution was stirred at the temperature for 1 h. Methanol (50 mL) was slowly added dropwise to quench the reaction solution, then the reaction solution was concentrated under reduced pressure, and the residue was separated and purified by silica gel column chromatography (ethyl acetate:petroleum ether = 1:3 to 1:2) to give compound **11-6.** LCMS: *m*/*z* = 403.2/405.2[M+H]⁺.

### Step 6

Sodium hydride (3.47 g, 86.78 mmol, 60% purity, 10 *eq*) was added to a solution of compound **11-6** (3.5 g, 8.68 mmol, 1 *eq*) in *N,N-*dimethylformamide (50 mL) at 25 °C, the reaction solution was stirred at 40 °C for 1 h, then iodoethane (4.06 g, 26.03 mmol, 2.08 mL, 3 eq) was added, and the mixture was stirred for another 12 h. The reaction solution was cooled to room temperature and slowly added to water (100 mL), the mixture was extracted 3 times with ethyl acetate (300 mL), and the combined organic phases were washed twice with saturated brine (300 mL), dried over anhydrous sodium sulfate, and filtered. The filtrate was concentrated under reduced pressure, and the residue was separated and purified by silica gel column chromatography (ethyl acetate:petroleum ether = 1:10) to give compound **11-7.** LCMS: *m*/*z* = 429.2/431.2[M+H]⁺.

### Step 7

Palladium acetate (520.46 mg, 2.32 mmol, 1 *eq*)*,* 1,3-bis(diphenylphosphino)propane (956.13 mg, 2.32 mmol, 1 *eq),* and *N,N-*diisopropylethylamine (898.84 mg, 6.95 mmol, 1.21 mL, 3 *eq*) were added to a solution of compound **11-7** (1 g, 2.32 mmol, 1 *eq*) in *N,N*-dimethylformamide (10 mL) and methanol (10 mL). The reaction solution was reacted at 75 °C for 12 h under carbon monoxide atmosphere. The reaction solution was diluted with water (4 mL), then the mixture was extracted 3 times with ethyl acetate (15 mL), and the combined organic phases were washed twice with saturated brine (20 mL), dried over anhydrous sodium sulfate, and filtered. The filtrate was concentrated under reduced pressure, and the residue was separated and purified by preparative plate chromatography (ethyl acetate:petroleum ether = 1:5) to give compound **11-8.** LCMS: *m*/*z* = 411.3 [M+H]⁺.

### Step 8

Ceric ammonium nitrate (3.34 g, 6.09 mmol, 3.04 mL, 5 *eq*) was added to a solution of compound **11-8** (0.5 g, 1.22 mmol, 1 *eq*) in acetonitrile (10 mL) and water (10 mL) at 25 °C, and the mixture was stirred at the temperature for 4 h. The reaction solution was quenched with 10 mL of a saturated sodium carbonate solution and stirred for 10 min, and then the mixture was extracted 3 times with ethyl acetate (30 mL), dried over anhydrous sodium sulfate, and filtered. The filtrate was concentrated under reduced pressure, and the residue was separated and purified by preparative plate chromatography (ethyl acetate:petroleum ether = 1:0) to give compound **11-9.** LCMS: *m*/*z* = 291.2[M+H]⁺.

### Step 9

Potassium iodide (114.34 mg, 688.80 µmol, 2 *eq*) and cesium carbonate (448.85 mg, 1.38 mmol, 4 *eq*) were added to a solution of compound **11-9** (100 mg, 344.40 µmol, 1 *eq*) and compound **N-2** (128.03 mg, 413.28 µmol, 1.2 *eq*) in *N,N*-dimethylformamide (2 mL) at 25 °C, and the mixture was stirred at the temperature for 13 h. Water (5 mL) was added to the reaction solution, then the mixture was extracted 3 times with ethyl acetate (30 mL), and the combined organic phases were washed 3 times with saturated brine (30 mL), dried over anhydrous sodium sulfate, and filtered. The filtrate was concentrated under reduced pressure, and the residue was separated and purified by preparative plate chromatography (ethyl acetate:petroleum ether = 1:5) to give compound **11-10.** LCMS: *m*/*z* = 564.3 [M+H]⁺.

### Step 10

Lithium hydroxide (1 M, 1.18 mL, 10 *eq*) was added to a solution of compound **11-10** (0.1 g, 117.54 µmol, 66% purity, 1 *eq*) in tetrahydrofuran (5 mL) and methanol (5 mL), and the mixture was stirred at 45 °C for 12 h. The reaction solution was concentrated, the resulting residue was adjusted to pH 7 with acetic acid and then concentrated under reduced pressure to give a residue, which was purified by high performance liquid chromatography (column: Phenomenex Unisil 3-100 C18 Ultra 150 × 50 mm × 3 µm; mobile phase: A: water (0.225% formic acid), B: acetonitrile; method: B: 3%-33%) to give compound **11.**

¹H NMR (400 MHz, DMSO-*d₆*) δ ppm 10.91 - 10.77 (m, 1H), 9.03 - 8.87 (m, 1H), 8.18 - 7.97 (m, 2H), 7.34 - 7.15 (m, 1H), 6.70 - 6.60 (m, 1H), 6.52 - 6.44 (m, 1H), 3.84 - 3.66 (m, 5H), 3.47 - 3.38 (m, 4H), 3.15 - 3.06 (m, 1H), 2.44 - 2.40 (m, 3H), 2.30 - 2.20 (m, 1H), 2.14 - 1.94 (m, 5H), 1.58 - 1.48 (m, 1H), 1.17 - 1.07 (m, 3H); LCMS: *m*/*z* = 450.3 [M+H]⁺.

### Example 12

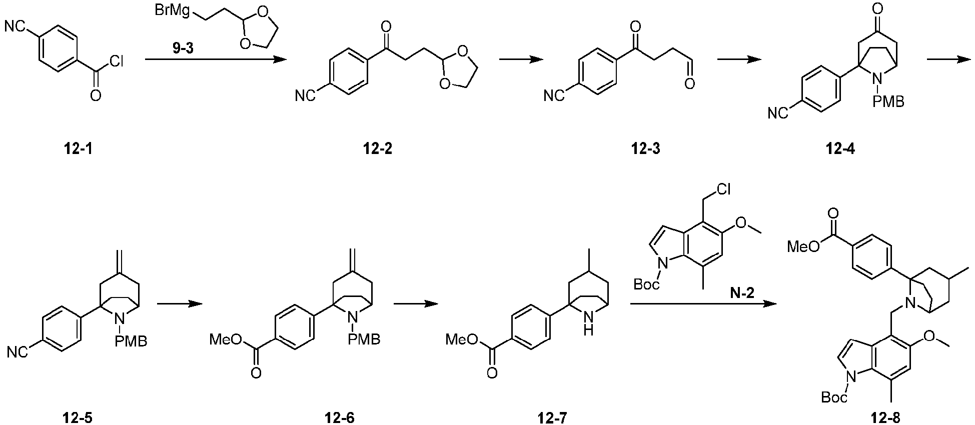

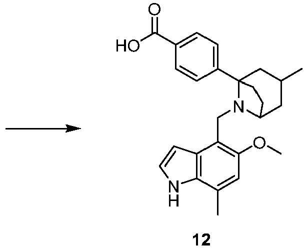

### Step 1

A solution of compound **9-3** in tetrahydrofuran (0.66 M, 2.01 L, 1 *eq*) was added dropwise to a solution of compound **12-1** (200 g, 1.21 mol, 1 *eq*) and cuprous bromide (17.30 g, 0.1 *eq*) in toluene (1000 mL) at -70 °C, and the reaction was carried out under nitrogen atmosphere with a dropwise addition duration of 3 h. After the dropwise addition, the mixture was stirred at -70 °C for 0.5 h. The reaction solution was poured into ice water (2000 mL), the pH was adjusted to 7 with diluted hydrochloric acid (1 M), then the mixture was extracted twice with ethyl acetate (2000 mL), and the organic phases were combined and washed successively with a saturated aqueous potassium carbonate solution (500 mL), a saturated ammonium chloride solution (500 mL), and a saturated sodium chloride solution (500 mL). The organic phase was dried over anhydrous sodium sulfate and concentrated under reduced pressure to give a crude product, which was then slurried with *n*-heptane (1000 mL) at 10-15 °C for 30 min and filtered, and the filter cake was collected to give compound **12-2.** ¹H NMR (400 MHz, CDCl₃) δ ppm 8.07 - 7.94 (m, 2H), 7.80 - 7.65 (m, 2H), 4.93 (t, *J* = 4.1 Hz, 1H), 3.96 - 3.76 (m, 4H), 3.05 (t, *J* = 7.3 Hz, 2H), 2.10 (dt, *J* = 4.1, 7.3 Hz, 2H).

### Step 2

Compound **12-2** (264 g, 1.14 mol, 1 *eq*) was dissolved in a mixed solvent of water (150 mL) and formic acid (1500 mL), and the reaction solution was warmed to 40 °C and stirred at the temperature for 2 h. The reaction solution was cooled to room temperature and slowly added to a 15% aqueous potassium carbonate solution (8 L). A solid was precipitated, the resulting mixture was filtered, and the solid was collected. The filter cake was washed with water (500 mL) and dried to give compound **12-3.** ¹H NMR (400 MHz, CDCl₃) δ ppm 9.92 (s, 1H), 8.20 - 8.03 (m, 2H), 7.95 - 7.68 (m, 2H), 3.51 - 3.22 (m, 2H), 3.09 - 2.88 (m, 2H).

### Step 3

4-Methoxybenzylamine (153.89 g, 1.12 mol, 145.18 mL, 2.1 *eq*) and potassium acetate (277.40 g, 3.38 mol, 6.33 *eq*) were dissolved in water (1000 mL), then concentrated hydrochloric acid (12 M, 53.42 mL, 1.2 *eq*) was added, and the mixture was cooled to 0 °C. Then the compound 1,3-acetonedicarboxylic acid (312.18 g, 2.14 mol, 4 *eq*) was added. The reaction solution was stirred at 0 °C for 0.5 h. Then a solution of compound **12-3** (100 g, 534.20 mmol, 1 eq) in dioxane (1000 mL) was added dropwise. The reaction solution was slowly warmed to room temperature and stirred at 45 °C for 11.5 h. The reaction solution was cooled to room temperature and extracted twice with ethyl acetate (2000 mL). The organic phases were combined, dried over anhydrous sodium sulfate, filtered, and concentrated under reduced pressure to give a crude product, which was then slurried with methyl *tert*-butyl ether (250 mL) at 10-15 °C for 30 min and filtered, and the filter cake was collected to give compound **12-4.** LC-MS: *m*/*z* = 347.2[M+H]⁺.

### Step 4

Potassium *tert*-butoxide (6.48 g, 57.73 mmol, 2.5 *eq*) was added to a solution of methyl triphenyl phosphonium bromide (20.62 g, 57.73 mmol, 2.5 *eq*) in tetrahydrofuran (100 mL) at 0 °C, and the reaction solution was stirred at 20 °C for 30 min. Then compound **12-4** (8 g, 23.09 mmol, 1 *eq*) was added to the reaction solution, and the mixture was stirred at 20 °C for 1.5 h. After the reaction was completed, the reaction solution was quenched with water (10 mL), and then the mixture was extracted 3 times with ethyl acetate (60 mL). The combined organic layers were washed 3 times with saturated brine (60 mL), dried over anhydrous sodium sulfate, and filtered, and the filtrate was concentrated under reduced pressure. The residue was separated and purified by silica gel column chromatography (ethyl acetate:petroleum ether = 1:7) to give compound **12-5.** LC-MS: *m*/*z* = 345.2[M+H]⁺.

### Step 5

Compound **12-5** (5.5 g, 15.97 mmol, 1 *eq*) was dissolved in a hydrogen chloride/methanol solution (4 M, 50 mL, 12.53 *eq*) at 60 °C, and the mixture was stirred at the temperature for 12 h. The reaction solution was cooled to room temperature and concentrated under reduced pressure to give compound **12-6.** LC-MS: *m*/*z* = 378.2[M+H]⁺.

### Step 6

Wet palladium on carbon (0.1 g, 10% content) and formic acid (7.64 g, 158.95 mmol, 20 *eq*) were added to a solution of compound **12-6** (3 g, 7.95 mmol, 1 *eq*) in methanol (30 mL) at room temperature under nitrogen atmosphere, and the reaction solution was purged with nitrogen several times and stirred at 45 °C for 1 h under nitrogen atmosphere. The reaction solution was cooled to room temperature and filtered, and the filtrate was concentrated to give compound **12-7.** LC-MS: *m*/*z* = 260.4[M+H]⁺.

### Step 7

Potassium iodide (3.71 g, 22.36 mmol, 2 *eq*) and cesium carbonate (14.57 g, 44.73 mmol, 4 *eq*) were added to a solution of compound **12-7** (2.9 g, 11.18 mmol, 1 *eq*) and compound **N-2** (4.16 g, 13.42 mmol, 1.2 *eq*) in *N,N-*dimethylformamide (50 mL) at 25 °C, and the mixture was stirred at the temperature for 12 h. Water (100 mL) was added to the reaction solution, then the mixture was extracted 3 times with ethyl acetate (300 mL), and the combined organic phases were washed 3 times with saturated brine (300 mL), dried over anhydrous sodium sulfate, and filtered. The filtrate was concentrated under reduced pressure, and the residue was separated and purified by preparative plate chromatography (ethyl acetate:petroleum ether = 1:8) to give compound **12-8.** LC-MS: *m*/*z* = 533.3[M+H]⁺.

### Step 8

Lithium hydroxide (1 M, 2.07 mL, 10 *eq)* was added to a solution of compound **12-8** (0.11 g, 206.51 µmol, 1 *eq*) in tetrahydrofuran (3 mL) and methanol (3 mL), and the mixture was stirred at 50 °C for 12 h. The reaction solution was concentrated, the resulting residue was adjusted to pH 7 with acetic acid and then concentrated under reduced pressure to give a residue, which was purified by high performance liquid chromatography (column: Unisil 3-100 C18 Ultra 150 × 50 mm × 3 µm; mobile phase: A: water (0.225% formic acid), B: acetonitrile; method: B: 9%-39%) to give compound **12.**

¹H NMR (400 MHz, DMSO-*d₆*) δ ppm 10.86 - 10.74 (m, 1H), 8.38 - 8.27 (m, 1H), 8.02 - 7.89 (m, 1H), 7.81 - 7.67 (m, 2H), 7.30 - 7.22 (m, 1H), 6.70 - 6.63 (m, 1H), 6.60 - 6.44 (m, 1H), 3.74 - 3.71 (m, 5H), 2.43 (s,3H), 2.20-2.15 (m, 2H), 1.75 - 1.67 (m, 3H), 1.65 - 1.52 (m, 2H), 1.52 - 1.40 (m, 2H), 1.20 - 1.16 (m, 2H), 0.95 - 0.89 (m, 2H);

LC-MS: *m*/*z* = 419.3[M+H]⁺.

### Biochemical determination: in vitro evaluation

### Experimental Example 1: Inhibition of Wieslab Complement Alternative Pathway Activation (Enzyme

### Activity Determination)

### Experimental objective:

To determine the inhibitory activity of the test compounds against the complement alternative pathway in human serum by using a Wieslab complement system alternative pathway kit.

### Experimental procedure:

The serum was diluted with a diluent (1:23). Drugs were added to the diluted serum in 8 concentration gradients, with the highest concentration of 10 mM or 50 mM and 5-fold gradient dilution. The resulting mixture was incubated at room temperature for 15 min. The compound and the serum mixture were added to a 96-well plate (100 µL/well) provided in the kit and activated at 37 °C for 1 h. The mixture was washed three times with a washing buffer. The detection antibody (100 µL) provided in the kit was added and the mixture was incubated at room temperature for 30 min. The mixture was washed three times with a washing buffer. A substrate (100 µL) was added and the mixture was incubated at room temperature for 30 min. The absorbance at 405 nM was measured using a microplate reader.

### Experimental results:

The results of the inhibitory activity of the test compounds against the complement alternative pathway are shown in Table 1, wherein A represented: the IC₅₀ value was less than 100 nM; B represented: IC₅₀ value was more than or equal to 100 nM and less than or equal to 1000 nM; C represented: the IC₅₀ value was more than 1000 nM.

**Table 1. Results of in vitro enzyme activity screening for compounds disclosed herein**

| Compound | **IC₅₀ for alternative pathway membrane attack complex (AP MAC) (nM)** |
|---|---|
| Compound **2** | A |
| Compound **3** | A |
| Formate salt of compound **5A** | A |
| Formate salt of compound **6B** | A |
| Compound **7A** | A |
| Compound **9A** | A |
| Compound **10A** | A |

Conclusion: the compound disclosed herein had significant inhibitory activity against the activation of the human serum alternative pathway.

### Experimental Example 2: Human Complement Factor B Protein Binding Determination (TR-FRET Method)

### Experimental objective:

To determine the binding activity of the test compounds to human complement Factor B protein by using TR-FRET method.

### Experimental procedure:

Drugs were added to phosphate buffered saline PBS (pH 7.4) starting at 10 µM with 4-fold dilution and 10 concentrations in total. A Cy5-labeled probe (75 nM) and europium chelate-labeled streptavidin (Perkin Elmer #AD0060; 0.225 nM) were added to biotinylated Factor B (25 nM) in the absence or presence of varying concentrations of test compounds, and the mixture was incubated at room temperature for 2 h. Time-resolved fluorescence resonance energy transfer (TR-FRET) data was measured using 330 nm as the excitation wavelength and 665 nm as the emission wavelength.

### Experimental results:

The binding activity of the test compounds to human complement Factor B protein is shown in Table 2. wherein A represented: the IC₅₀ value was less than 100 nM; B represented: IC₅₀ value was more than or equal to 100 nM and less than or equal to 1000 nM; C represented: the IC₅₀ value was more than 1000 nM.

**Table 2. Results of in vitro activity screening for compounds disclosed herein binding to human complement Factor B protein**

| Compound | **IC₅₀ for binding of human complement Factor B protein (nM)** |
|---|---|
| Formate salt of compound **5A** | A |
| Compound **7A** | A |
| Hydrochloride of compound **8** | A |
| Compound **9A** | A |
| Compound **10A** | A |
| Compound **11** | A |
| Compound **12** | A |

Conclusion: the compound disclosed herein had significant binding activity to human complement Factor B protein.

### Experimental Example 3: Pharmacokinetic Study in Rats

### Experimental objective:

To determine the drug concentration in the plasma of the test animals (male SD rats) at different time points after oral intragastric administration of the compounds disclosed herein by an LC/MS/MS method. To investigate the pharmacokinetic performance of the compounds disclosed herein in rats and to evaluate the pharmacokinetic characteristics.

### Experimental procedure:

Healthy male rats weighing 200-300 g, 2 rats in each group.

The compound disclosed herein was formulated into a 1 mg/mL clear solution with an aqueous solution of 0.5% MC (4000 cP)/0.5% Tween 80. Rats, after fasting overnight, were subjected to oral intragastric administration at a dose of 10 mg/kg. Blood was collected before administration and 0.25, 0.5, 1, 2, 4, 7, and 24 h after the administration, placed in heparin anticoagulation tubes, and centrifuged at 7000 rpm (5204 g) at 4 °C. The plasma was separated and stored at -80 °C. Four hours after administration, feeding was resumed. LC/MS/MS method was used to determine the content of the test compound in the plasma of the rats after oral administration. The plasma samples were analyzed after pretreatment to precipitate proteins.

### Experimental results:

The pharmacokinetic parameters are shown in Table 3.

**Table 3. Pharmacokinetic data of compound disclosed herein in rats**

| Parameter | **Cₘₐₓ (nM)** | **Tₘₐₓ (h)** | **T_{1/2} (h)** | **AUC**_{**0-**la**s**t} **(nM·h)** |
|---|---|---|---|---|
| Compound **2** | 3247 | 0.50 | 4.65 | 14663 |

Conclusion: the compound disclosed herein had good oral exposure, long half-life period, and excellent pharmacokinetic properties.

### Experimental Example 4: Mouse in Vivo PD Model of LPS-Induced Complement Activation

### Experimental objective:

To investigate the inhibitory effect of the compounds of the examples disclosed herein in the complement activation in mice stimulated and induced by LPS.

### Animals:

Female C57BL/6J mice, 7-9 weeks old, weighing 17-23 grams; supplier: Shanghai Sippe-Bk Lab Animal Co., Ltd.

### Experimental procedures:

Four hours before administration, 100 µg of lipopolysaccharide (LPS) in *Salmonella typhimurium* (Sigma) was dissolved in 100 µL of sterile PBS (phosphate buffered saline pH 7.2-7.4) to induce complement activation in mice by intraperitoneal injection. Normal mouse group (negative control): the animals received an intraperitoneal injection of 100 µL of sterile PBS and were administered vehicles alone via gavage (PO). Model group (positive control): the animals received intraperitoneal LPS and were administered vehicles (20% PEG400/10% solutol/70% water) PO. Drug group: samples were collected 4 h after administration of the compound (vehicle: 20% PEG400/10% solutol/70% water, administration volume: 5 mL/kg, dose: 10 mg/kg).

Sample collection: 0.3 mL of a blood sample was collected from the orbital venous plexus. All blood samples were added to a commercial 1.5-mL EDTA-K2 anticoagulation tube (supplier: Jiangsu KANGJIAN Medical Apparatus Co., Ltd.). After the blood collection, the plasma supernatant was taken by centrifugation at 3000 g at 4 °C for 10 min within half an hour, immediately placed in dry ice, stored in a refrigerator at -80 °C and used for Western blot analysis of the downstream C3d protein level after the complement activation.

Sample analysis: the mouse plasma (5 µL), a lysis buffer (27.5 µL), a loading buffer (12.5 µL), and a reducing buffer (5 µL) were mixed and incubated at 100 °C for 15 min with a loading amount of 5 µL/well, i.e., a loading amount of 0.5 µL of plasma per well.

Experimental results: after the intraperitoneal injection was carried out to induce the activation of the complement of the mice, the C3d protein level in the serum of the mice in the model group was increased by 40%-50% compared with that in the normal group; after the compound disclosed herein was orally administered, the C3d protein level in the serum of the mice in each of the drug groups was reduced by 60%-80% relative to that in the model group, showing a significantly reduced C3d protein level.

Experimental conclusion: the compound disclosed herein was capable of or capable of significantly inhibiting the LPS-stimulated complement activation compared with the normal and model groups.

## Claims

1. A compound of formula (I) or a pharmaceutically acceptable salt thereof, 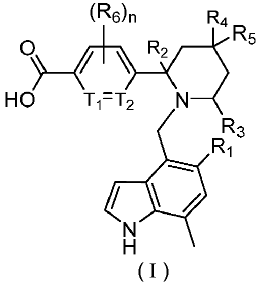 wherein
T₁ and T₂ are each independently selected from the group consisting of C, CH, and N;
R₁ is selected from the group consisting of C₁₋₃ alkoxy and C₁₋₃ alkyl, wherein the C₁₋₃ alkoxy and the C₁₋₃ alkyl are each independently optionally substituted with 1, 2, or 3 Rₐ;
R₅ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
when R₂ and R₃, together with the atom linked thereto, form 3- to 6-membered heterocyclyl, wherein the 3- to 6-membered heterocyclyl is optionally substituted with 1, 2, or 3 R_{b}, R₄ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
alternatively, when R₂ and R₄, together with the atom linked thereto, form 3- to 6-membered heterocyclyl, wherein the 3- to 6-membered heterocyclyl is optionally substituted with 1, 2, or 3 R_{b}, R₃ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
R₆ is selected from the group consisting of halogen, CN, C₁₋₃ alkyl, and C₁₋₃ alkoxy, wherein the C₁₋₃ alkyl and the C₁₋₃ alkoxy are each independently optionally substituted with 1, 2, or 3 R_{c};
each Rₐ is independently selected from the group consisting of F, Cl, Br, and I;
each R_{c} is independently selected from the group consisting of F, Cl, Br, and I;
each R_{b} is independently selected from the group consisting of halogen, C₁₋₃ alkyl, C₁₋₃ alkoxy, -C(=O)-C₁₋₃ alkyl, and -S(=O)ₘ-C₁₋₃ alkyl, wherein the C₁₋₃ alkyl, the C₁₋₃ alkoxy, the -C(=O)-C₁₋₃ alkyl, and the -S(=O)ₘ-C₁₋₃ alkyl are each independently optionally substituted with 1, 2, or 3 R;
each R is independently selected from the group consisting of F, Cl, Br, I, and OH;
n is selected from the group consisting of 0, 1, and 2;
m is selected from the group consisting of 0, 1, and 2.

2. A pharmaceutically acceptable salt of a compound of formula (I), 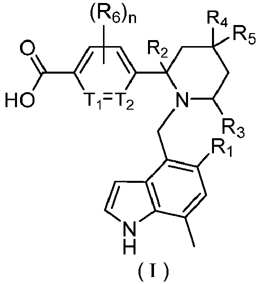 wherein
T₁ and T₂ are each independently selected from the group consisting of C, CH, and N;
R₁ is selected from the group consisting of C₁₋₃ alkoxy and C₁₋₃ alkyl, wherein the C₁₋₃ alkoxy and the C₁₋₃ alkyl are each independently optionally substituted with 1, 2, or 3 Rₐ;
R₅ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
when R₂ and R₃, together with the atom linked thereto, form 3- to 6-membered heterocyclyl, wherein the 3- to 6-membered heterocyclyl is optionally substituted with 1, 2, or 3 R_{b}, R₄ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
alternatively, when R₂ and R₄, together with the atom linked thereto, form 3- to 6-membered heterocyclyl, wherein the 3- to 6-membered heterocyclyl is optionally substituted with 1, 2, or 3 R_{b}, R₃ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
R₆ is selected from the group consisting of halogen, CN, C₁₋₃ alkyl, and C₁₋₃ alkoxy, wherein the C₁₋₃ alkyl and the C₁₋₃ alkoxy are each independently optionally substituted with 1, 2, or 3 R_{c};
each Rₐ is independently selected from the group consisting of F, Cl, Br, and I;
each R_{c} is independently selected from the group consisting of F, Cl, Br, and I;
each R_{b} is independently selected from the group consisting of halogen, C₁₋₃ alkyl, C₁₋₃ alkoxy, -C(=O)-C₁₋₃ alkyl, and -S(=O)ₘ-C₁₋₃ alkyl, wherein the C₁₋₃ alkyl, the C₁₋₃ alkoxy, the -C(=O)-C₁₋₃ alkyl, and the -S(=O)ₘ-C₁₋₃ alkyl are each independently optionally substituted with 1, 2, or 3 R;
each R is independently selected from the group consisting of F, Cl, Br, I, and OH;
n is selected from the group consisting of 0, 1, and 2;
m is selected from the group consisting of 0, 1, and 2.

3. The compound or the pharmaceutically acceptable salt thereof according to claim 1, or the pharmaceutically acceptable salt of the compound according to claim 2, wherein each R_{b} is independently selected from the group consisting of F, CH₃, CH₂CH₃, OCH₃, OCH₂CH₃, -C(=O)CH₃, -C(=O)CH₂CH₃, SCH₃, SCH₂CH₃, S(=O)CH₃, and S(=O)₂CH₃, wherein the CH₃, the CH₂CH₃, the OCH₃, the OCH₂CH₃, the -C(=O)CH₃, the -C(=O)-CH₂CH₃, the SCH₃, the SCH₂CH₃, the S(=O)CH₃, and the S(=O)₂CH₃ are each independently optionally substituted with 1, 2, or 3 R; preferably, each R_{b} is independently selected from the group consisting of CH₂CH₃, CH₂CH₂F, CH₂CHF₂, CH₂CF₃, -C(=O)CH₃, and S(=O)₂CH₃.

4. The compound or the pharmaceutically acceptable salt thereof according to claim 1, or the pharmaceutically acceptable salt of the compound according to claim 2, wherein R₁ is selected from the group consisting of CH₃, CH₂CH₃, CH₂CH₂CH₃, CH(CH₃)₂, OCH₃, OCH₂CH₃, OCH₂CH₂CH₃, and OCH(CH₃)₂, wherein the CH₃, the CH₂CH₃, the CH₂CH₂CH₃, the CH(CH₃)₂, the OCH₃, the OCH₂CH₃, the OCH₂CH₂CH₃, and the OCH(CH₃)₂ are each independently optionally substituted with 1, 2, or 3 Rₐ; preferably, R₁ is selected from the group consisting of CH₃, CF₃, OCF₃, and OCH₃.

5. The compound or the pharmaceutically acceptable salt thereof according to claim 1, or the pharmaceutically acceptable salt of the compound according to claim 2, wherein R₅ is selected from the group consisting of H, F, CH₃, CH₂CH₃, CH₂CH₂CH₃, CH(CH₃)₂, OCH₃, OCH₂CH₃, OCH₂CH₂CH₃, and OCH(CH₃)₂, wherein the CH₃, the CH₂CH₃, the CH₂CH₂CH₃, the CH(CH₃)₂, the OCH₃, the OCH₂CH₃, the OCH₂CH₂CH₃, and the OCH(CH₃)₂ are each independently optionally substituted with 1, 2, or 3 Rₐ; preferably, R₅ is selected from the group consisting of H, CH₃, OCH₃, CHF₂, and OCH₂CH₃.

6. The compound or the pharmaceutically acceptable salt thereof according to claim 1, or the pharmaceutically acceptable salt of the compound according to claim 2, wherein R₂ and R₃, together with the atom linked thereto, form aza-cyclobutyl, aza-cyclopentyl, and aza-cyclohexyl, wherein the aza-cyclobutyl, the aza-cyclopentyl, and the aza-cyclohexyl are each independently optionally substituted with 1, 2, or 3 R_{b}; preferably, R₄ is selected from the group consisting of H, CH₃, OCH₃, and F.

7. The compound or the pharmaceutically acceptable salt thereof according to claim 1, or the pharmaceutically acceptable salt of the compound according to claim 2, wherein R₂ and R₄, together with the atom linked thereto, form oxa-cyclobutyl, oxa-cyclopentyl, oxa-cyclohexyl, aza-cyclobutyl, aza-cyclopentyl, and aza-cyclohexyl, wherein the oxa-cyclobutyl, the oxa-cyclopentyl, the oxa-cyclohexyl, the aza-cyclobutyl, the aza-cyclopentyl, and the aza-cyclohexyl are each independently optionally substituted with 1, 2, or 3 R_{b}; preferably, R₃ is selected from the group consisting of H, CH₃, OCH₃, and F.

8. The compound or the pharmaceutically acceptable salt thereof according to claim 1, or the pharmaceutically acceptable salt of the compound according to claim 2, wherein the structural unit 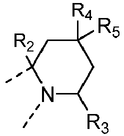 is selected from the group consisting of 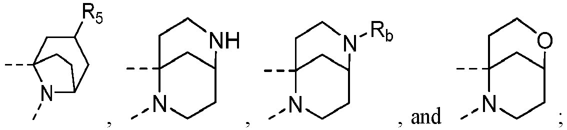 preferably, the structural unit 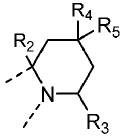 is selected from the group consisting of

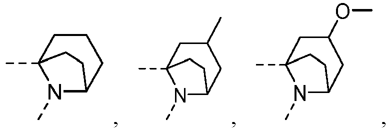

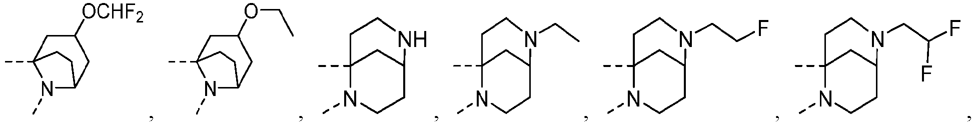

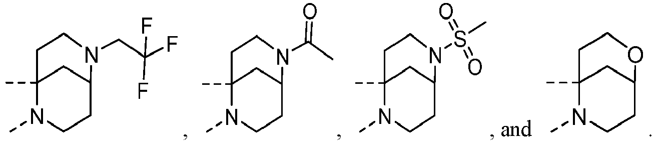

9. The compound or the pharmaceutically acceptable salt thereof according to claim 1, or the pharmaceutically acceptable salt of the compound according to claim 2, wherein R₆ is selected from the group consisting of F and CN.

10. The compound or the pharmaceutically acceptable salt thereof according to claim 1, or the pharmaceutically acceptable salt of the compound according to claim 2, wherein the structural unit 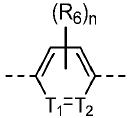 is selected from the group consisting of 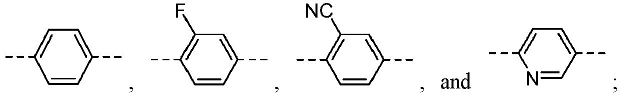 preferably, the structural unit 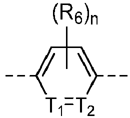 is selected from the group consisting of

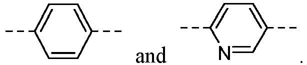

11. The compound or the pharmaceutically acceptable salt thereof according to claim 1, or the pharmaceutically acceptable salt of the compound according to claim 2, wherein the compound is selected from the group consisting of 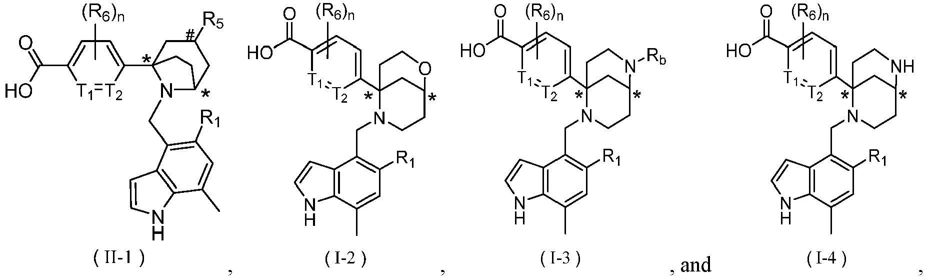 wherein
R₁, R₅, R₆, R_{b}, T₁, T₂, and n are as defined in any one of claims 1 or 2;
when R₅ is not H, the carbon atom with "#" is a chiral carbon atom present in a form of a single (*R*) or (S) enantiomer or in a form enriched with one enantiomer;
the carbon atom with "*" is a chiral carbon atom present in a form of a single (*R*) or (S) enantiomer or in a form enriched with one enantiomer.

12. The compound or the pharmaceutically acceptable salt thereof according to claim 1, wherein the compound is as follows: 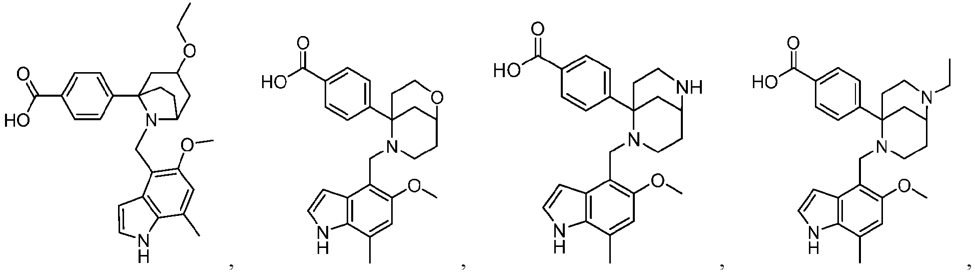 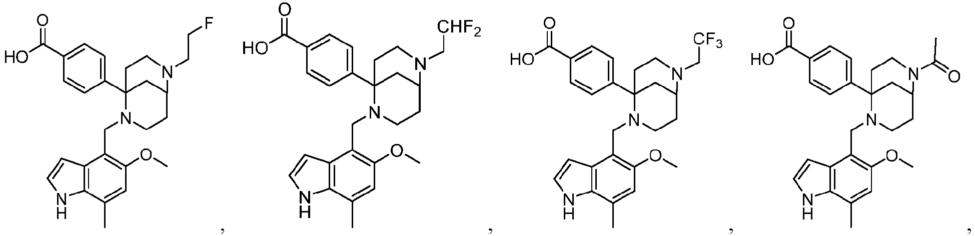 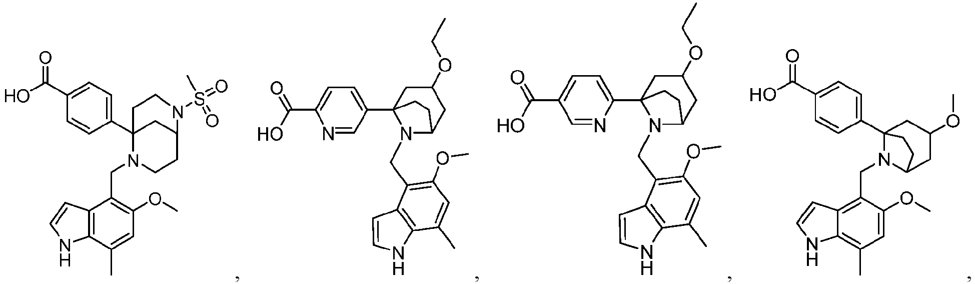 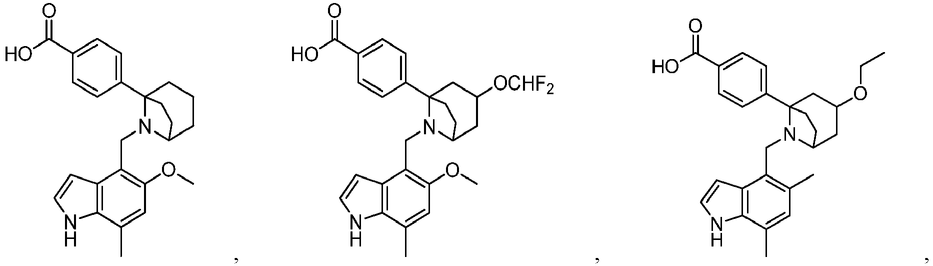 and

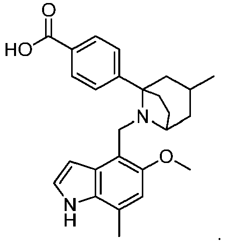

13. The pharmaceutically acceptable salt of the compound according to claim 2, wherein the compound is as follows:

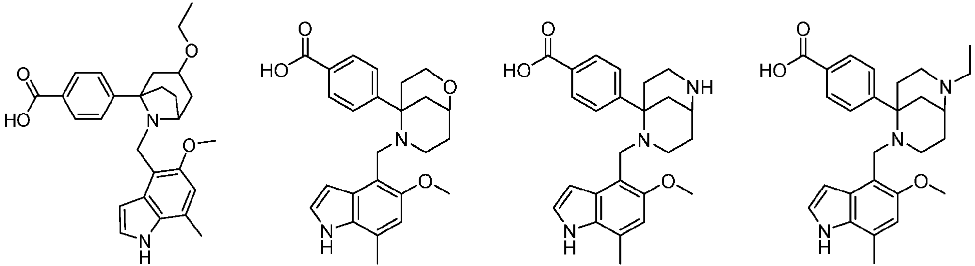

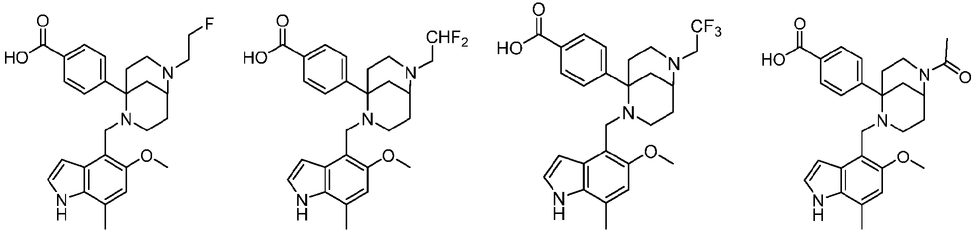

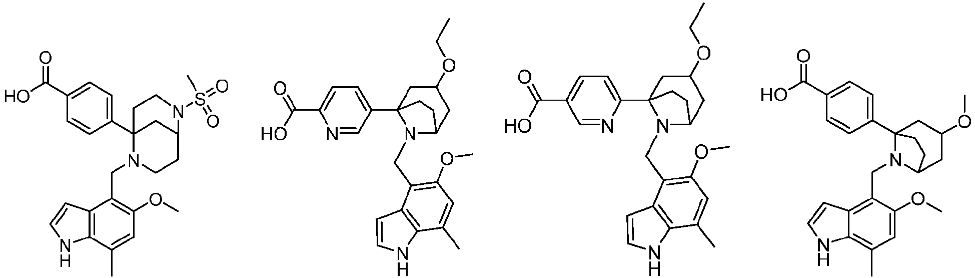

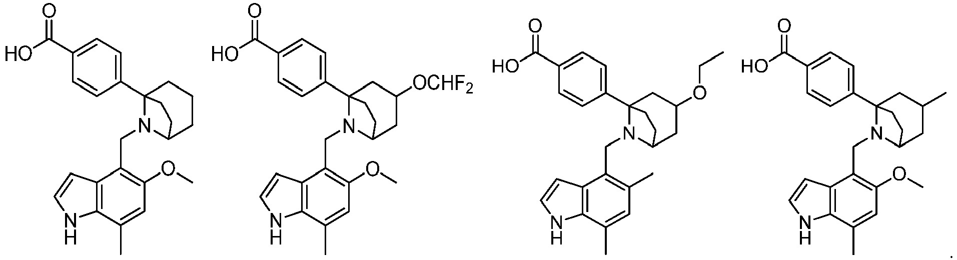

14. The compound or the pharmaceutically acceptable salt thereof according to claim 1, wherein the compound is selected from the group consisting of

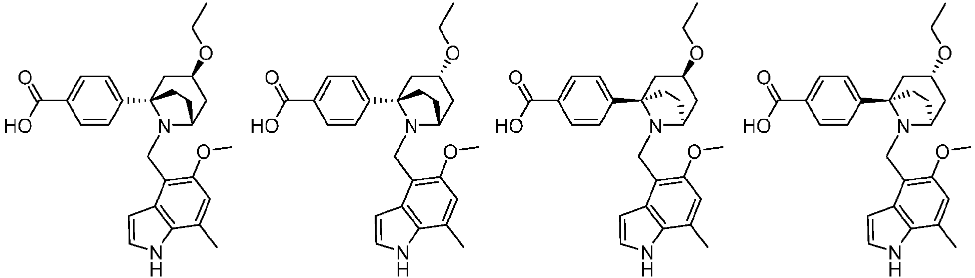

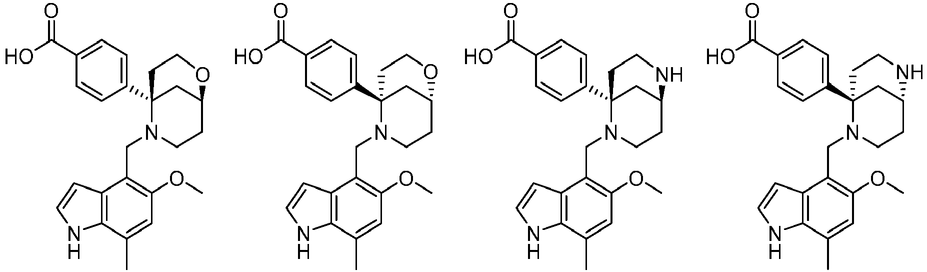

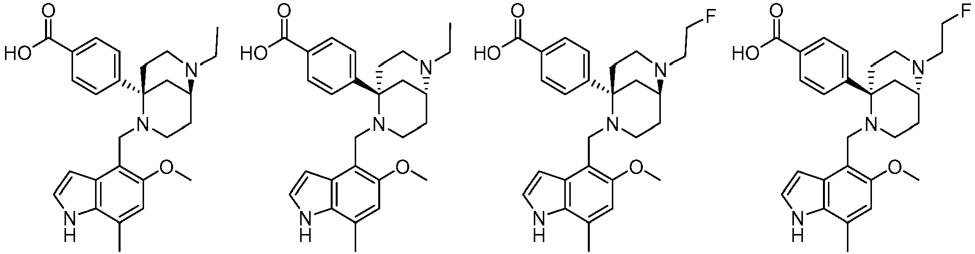

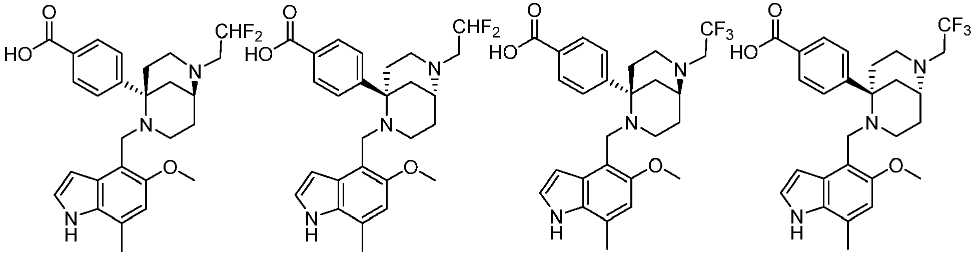

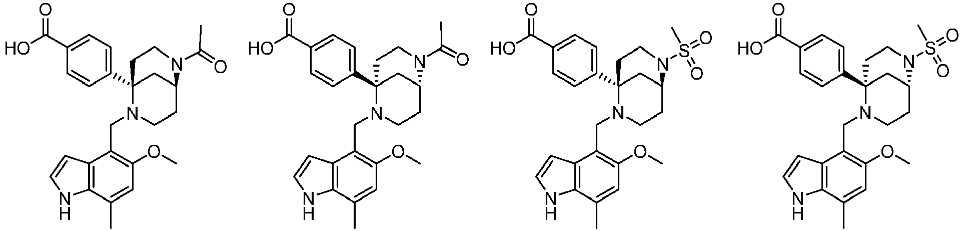

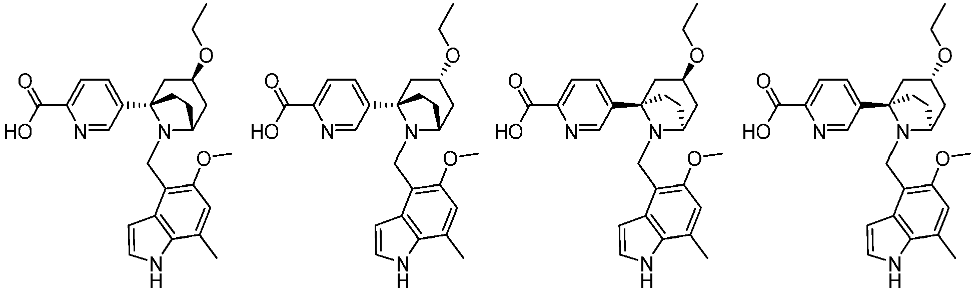

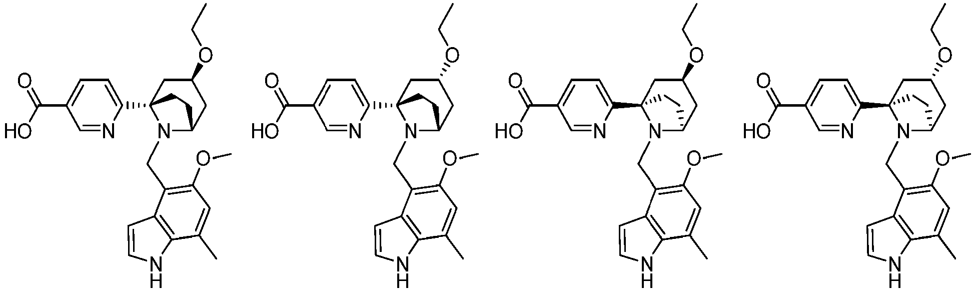

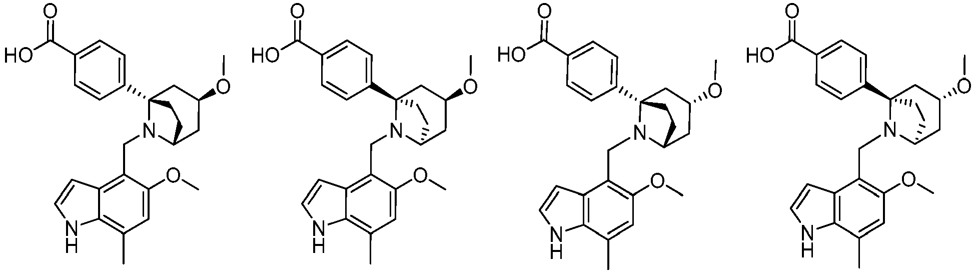

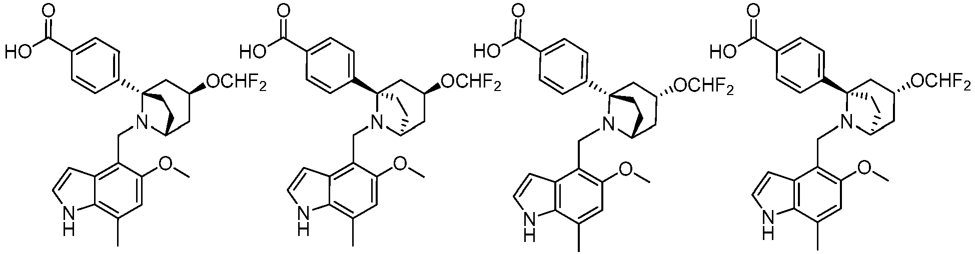

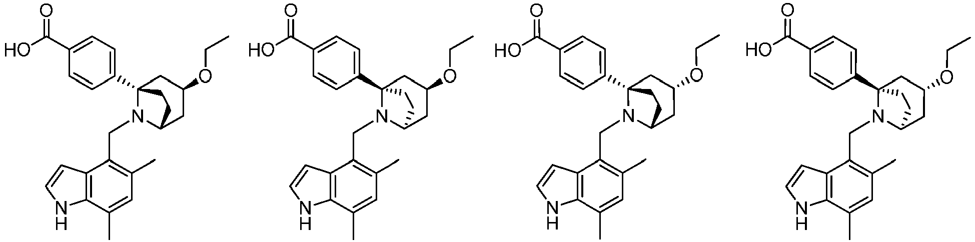

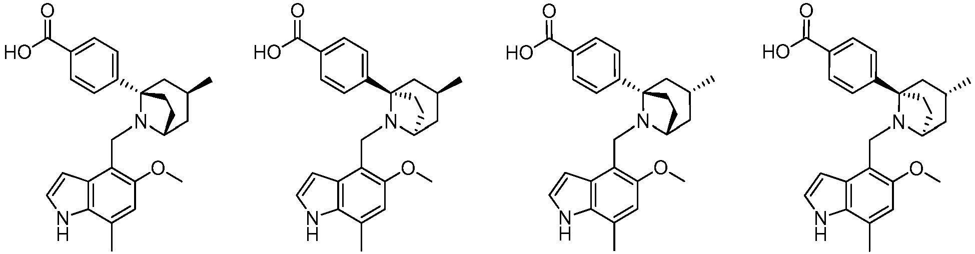

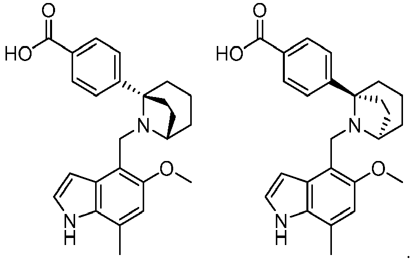

15. The pharmaceutically acceptable salt of the compound according to claim 2, wherein the compound is selected from the group consisting of

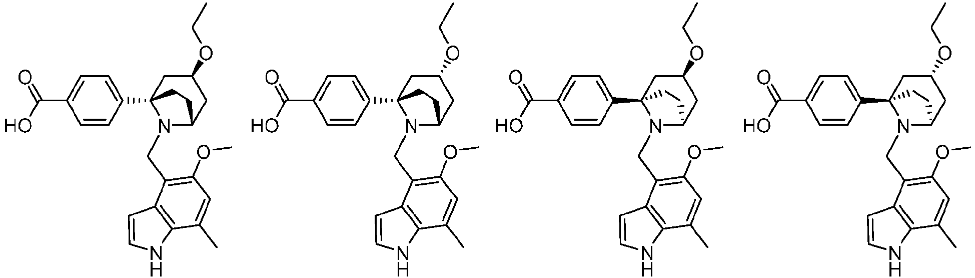

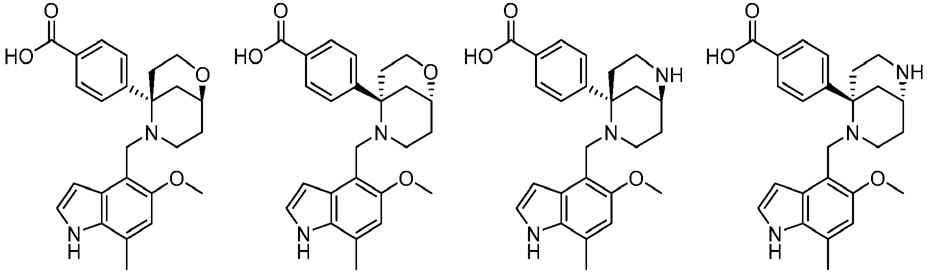

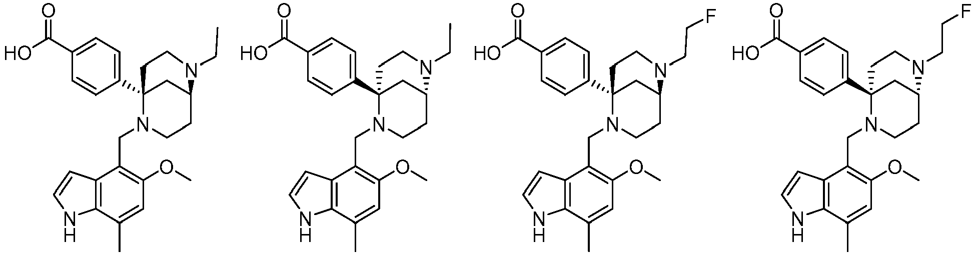

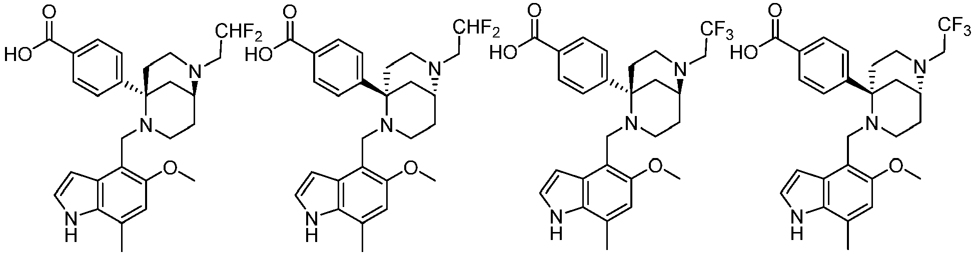

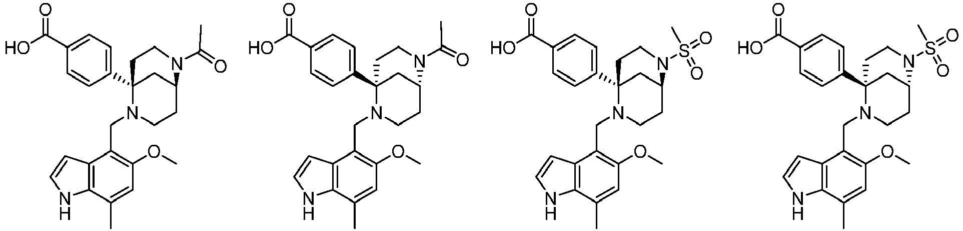

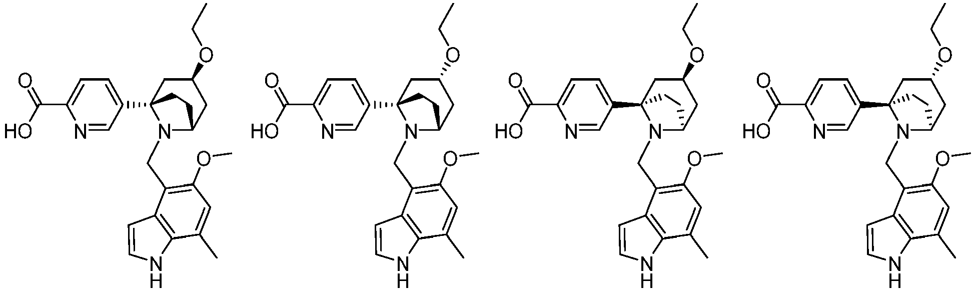

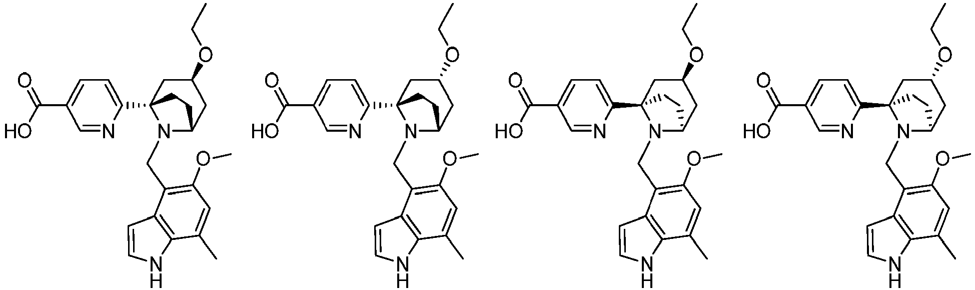

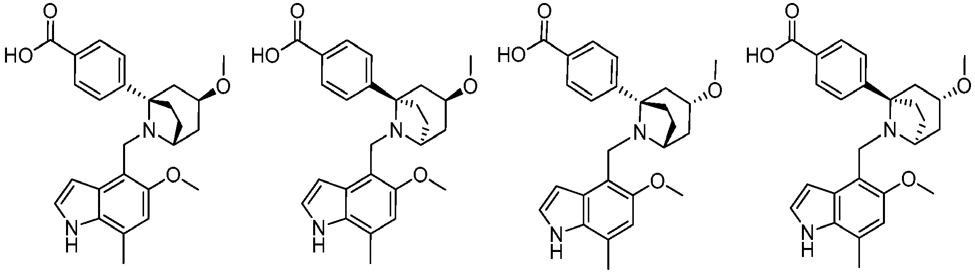

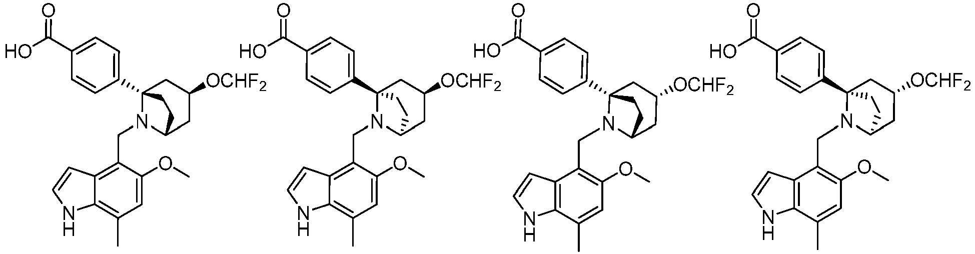

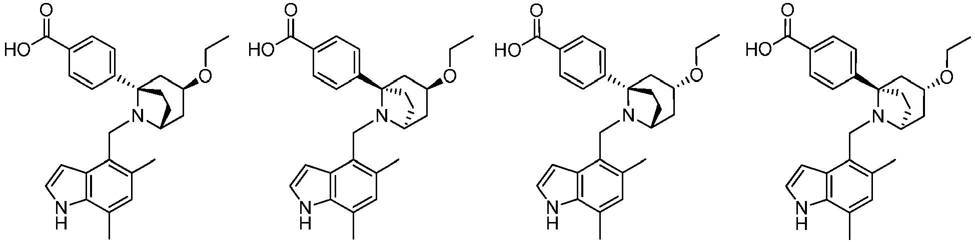

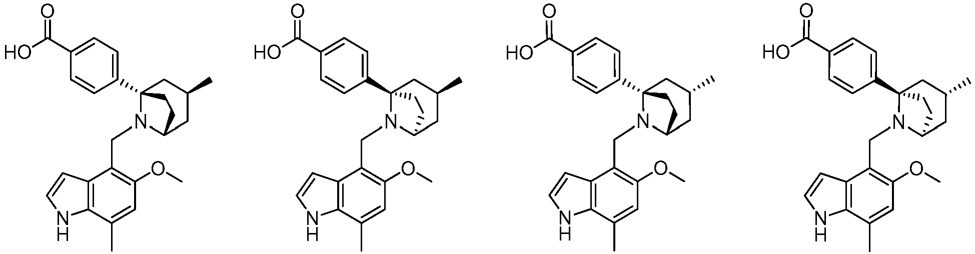

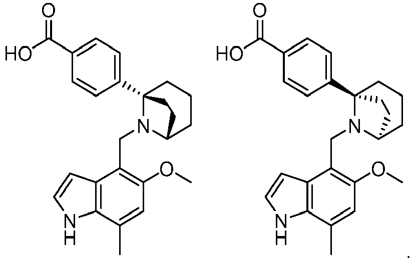

16. Use of the compound or the pharmaceutically acceptable salt thereof according to claim 1 or the pharmaceutically acceptable salt of the compound according to claim 2 in a medicament for treating a disease related to complement factor B.

17. A compound of formula (P) and a pharmaceutically acceptable salt thereof, 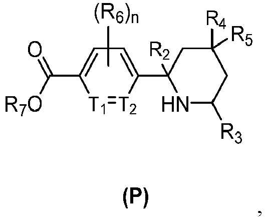 wherein
T₁ and T₂ are each independently selected from the group consisting of C, CH, and N;
R₅ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
when R₂ and R₃, together with the atom linked thereto, form 3- to 6-membered heterocyclyl, wherein the 3- to 6-membered heterocyclyl is optionally substituted with 1, 2, or 3 R_{b}, R₄ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
alternatively, when R₂ and R₄, together with the atom linked thereto, form 3- to 6-membered heterocyclyl, wherein the 3- to 6-membered heterocyclyl is optionally substituted with 1, 2, or 3 R_{b}, R₃ is selected from the group consisting of H, halogen, C₁₋₃ alkyl, and C₁₋₃ alkoxy, wherein the C₁₋₃ alkyl and the C₁₋₃ alkoxy are each independently optionally substituted with 1, 2, or 3 halogens;
R₆ is selected from the group consisting of halogen, CN, C₁₋₃ alkyl, and C₁₋₃ alkoxy, wherein the C₁₋₃ alkyl and the C₁₋₃ alkoxy are each independently optionally substituted with 1, 2, or 3 R_{c};
R₇ is selected from the group consisting of H and C₁₋₄ alkyl;
each R_{c} is independently selected from the group consisting of F, Cl, Br, and I;
each R_{b} is independently selected from the group consisting of halogen, C₁₋₃ alkyl, C₁₋₃ alkoxy, -C(=O)-C₁₋₃ alkyl, -S(=O)ₘ-C₁₋₃ alkyl, and N atom-protecting groups, wherein the C₁₋₃ alkyl, the C₁₋₃ alkoxy, the -C(=O)-C₁₋₃ alkyl, and the -S(=O)ₘ-C₁₋₃ alkyl are each independently optionally substituted with 1, 2, or 3 R;
each R is independently selected from the group consisting of F, Cl, Br, I, and OH;
n is selected from the group consisting of 0, 1, and 2;
m is selected from the group consisting of 0, 1, and 2.

18. The compound or the pharmaceutically acceptable salt thereof according to claim 17, wherein the compound is selected from the group consisting of

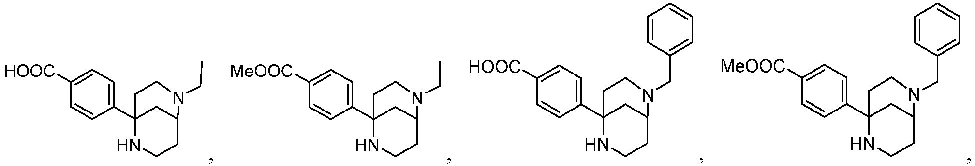

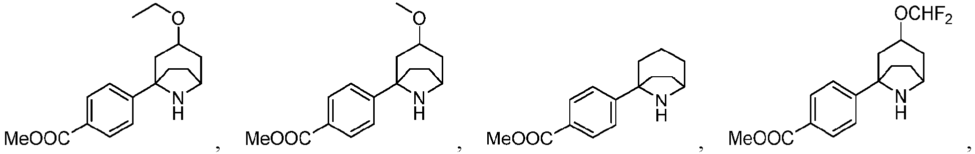

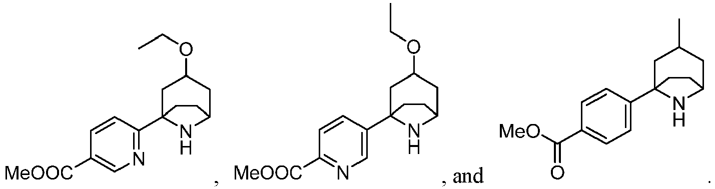

19. A method for preparing the compound of formula (I) according to claim 1, comprising: reacting a compound of formula (P) with a compound of formula (Q) to give a compound of formula (W); subjecting the compound of formula (W) to deprotection reaction to give the compound of formula (I), 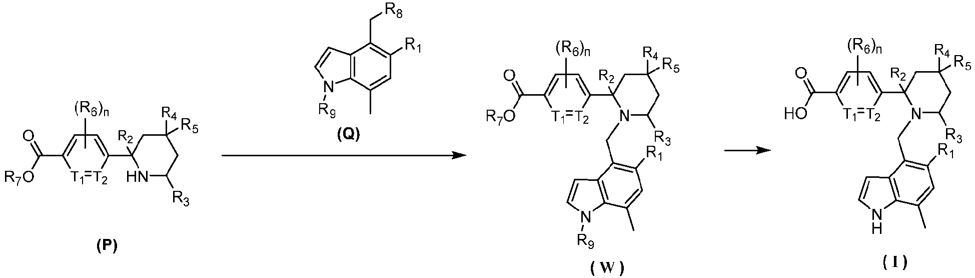
wherein R₉ is selected from the group consisting of N atom-protecting groups;
wherein R₂, R₃, R₄, R₅, R₆, R₇, R_{b}, Rₑ, R, T₁, T₂, n, and m in the compound of formula (P) are as defined for the compound of formula (P) in claim 17;
wherein R₁ and Rₐ in the compound of formula (Q) are as defined for the compound of formula (I) in claim 1; R₈ is selected from the group consisting of F, Cl, Br, I, and OH; preferably, R₈ is selected from the group consisting of Cl and OH;
wherein R₁, R₂, R₃, R₄, R₅, R₆, Rₐ, R_{b}, R_{c}, R, T₁, T₂, n, and m in the compound of formula (W) are as defined for the compound of formula (I) in claim 1; R₇ is selected from the group consisting of H and C₁₋₄ alkyl;
wherein R₁, R₂, R₃, R₄, R₅, R₆, Rₐ, R_{b}, R_{c}, R, T₁, T₂, n, and m in the compound of formula (I) are as defined for the compound of formula (I) in claim 1.

20. A method for preparing the compound of formula (I-3) according to claim 11, comprising: reacting a compound of formula (P-1) with a compound of formula (Q) to give a compound of formula (V); subjecting the compound of formula (V) to N-substitution reaction and deprotection reaction respectively to give the compound of formula (I-3), 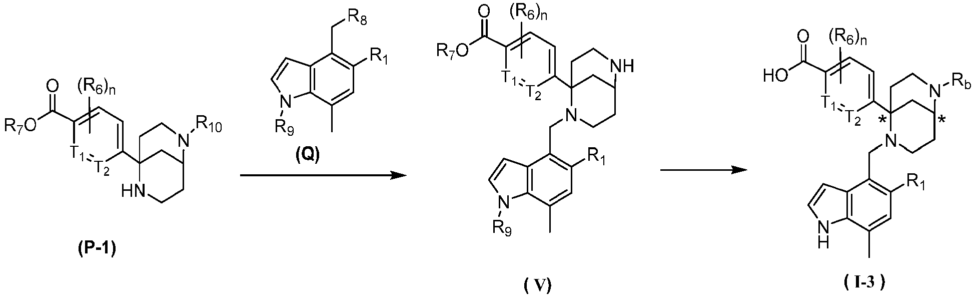
wherein R₉ and R₁₀ are each independently selected from the group consisting of N atom-protecting groups; wherein R₆, R₇, T₁, T₂, and n in the compound of formula (P-1) are as defined for the compound of formula (P) in claim 17;
wherein R₁ and Rₐ in the compound of formula (Q) are as defined for the compound of formula (I) in claim 1; R₈ is selected from the group consisting of F, Cl, Br, I, and OH; preferably, R₈ is selected from the group consisting of Cl and OH;
wherein R₁, R₆, Rₐ, T₁, T₂, and n in the compound of formula (V) are as defined for the compound of formula (I) in claim 1; R₇ is selected from the group consisting of H and C₁₋₄ alkyl;
wherein R₁, R_{b}, Rₐ, R_{b}, T₁, T₂, n, and m in the compound of formula (I-3) are as defined for the compound of formula (I) in claim 1;
the carbon atom with "*" is a chiral carbon atom present in a form of a single (*R*) or (S) enantiomer or in a form enriched with one enantiomer.
